# EUROPEAN PATENT SPECIFICATION

(11) **EP 2 867 255 B1**
(45) Date of publication and mention of the grant of the patent: **19.07.2017**
(21) Application number: 13734693.8
(22) Date of filing: 25.06.2013
(51) Int. Cl.: C07K 16/00, C07K 16/46, A61K 39/395

(54) **METHOD FOR THE SELECTION AND PRODUCTION OF TAILOR-MADE, SELECTIVE AND MULTI-SPECIFIC THERAPEUTIC MOLECULES COMPRISING AT LEAST TWO DIFFERENT TARGETING ENTITIES AND USES THEREOF**
VERFAHREN ZUR AUSWAHL UND HERSTELLUNG MASSGESCHNEIDERTER, SELEKTIVER UND MULTISPEZIFISCHER THERAPIEMOLEKÜLE MIT MINDESTENS ZWEI VERSCHIEDENEN TARGETING-EINHEITEN SOWIE VERWENDUNGEN DAVON
PROCÉDÉ POUR LA SÉLECTION ET LA PRODUCTION DE MOLÉCULES THÉRAPEUTIQUES FABRIQUÉES SUR MESURE, SÉLECTIVES ET MULTI-SPÉCIFIQUES COMPRENANT AU MOINS DEUX ENTITÉS DE CIBLAGE DIFFÉRENTES ET LEURS UTILISATIONS

(30) Priority: 27.06.2012 EP 12173878
(43) Date of publication of application: 06.05.2015
(73) Proprietor: F. Hoffmann-La Roche AG, 4070 Basel (CH)
(72) Inventor: HEINDL, Dieter, 81377 München (DE); HUELSMANN, Peter, Michael, 82392 Habach (DE); KALUZA, Brigitte, 82362 Weilheim (DE); KOPETZKI, Erhard, 82377 Penzberg (DE); NIEDERFELLNER, Gerhard, 82386 Oberhausen (DE); TIEFENTHALER, Georg, 82404 Sindelsdorf (DE)
(74) Representative: Burger, Alexander
(86) International application number: PCT/EP2013/063260
(87) International publication number: WO 2014/001326

(56) References cited:
- WO-A1-95/05399
- WO-A1-2012/085111
- WO-A1-2012/085113
- WO-A2-2006/137932
- WO-A2-2009/037659
- WO-A2-2012/085069
- US-A1- 2002 051 986
- US-A1- 2008 044 834
- HAYASHI GOSUKE ET AL: "Application of L-DNA as a molecular tag", NUCLEIC ACIDS SYMPOSIUM SERIES, OXFORD UNIVERSITY PRESS, GB, no. 49, 1 January 2005 (2005-01-01), pages 261-262, XP002670990, ISSN: 0261-3166
- PACK P ET AL: "MINIANTIBODIES: USE OF AMPHIPATHIC HELICES TO PRODUCE FUNCTIONAL, FLEXIBLY LINKED DIMERIC FV FRAGMENTS WITH HIGH AVIDITY IN ESCHERICHIA COLI", BIOCHEMISTRY, AMERICAN CHEMICAL SOCIETY, US, vol. 31, no. 6, 18 February 1992 (1992-02-18), pages 1579-1584, XP002026334, ISSN: 0006-2960, DOI: 10.1021/BI00121A001
- KOSTELNY S A ET AL: "FORMATION OF A BISPECIFIC ANTIBODY BY THE USE OF LEUCINE ZIPPERS", THE JOURNAL OF IMMUNOLOGY, THE AMERICAN ASSOCIATION OF IMMUNOLOGISTS, US, vol. 148, no. 5, 1 March 1992 (1992-03-01) , pages 1547-1553, XP001088092, ISSN: 0022-1767
- MULLER K M ET AL: "A dimeric bispecific miniantibody combines two specificities with avidity", FEBS LETTERS, ELSEVIER, AMSTERDAM, NL, vol. 432, no. 1-2, 31 July 1998 (1998-07-31), pages 45-49, XP004259191, ISSN: 0014-5793, DOI: 10.1016/S0014-5793(98)00829-1
- D. M. GOLDENBERG ET AL: "Multifunctional Antibodies by the Dock-and-Lock Method for Improved Cancer Imaging and Therapy by Pretargeting", THE JOURNAL OF NUCLEAR MEDICINE, vol. 49, no. 1, 1 January 2008 (2008-01-01), pages 158-163, XP055028152, ISSN: 0161-5505, DOI: 10.2967/jnumed.107.046185
- CHAN ANDREW C ET AL: "Therapeutic antibodies for autoimmunity and inflammation", NATURE REVIEWS. IMMUNOLOGY, NATURE PUBLISHING GROUP, GB, vol. 10, no. 5, 1 May 2010 (2010-05-01), pages 301-316, XP002665072, ISSN: 1474-1733, DOI: 10.1038/NRI2761
- ROLAND KONTERMANN: "Dual targeting strategies with bispecific antibodies", MABS, LANDES BIOSCIENCE, US, vol. 4, no. 2, 1 March 2012 (2012-03-01), pages 182-197, XP009160769, ISSN: 1942-0870, DOI: 10.4161/MABS.4.2.19000

## Description

Herein are reported methods for selecting and producing multispecific therapeutic molecules made of a polypeptide-polynucleotide-complex, wherein the specificities of the therapeutic molecule are chosen depending on the phenotype of the therapeutic targets.

### Background of the Invention

Over the past years, a wide variety of tumor-specific therapeutic proteins, including antibodies, antibody fragments, and ligands for cell surface receptors have been developed and clinically tested. These therapeutic proteins have been conjugated to several classes of therapeutic toxins such as small molecule drugs, enzymes, radioisotopes, protein toxins, and other toxins for specific delivery to patients.

Effective delivery to the site of disease is a prerequisite for high efficacy and low toxicity of any therapeutic molecule. For example antibodies can participate in this context. If the antibody is not the therapeutic by itself conjugation of a drug to an antibody makes it possible to achieve excellent localization of the drug at the desired site within the human body. This increases the effective drug concentration within this target area, thereby optimizing the therapeutic effect of the agent. Furthermore, with targeted delivery, the clinician may be able to lower the dose of the therapeutic agent - something that is particularly relevant if the drug payload has associated toxicities or if it is to be used in the treatment of chronic conditions (see e.g. McCarron, P.A., et al., Mol. Interventions 5 (2005) 368-380).

The generation of bispecific antibodies is e.g. reported in WO 2004/081051. A broad spectrum of bispecific antibody formats has been designed and developed (see e.g. Fischer, N. and Leger, O., Pathobiology 74 (2007) 3-14). Chelating recombinant antibodies (CRAbs) are originally reported by Neri, D., et al. (Neri, D., et al., J. Mol. Biol. 246 (1995) 367-373). Wright, M.J. and Deonarain, M.P. (Molecular Immunology 44 (2007) 2860-2869) reported a phage display library for generation of chelating recombinant antibodies.

Molecular vehicles for targeted drug delivery are reported by Backer, M.V., et al., Bioconjugate Chem. 13 (2002) 462-467. WO 2010/118169 reports human protein scaffolds with controlled serum pharmacokinetics. Methods and compositions related to peptides and proteins with C-terminal elements cross-reference to related applications is reported in WO 2009/105671. In WO 2007/038658 antibody-drug conjugates and methods of use are reported. Compositions and methods for targeted biological delivery of molecular carriers are reported in WO 2004/062602. In WO 2002/072141 targeted ligands are reported.

In WO 2009/037659 magnetic detection of small entities is reported. Homogeneous analyte detection is reported in WO 2006/137932. In US 2008/0044834 a three-component biosensor for detecting macromolecules and other analytes is reported. The design and synthesis of bispecific reagents is reported in WO 95/05399.

In US 2002/051986 methods for the detection of an analyte by means of a nucleic acid reporter is reported. Design and synthesis of bispecific reagents by use of double-stranded DNAs as chemically and spatially defined cross-linkers is reported in WO 95/05399.

Gosuke, H., et al. report the application of L-DNA as molecular tag (Nucl. Acids Symp. Ser. 49 (2005) 261-262. The use of amphiphatic helices to produce functional, flexibly linked dimeric Fv fragments with high avidity in E.coli is reported by Pack, P., et al. (Biochem. 31 (1992) 1579-1584). Kostelny, S.A., et al., report the formation of a bispecific antibody by the use of leucine zippers (J. Immunol. 148 (1992) 1547-1553). A dimeric bispecific miniantibody combining two specificities with avidity is reported by Muller, K.M., et al. (FEBS Lett. 432 (1998) 45-49). Goldenberg, D.M., et al. report the production of multifunctional antibodies by the dock-and-lock method for improved cancer imaging and therapy by pretargeting (J. Nuc. Med. 49 (2008) 158-163).

### Summary of the Invention

Herein is reported a method for providing a tailor-made, highly specific multispecific therapeutic molecule for the treatment of a disease, such as cancer, in a patient in need of a treatment, whereby the therapeutic molecule is adapted to the characteristics of the disease of the patient and/or to the genotype/phenotype of the patient.

Such adaptation is achieved by making a tailor-made molecule taking into account the genotype/phenotype of the disease harboring/affected cells of the patient.

In a first step the genotype/phenotype of the cells (e.g. the presence and number/quantity of disease-specific cell surface antigens) that are intended to be targeted with the therapeutic molecule is determined. This can be achieved, e.g. by cell imaging techniques such as immunohistochemical staining (IHC, immunohistochemistry) of patient's cells derived e.g. from blood and/or biopsied material using fluorescently labeled monospecific (therapeutic or diagnostic) antibodies. Alternatively the genotype/phenotype of the cells can be analyzed after staining with labeled therapeutic or diagnostic antibodies using FACS-based methods. In vivo imaging techniques including optical imaging, molecular imaging, fluorescence imaging, bioluminescence Imaging, MRI, PET, SPECT, CT, and intravital microscopy may be used also for determination of the genotype/phenotype of disease-related cells of a patient. Depending on the determined genotype/phenotype of the disease-related cells of a patient a tailor-made combination of targeting/binding entities can be/is chosen and are combined in a therapeutic molecule. Such a therapeutic molecule may be for example a bispecific antibody.

Such tailor-made therapeutic molecules i) will be highly specific, ii) will have a good efficacy, and iii) will induce less side effects compared to conventionally chosen therapeutics. This can be achieved by endowing the therapeutic molecule with improved targeting and/or improved tailor-made delivery properties, e.g. for a therapeutic payload to its intended site of action.

The improved delivery of the therapeutic molecule to its site of action, such as e.g. a cancer cell, can be achieved by a higher/increased selectivity and/or specificity of the targeted therapeutic molecule compared to conventionally chosen therapeutic molecules. The therapeutic molecule comprises at least two entities that specifically bind to different antigens (e.g. two different surface markers) or to different epitopes on the same antigen (e.g. two different epitopes on the same surface marker).

The increased selectivity and/or specificity of the tailor-made therapeutic molecule can be achieved by the simultaneous binding of both targeting entities to their respective targets/epitopes, i.e. it is achieved by avidity effects. Especially suited is the combination of two binding entities having a low to medium affinity for its respective targets/epitopes. Additionally, off-target binding is greatly reduced or can even be eliminated totally.

The binding specificities are provided separately by the starting components of which the multispecific therapeutic molecule is formed. Thus, it is possible to tailor-make a multispecific therapeutic molecule, such as a bispecific antibody, simply by determining the surface markers present on a cell, e.g. on a cancer cell, and conjugating the respective binding entities, such as antibody fragments, that specifically bind to these surface markers to a nucleic acid and linking these by a linker nucleotide.

It has been found that for the targeted delivery of an effector moiety a complex comprising polypeptide and polynucleotide components is especially useful. The effector moiety, the polypeptide component and the polynucleotide linker of the complex are non-covalently bound to each other. This allows a modular production of the individual components of the complex. Due to the modular architecture of the complex's individual components can be changed without the need to change the other components of the complex. This allows for an easy and efficient assembly of a multitude of complex variants, e.g. for the provision of a library, based on which tailor-made, highly specific multispecific therapeutic molecule can be selected.

Reported herein is a method for the selection of at least two binding entities from a collection/library of binding entities which are assembled in a single multispecific binding molecule by incubating (a) an antibody Fab fragment or a scFv antibody fragment each comprising or conjugated to a first partner or member of a first binding pair, whereby the Fab fragment or scFv specifically binds to a first cell surface marker or to a first epitope of a first cell surface marker, (b) an antibody Fab fragment or a scFv antibody fragment each comprising or conjugated to a first partner or member of a second binding pair, whereby the Fab fragment or scFv antibody fragment specifically binds to a second cell surface marker or to a second epitope of a first cell surface marker, and (c) a linker comprising at one of its termini the second member of the first binding pair and at the respective other terminus the second member of the second binding pair, for use as a therapeutic agent. Such an agent has improved targeting/delivery properties.

Reported herein is a method for producing a multispecific binding molecule comprising the following steps
(i) determining the cell surface makers present in a cell containing sample and i) selecting thereof at least a first cell surface marker and optionally a second cell surface marker, or ii) selecting thereof a multitude of cell surface markers corresponding to the number of binding specificities of the multispecific binding molecule,
(ii) incubating (a) a multitude of binding entities each comprising a first partner or member of a binding pair, whereby each of the binding entities specifically binds to a different cell surface marker or its ligand or epitope of the same cell surface marker, whereby each first partner or member of a binding pair does bind only to its corresponding second partner or member and does not bind to any of the other second partners or members of binding pairs, and (b) a linker comprising the corresponding second members of the binding pairs,
and thereby producing the multispecific binding molecule.

One aspect as reported herein is a method for producing a bispecific antibody comprising the following steps
(i) determining cell surface makers present on the surface of a cell in a sample and selecting thereof a first surface marker and a second surface marker,
(ii) incubating (a) an antibody Fab fragment or a scFv antibody fragment comprising or conjugated to a first partner or member of a first binding pair, whereby the Fab fragment or scFv specifically binds to the first cell surface marker, (b) an antibody Fab fragment or a scFv antibody fragment comprising or conjugated to a first partner or member of a second binding pair, whereby the Fab fragment or scFv antibody fragment specifically binds to the second cell surface marker, and (c) an enantiomeric DNA polynucleotide linker comprising at one of its termini the second member of the first binding pair and at the respective other terminus the second member of the second binding pair,
and thereby producing the bispecific antibody.

Reported herein is a method for determining a combination of binding entities for a multispecific binding molecule comprising the following steps
(i) determining the binding specificity and/or selectivity and/or affinity and/or effector function and/or in vivo half-life of a multitude of multispecific binding molecules whereby in the multitude of multispecific binding molecules each (possible) combination of binding entities is comprised,
   and
(ii) choosing the multispecific binding molecule with suitable binding specificity and/or selectivity and/or affinity and/or effector function and/or in vivo half-life and thereby determining a combination of antigen binding entities.

One aspect as reported herein is a method for determining a combination of antigen binding sites comprising the following steps
(i) determining the binding specificity and/or affinity of a multitude of bispecific antibodies prepared by combining each member of a first multitude of antibody Fab fragments or scFv antibody fragments each comprising or conjugated to the same first member of a first binding pair with each member of a second multitude of antibody Fab fragments or scFv antibody fragments each comprising or conjugated to the same first member of a second binding pair and an enantiomeric DNA polynucleotide linker comprising at one of its termini the second member of the first binding pair and at the respective other terminus the second member of the second binding pair,
   whereby the first multitude specifically binds to a first cell surface marker and the second multitude specifically binds to a second cell surface marker,
   and
(ii) choosing the bispecific antibody with suitable binding specificity and/or affinity and thereby determining a combination of antigen binding sites.

Reported herein is a bispecific antibody comprising
a) a first Fab fragment or scFv antibody fragment
   i) that specifically binds to a first surface marker, and
   ii) that is conjugated to a first member of a first binding pair,
b) a second Fab fragment or scFv antibody fragment
   i) that specifically binds to a second surface marker, and ii) that is conjugated to a first member of a second binding pair, and
c) an enantiomeric DNA polynucleotide linker
   i) that is conjugated to the second member of the first binding pair, and
   ii) that is conjugated to the second member of the second binding pair,
whereby the first and second Fab fragment or scFv antibody fragment form a non-covalent complex.

The following are embodiments of all aspects as reported herein. It is herewith pointed out that each embodiment can be combined with each aspect and also with all other individual embodiments as given herein.

In one embodiment the binding entities are independently of each other selected from a darpin domain based binding entity, an anticalin domain based binding entity, a T-cell receptor fragment like scTCR domain based binding entity, a camel VH domain based binding entity, a tenth fibronectin 3 domain based binding entity, a tenascin domain based binding entity, a cadherin domain based binding entity, an ICAM domain based binding entity, a titin domain based binding entity, a GCSF-R domain based binding entity, a cytokine receptor domain based binding entity, a glycosidase inhibitor domain based binding entity, a superoxide dismutase domain based binding entity, or antibody fragments (Fab or scFv fragments).

In one embodiment of all aspects the first and second binding entity is independently of each other an antibody fragment.

In one embodiment the antibody fragment is selected from the group comprising Fv, Fab, Fab', Fab'-SH, F(ab')₂, diabody, linear antibody, scFv, scFabs, and dsFvs.

In one embodiment at least two components of the bispecific antibody comprising the effector moiety, the binding specificities and the polynucleotide linker are non-covalently associated with each other.

In one embodiment the binding entity is selected from antibodies, antibody fragments, receptors, receptor ligands, and target binding scaffolds, with the proviso that the receptor ligand is not an incretin receptor ligand polypeptide.

In one embodiment the antibody fragment is selected from the group comprising Fv, Fab, Fab', Fab'-SH, F(ab')₂, diabody, linear antibody, scFv, scFabs, and dsFvs. In one embodiment the target binding scaffold is selected from darpins, hemopexin-like molecule, and anticalins.

In one embodiment the receptor is selected from T-cell receptor fragments and scTCR.

In one example the multispecific binding molecule is a complex comprising
a) a first binding entity
   i) that specifically binds to a first cell surface marker or its ligand, and
   ii) that is conjugated to a first member of a first binding pair,
b) a second binding entity
   i) that specifically binds to a second cell surface marker or its ligand, and
   ii) that is conjugated to a first member of a second binding pair, and
c) a polynucleotide linker
   i) that is conjugated to the second member of the first binding pair, and
   ii) that is conjugated to the second member of the second binding pair.

In one embodiment the bispecific antibody is a complex comprising
a) a first Fab fragment or scFv antibody fragment
   i) that specifically binds to a first cell surface marker, and
   ii) that is conjugated to a first member of a first binding pair,
b) a second Fab fragment or scFv antibody fragment
   i) that specifically binds to a second cell surface marker, and
   ii) that is conjugated to a first member of a second binding pair, and
c) a polynucleotide linker
   i) that is conjugated to the second member of the first binding pair, and
   ii) that is conjugated to the second member of the second binding pair.

In one embodiment the complex is a non-covalent complex.

In one embodiment the complex further comprises a further polypeptide i) that specifically binds to a second target, and ii) that is conjugated to a first member of a second binding pair, and the polynucleotide linker is conjugated to the second member of the second binding pair.

In one embodiment the complex further comprises an effector moiety that is conjugated to a polynucleotide that is complementary to at least a part of the polynucleotide linker.

In one embodiment the complex further comprises an effector moiety conjugated to a polynucleotide that is i) complementary to at least a part of the polynucleotide that is conjugated to the first or second binding entity or Fab fragment or scFv antibody fragment and ii) not complementary to the polynucleotide linker.

In one embodiment the first and second binding entity or Fab fragment or scFv antibody fragment bind to the same target and to non-overlapping epitopes thereon.

In one embodiment the polynucleotide linker comprises of from 8, 10, 15, 20, 25, 50, 100 nucleotides. In one embodiment the polynucleotide linker comprises up to 500, 750, 1000, or 2000 nucleotides. In one embodiment the polynucleotide linker comprises of from 10 to 500 nucleotides.

The polynucleotide linker is enantiomeric DNA. In one embodiment the enantiomeric DNA is L-DNA. In one embodiment the L-DNA is single stranded L-DNA (ss-L-DNA).

In one embodiment the effector moiety is selected from the group consisting of a binding moiety, a labeling moiety, and a biologically active moiety.

In one embodiment the polynucleotide linker is conjugated to the binding entity, or Fab fragment, or scFv antibody fragment at its first or second terminus.

In one embodiment the polynucleotide linker is conjugated to two second members of two binding pairs, whereby the second member of the first binding pair is conjugated to the first terminus of the polynucleotide linker and the second member of the second binding pair is conjugated to the second terminus of the polynucleotide linker.

In one embodiment the first and second members of the first binding pair comprise the nucleic acid sequences of SEQ ID NO: 05 and SEQ ID NO: 08, respectively.

In one embodiment the first and second members of the second binding pair comprise the nucleic acid sequences of SEQ ID NO: 06 and SEQ ID NO: 07, respectively.

In one embodiment the method comprises the steps of:
a) synthesizing the first binding entity, or Fab fragment, or scFv antibody fragment that specifically binds to a first cell surface marker or its ligand and that is conjugated to a first member of a first binding pair,
b) synthesizing the second binding entity, or Fab fragment, or scFv antibody fragment that specifically binds to a second cell surface marker or its ligand and that is conjugated to a first member of a second binding pair,
c) synthesizing the polynucleotide linker that is conjugated to the second member of the first binding pair and that is conjugated to the second member of the second binding pair, and
d) forming the complex by combining the synthesized components.

Reported herein is a pharmaceutical formulation comprising the multispecific binding molecule or the bispecific antibody as reported herein and optionally a pharmaceutically acceptable carrier.

Reported herein is the multispecific binding molecule or the bispecific antibody as reported herein for use as a medicament.

Reported herein is the multispecific binding molecule or the bispecific antibody as reported herein for use in treating cancer.

Reported herein is the use of the multispecific binding molecule or the bispecific antibody as reported herein in the manufacture of a medicament.

In one example the medicament is for treatment of cancer.

Reported herein is a method of treating an individual having cancer comprising administering to the individual an effective amount of the multispecific binding molecule or the bispecific antibody as reported herein.

### Detailed Description of the Invention

### I. Definitions

The articles "a" and "an" are used herein to refer to one or to more than one (i.e., to at least one) of the grammatical object of the article. By way of example, "an antibody" means one antibody or more than one antibody.

An "acceptor human framework" is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

The term "affinity" denotes the strength of the sum total of non-covalent interactions between a single binding site of a molecule (e.g. a polypeptide or an antibody) and its binding partner (e.g. a target or an antigen). Unless indicated otherwise, as used herein, "binding affinity" refers to intrinsic binding affinity which reflects a 1:1 interaction between members of a binding pair (e.g. in a polypeptide-polynucleotide-complex, or between a polypeptide and its target, or between an antibody and its antigen). The affinity of a molecule X for its partner Y can generally be represented by the dissociation constant (kD). Affinity can be measured by common methods known in the art, such as surface plasmon resonance and also including those reported herein.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The term "caged" denotes that the effector is protected with a protecting group which has a controlled half-life in serum and body fluids. The protecting group can be enzymatically cleaved by endogenous enzymes. The protecting group can be removed, cleaved, degraded, enzymatically digested or metabolized by a second effector which is externally administered by injection or given orally, such as ascorbic acid. The caged effector molecules can be activated by enzymes which are naturally occurring in body fluids. The caged effector moieties can be activated by reducing agents also occurring in body fluids such as ascorbic acid.

The term "effector moiety" denotes any molecule or combination of molecules whose activity it is desired to be delivered (in)to and/or localize at a cell. Effector moieties include, but are not limited to labels, cytotoxins (e.g. Pseudomonas exotoxin, ricin, abrin, Diphtheria toxin, and the like), enzymes, growth factors, transcription factors, drugs, radionuclides, ligands, antibodies, antibody Fc-regions, liposomes, nanoparticles, viral particles, cytokines, and the like.

The term "antibody" herein is used in the broadest sense and encompasses various antibody structures, including but not limited to monoclonal antibodies and antibody fragments so long as they exhibit the desired antigen-binding activity.

The term "antibody fragment" denotes a fragment of a complete or full length antibody that retains the ability to specifically bind to an antigen. Examples of antibody fragments include but are not limited to Fv, FAB, FAB', FAB'-SH, F(ab')₂; diabodies; linear antibodies; single-chain antibody molecules (e.g. scFv). For a review of certain antibody fragments, see Hudson, P.J., et al., Nat. Med. 9 (2003) 129-134. In more detail encompassed within the term "antibody fragment" is (i) a FAB fragment, i.e. a monovalent antibody fragment consisting of the VL, VH, CL and CH1 domains (for discussion of FAB and F(ab')₂ fragments comprising salvage receptor binding epitope residues and having increased in vivo half-life, see US 5,869,046), (ii) a F(ab')2 fragment, i.e. a bivalent fragment comprising two FAB fragments linked by a disulfide bridge at the hinge region, (iii) a Fd fragment consisting of the VH and CH1 domains, (iv) a Fv fragment consisting of the VL and VH domains of a single arm of an antibody (see, e.g., Plueckthun, in The Pharmacology of Monoclonal Antibodies, vol. 113, Rosenburg and Moore (eds.), (Springer-Verlag, New York), (1994) pp. 269-315, WO 93/16185, US 5,571,894, US 5,587,458), (v) a dAb fragment (see e.g. Ward, E.S., et al., Nature 341 (1989) 544-546), which consists of a VH domain, and (vi) an isolated complementarity determining region (CDR). Furthermore, although the two domains of the Fv fragment, VL and VH, are coded by separate genes, they can be joined, using recombinant methods, by a synthetic linker that enables them to be made as a single protein chain in which the VL and VH regions pair to form monovalent molecules (known as single chain Fv (scFv), see e.g., Bird, R.E., et al., Science 242 (1988) 423-426; Huston, J.S., et al., Proc. Natl. Acad. Sci. USA 85 (1988) 5879-5883). These antibody fragments can be obtained using conventional techniques known to those with skill in the art and can be screened for their binding properties in the same manner as are intact antibodies.

An "antibody that binds to the same epitope" as a reference antibody refers to an antibody that blocks binding of the reference antibody to its antigen in a competition assay by 50 % or more, and conversely, the reference antibody blocks binding of the antibody to its antigen in a competition assay by 50 % or more.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

A "chemotherapeutic agent" is a chemical compound useful in the treatment of cancer. Examples of chemotherapeutic agents include alkylating agents such as thiotepa and cyclosphosphamide (CYTOXAN^{™}); alkyl sulfonates such as busulfan, improsulfan and piposulfan; aziridines such as benzodopa, carboquone, meturedopa, and uredopa; ethylenimines and methylamylamines including altretamine, triethylenemelamine, trietylenephosphoramide, triethylenethiophosphoramide and trimethylomelamine; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitroureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxanes, e.g. paclitaxel (TAXOL®, Bristol-Myers Squibb Oncology, Princeton, NJ) and docetaxel (TAXOTERE®, Rh6ne-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-II; 35 topoisomerase inhibitor RFS 2000; difluoromethylornithine (DMFO); retinoic acid; esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

An "anti-angiogenic agent" refers to a compound which blocks, or interferes with to some degree, the development of blood vessels. The anti-angiogenic agent may, for instance, be a small molecule or an antibody that binds to a growth factor or growth factor receptor involved in promoting angiogenesis. The anti-angiogenic factor is in one embodiment an antibody that binds to Vascular Endothelial Growth Factor (VEGF).

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-a and -P; mullerian-inhibiting substance; mouse gonadotropin-associated peptide; inhibin; activin; vascular endothelial growth factor; integrin; thrombopoietin (TPO); nerve growth factors such as NGF-p; platelet growth factor; transforming growth factors (TGFs) such as TGF-a and TGF-p; insulin-like growth factor-I and -II; erythropoietin (EPO); osteoinductive factors; interferons such as interferon-a, -P, and -y; colony stimulating factors (CSFs) such as macrophage-CSF (M-CSF); granulocyte-macrophage-CSF (GM-CSF); and granulocyte-CSF (GCSF); interleukins (ILs) such as IL-I, IL-la, IL-2, IL-3, IL-4, IL-5, IL-6, IL-7, IL-8, IL-9, IL-IO, IL-II, IL-12; a tumor necrosis factor such as TNF-α or TNF-P; and other polypeptide factors including LIF and kit ligand (KL). As used herein, the term cytokine includes proteins from natural sources or from recombinant cell culture and biologically active equivalents of the native sequence cytokines.

The term "fMLP" denotes the tripeptide consisting of N-formylmethionine, leucine and phenylalanine. In one embodiment the effector moiety is fMLP or a derivative thereof.

The term "phenotype of a patient" denotes the composition of cell surface receptors in a kind of cells from a patient. The composition can be a qualitative as well as a quantitative composition. The cell for which the genotype is determined/given can be a single cell or a sample comprising cells.

The term "prodrug" refers to a precursor or derivative form of a pharmaceutically active substance that is less cytotoxic to tumor cells compared to the parent drug and is capable of being enzymatically activated or converted into the more active parent form. See, e.g., Wilman, "Prodrugs in Cancer Chemotherapy" Biochemical Society Transactions, Vol. 14, 615th Meeting Belfast (1986) pp. 375-382 and Stella, et al., "Prodrugs: A Chemical Approach to Targeted Drug Delivery", Directed Drug Delivery, Borchardt, et al., (eds.), pp. 247-267, Humana Press (1985). The prodrugs that can be used as effector moiety include, but are not limited to, phosphate-containing prodrugs, thiophosphate-containing prodrugs, sulfate-containing prodrugs, peptide-containing prodrugs, D-amino acid-modified prodrugs, glycosylated prodrugs, b-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described herein.

The term "cytotoxic moiety" refers to a substance that inhibits or prevents a cellular function and/or causes cell death or destruction. Cytotoxic agents include, but are not limited to, radioactive isotopes (e.g., At²¹¹ , I¹³¹, I¹²⁵, Y⁹⁰, Re , Re , Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed herein.

An "effective amount" of an agent, e.g., a pharmaceutical formulation, refers to an amount effective, at dosages and for periods of time necessary, to achieve the desired therapeutic or prophylactic result.

The term "Fc-region" is used herein to define a C-terminal region of an immunoglobulin heavy chain that contains at least a portion of the constant region. The term includes native sequence Fc-regions and variant Fc-regions. In one embodiment, a human IgG heavy chain Fc-region extends from Cys226, or from Pro230, to the carboxyl-terminus of the heavy chain. However, the C-terminal lysine (Lys447) of the Fc-region may or may not be present. Unless otherwise specified herein, numbering of amino acid residues in the Fc-region or constant region is according to the EU numbering system, also called the EU index, as described in Kabat, et al., Sequences of Proteins of Immunological Interest, 5th Ed. Public Health Service, National Institutes of Health, Bethesda, MD (1991).

The term "framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody", "intact antibody", and "whole antibody" are used herein interchangeably to refer to an antibody having a structure substantially similar to a native antibody structure or having heavy chains that contain an Fc-region as defined herein. Such an antibody generally comprises two heavy chains and two light chains.

A "human antibody" is an antibody which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g. CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3) (see Chothia, C. and Lesk, A.M., J. Mol. Biol. 196 (1987) 901-917). Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of L1, 50-65 of H2, and 95-102 of H3 (see Kabat, et al., Sequences of Proteins of Immunological Interest, 5th Ed. Public Health Service, National Institutes of Health, Bethesda, MD (1991)). With the exception of CDR1 in VH, CDRs generally comprise the amino acid residues that form the hypervariable loops. CDRs also comprise "specificity determining residues" or "SDRs," which are residues that contact the antigen. SDRs are contained within regions of the CDRs called abbreviated-CDRs, or α-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of L1, 50-58 of H2, and 95-102 of H3 (see Almagro, J.C. and Fransson, J., Front. Biosci. 13 (2008) 1619-1633). Unless otherwise indicated, HVR residues and other residues in the variable domain (e.g., FR residues) are numbered herein according to Kabat et al., supra.

An "immunoconjugate" is an antibody or antibody fragment conjugated to one or more non-antibody derived molecules, including but not limited to a member of a binding pair, a nucleic acid, or an effector moiety.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

The term "monoclonal antibody" refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies or monoclonal antibody fragments to be used in the complex as reported herein may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The term "monovalent binding polypeptide" or "monovalent binding antibody fragment" denotes a molecule that has only a single site or region for binding to its target or antigen. Examples of monovalent binding polypeptides are peptides, peptide mimetics, aptamers, small organic molecules (inhibitors capable of specific binding to a target polypeptide), darpins, ankyrin repeat proteins, Kunitz type domain, single domain antibodies (see: Hey, T., et al., Trends Biotechnol. 23 (2005) 514-522), (natural) ligands of a cell surface receptor, monovalent fragments of full length antibodies, and the like. For example a full length antibody has two bindings sites for its target and is, thus, bivalent, where as a scFv or FAB' antibody fragment has only one binding site for its target and is, thus, monovalent. In case monovalent antibodies or antibody fragments are used as a polypeptide this site is called the paratope.

The term "naked antibody" or "naked antibody fragment" denotes an antibody or antibody fragment that is not conjugated to a non-antibody moiety (e.g. a nucleic acid, or a cytotoxic moiety, or radiolabel).

"Native antibodies" refer to naturally occurring immunoglobulin molecules with varying structures. For example, native IgG antibodies are heterotetrameric glycoproteins of about 150,000 Daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

The term "polynucleotide" or "nucleic acid sequence" denotes a short, generally single stranded, polynucleotides that comprise at least 8 nucleotides and at most about 1000 nucleotides. In one embodiment a polynucleotide has a length of at least 9, or 10, or 11, or 12, or 15, or 18, or 21, or 24, or 27, or 30 nucleotides. In one embodiment a polynucleotide has a length of no more than 200, or 150, or 100, or 90, or 80, or 70, or 60, or 50, or 45, or 40, or 35, or 30 nucleotides. In a further embodiment a polynucleotide has a length of at least 9, or 10, or 11, or 12, or 15, or 18, or 21, or 24, or 27, or 30 nucleotides and of no more than 200, or 150, or 100, or 90, or 80, or 70, or 60, or 50, or 45, or 40, or 35, or 30 nucleotides.

The term "L-polynucleotide" denotes a nucleic acid that comprises more than 50 % L-nucleotides as monomeric building blocks, such as L-DNA. In one embodiment an L-polynucleotide comprises only L-nucleotides. The number of nucleotides of such a L-polynucleotides it is to be understood to range from one L-nucleotide to any number. However, in one embodiment the number or L-nucleotides is at least 10, or 15, or 20, or 25, or 30, or 35, or 40, or 45, or 50, or 55, or 60, or 70, or 80, or 90, or 100 nucleotides. The L-polynucleotides are made of L-A, L-G, L-C, L-U, L-T and combinations thereof, whereby L-A denotes L-ribose-adenine etc. The L-polydeoxynucleotides are made of L-dA, L-dG, L-dC, L-dU, L-dT and combinations thereof, whereby L-dA denotes L-deoxyribose-adenine etc.

The term "polynucleotide linker" denotes a moiety linking two nucleotide sequences together. In one embodiment the polynucleotide linker is a polynucleotide. In one embodiment the polynucleotide linker comprises at least one polynucleotide and at least one non-polynucleotide. The non-polynucleotide can be a polypeptide, a polymer, or a polysaccharide. In one embodiment the polynucleotide linker comprises a polynucleotide of from 10 to 30 nucleotides in length and a linear poly (ethylene glycol).

A "polypeptide" is a polymer consisting of amino acids joined by peptide bonds, whether produced naturally or synthetically. Polypeptides of less than about 20 amino acid residues may be referred to as "peptides", whereas molecules consisting of two or more polypeptides or comprising one polypeptide of more than 100 amino acid residues may be referred to as "proteins". A polypeptide may also comprise non-amino acid components, such as carbohydrate groups, metal ions, or carboxylic acid esters. The non-amino acid components may be added by the cell, in which the polypeptide is expressed, and may vary with the type of cell. Polypeptides are defined herein in terms of their amino acid backbone structure or the nucleic acid encoding the same. Additions such as carbohydrate groups are generally not specified, but may be present nonetheless.

A "polypeptide epitope" denotes the binding site on a polypeptidic target bound by a corresponding monovalent binding polypeptide. It is generally composed of amino acids. The binding polypeptide either binds to a linear epitope, i.e. an epitope consisting of a stretch of 5 to 12 consecutive amino acids, or the binding polypeptide binds to a three-dimensional structure formed by the spatial arrangement of several short stretches of the polypeptidic target. Three-dimensional epitopes recognized by a binding polypeptide, e.g. by the antigen recognition site or paratope of an antibody or antibody fragment, can be thought of as three-dimensional surface features of an antigen molecule. These features fit precisely (in)to the corresponding binding site of the binding polypeptide and thereby binding between the binding polypeptide and its target is facilitated.

The term "specifically binding" denotes that the polypeptide or antibody or antibody fragments binds to its target with an dissociation constant (KD) of 10⁻⁸ M or less, in one embodiment of from 10⁻⁵ M to 10⁻¹³ M, in one embodiment of from 10⁻⁵ M to 10⁻¹⁰ M, in one embodiment of from 10⁻⁵ M to 10⁻⁷ M, in one embodiment of from 10⁻⁸ M to 10⁻¹³ M, or in one embodiment of from 10⁻⁹ M to 10⁻¹³ M. The term is further used to indicate that the polypeptide does not specifically bind to other biomolecules present, i.e. it binds to other biomolecules with a dissociation constant (KD) of 10⁻⁴ M or more, in one embodiment of from 10⁻⁴ M to 1 M.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, complexes as reported herein are used to delay development of a disease or to slow the progression of a disease.

The term "variable region" or "variable domain" refers to the domain of an antibody heavy or light chain that is involved in binding the antibody to its antigen. The variable domains of the heavy chain and light chain (VH and VL, respectively) of a native antibody generally have similar structures, with each domain comprising four conserved framework regions (FRs) and three hypervariable regions (HVRs) (see, e.g., Kindt, et al., Kuby Immunology, 6th ed., W.H. Freeman and Co., page 91 (2007)). A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively (see, e.g., Portolano, S., et al., J. Immunol. 150 (1993) 880-887, Clarckson, T., et al., Nature 352 (1991) 624-628).

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

### II. Tailor-made multispecific binding molecules

In most cell based diseases the targeting of the disease-related cells via antibody based binding of receptor molecules is one promising approach. However, the expression level of clinically relevant surface receptors (=target) varies from patient to patient and efficacy of standardized antibody based drugs is thus very different. This applies specifically for bi- and multispecific binding molecules whose mode of action is to target two different epitopes/receptors simultaneously.

One promising approach is to design a drug (here a bi- or multispecific binding molecule) specifically for the particular/individual situation of the respective patient.

Based on expression profile data of clinically relevant surface receptors on disease-associated cells of a patient a series of binding entities (for example Fab fragments) are specifically chosen from a library and combined to a multispecific binding molecule as the patient specific drug. These selected binding molecules are specifically chosen with respect to the respective disease-associate cell such as e.g. a tumor cell based e.g. on the expression level of surface receptors and, thus, the need and phenotype of the individual patient.

Variations in length of the linker that combines/connects the binding entities enables the choice of the right flexibility and distances which might be required for simultaneously binding of both binding entities and, thus, for selectivity and/or specificity and/or efficacy.

In addition, payloads, such as effector functions or toxins, can be added by specific hybridization of the payload with the linker. This possibility further increases the breath of therapeutic applications.

Selected patient specific multispecific binding molecules can be tested in various cellular in vitro assays/cell samples for relevant criteria (for example optimal binding/binding partners, optimal linker length etc.):
- determining the phosphorylation status of phospho tyrosine kinases
- determining JNK inhibition
- determining molecule induced apoptosis
- binding assay performed with monospecific vs. multispecific binding molecule
- determining of proliferation inhibition

With such an approach the generation of tailor-made and, thus, highly efficient therapeutic molecules is possible. These molecules will have reduced side effects by improved targeting/delivery (e.g. payload for tumor cells) and improved targeting to target cell is based on higher selectivity and specificity of targeting component (comprising at least two binding molecules).

The higher selectivity and specificity of multispecific binding molecule is due to simultaneous binding (avidity) by the combination of two "low affinity" binders, which reduces possible "off-target" bindings.

Each cell from an individual is different in view of the expressed cell surface molecules, such as receptors, in number and kind. This is especially true for cancer cells and non-cancer cells. Thus, a cell can be characterized by the cell surface molecules presented.

Such a characterization can be effected by in vitro and in vivo based cell imaging techniques. In vivo imaging techniques include e.g. optical imaging, molecular imaging, fluorescence imaging, bioluminescence Imaging, MRI, PET, SPECT, CT, and intravital microscopy. In vitro imaging techniques include e.g. immunohistochemical staining of patient cells with e.g. fluorescently labeled antibodies recognizing specific cell surface markers and analysis of the fluorescence signals by microscopy. Alternatively the genotype/phenotype of the cells can be analyzed after staining with labeled therapeutic or diagnostic antibodies using FACS-based methods.

In one embodiment the genotype/phenotype of patient-derived cells is determined by a FACS-based method. In one embodiment the cell surface markers are determined by using fluorescently labeled diagnostic or therapeutic antibodies. In one embodiment fluorescently labeled therapeutic antibodies are used.

Certain diseases can be correlated with a change in the number of specific cell surface molecules or with occurrence of a new cell surface molecule.

Individuals affected by such a disease will display within certain ranges a disease and/or an individual-specific cell surface marker pattern.

This has to be taken into consideration in order to provide to such an individual a tailor-made, targeted therapeutic.

A number of therapeutic antibodies directed against cell surface molecules and their ligands are known which can be used for the selection and construction of tailor-made multi-specific targeting entities, such as Rituxan/MabThera/Rituximab, 2H7/Ocrelizumab, Zevalin/Ibrizumomab, Arzerra/Ofatumumab (CD20), HLL2/Epratuzumab, Inotuzomab (CD22), Zenapax/Daclizumab, Simulect/Basiliximab (CD25), Herceptin/Trastuzumab, Pertuzumab (Her2/ERBB2), Mylotarg/Gemtuzumab (CD33), Raptiva/Efalizumab (Cd11a), Erbitux/Cetuximab (EGFR, epidermal growth factor receptor), IMC-1121B (VEGF receptor 2), Tysabri/Natalizumab (α4-subunit of α4β1 and α4β7 integrins), ReoPro/Abciximab (gpIIb-gpIIa and αvβ3-integrin), Orthoclone OKT3/Muromonab-CD3 (CD3), Benlysta/Belimumab (BAFF), Tolerx/Oteliximab (CD3), Soliris/Eculizumab (C5 complement protein), Actemra/Tocilizumab (IL-6R), Panorex/Edrecolomab (EpCAM, epithelial cell adhesion molecule), CEACAM5/Labetuzumab (CD66/CEA, carcinoembryonic antigen), CT-11 (PD-1, programmed death-1 T-cell inhibitory receptor, CD-d279), H224G11 (c-Met receptor), SAR3419 (CD19), IMC-A12/Cixutumumab (IGF-1R, insulin-like growth factor 1 receptor), MEDI-575 (PDGF-R, platelet-derived growth factor receptor), CP-675, 206/Tremelimumab (cytotoxic T lymphocyte antigen 4), RO5323441 (placenta growth factor or PGF), HGS1012/Mapatumumab (TRAIL-R1), SGN-70 (CD70), Vedotin(SGN-35)/Brentuximab (CD30), and ARH460-16-2 (CD44).

For the determination of the cell surface markers present in a sample of e.g. a patient, different methods are known. One exemplary method is based on fluorescence activated cell sorting (FACS), in particular, the analysis of specifically stained and sorted cell populations. In this method the phenotyping of the sample (cell population) is achieved by analyzing individual cells with respect to the presented cell surface markers using fluorescently labeled antibodies directed against these markers optionally including the statistical distribution of surface markers in the cell population. It is especially suitable to use therapeutic antibodies that have been labeled with a fluorescent label for this purpose as therewith it is ensured that the later tailor-made multispecific binding molecule will bind to the same epitope as the diagnostic antibody. The multispecific binding molecules/bispecific antibodies as reported herein can be used in the preparation of medicaments for the treatment of e.g. an oncologic disease, a cardiovascular disease, an infectious disease, an inflammatory disease, an autoimmune disease, a metabolic (e.g., endocrine) disease, or a neurological (e.g. neurodegenerative) disease. Exemplary non-limiting examples of these diseases are Alzheimer's disease, non-Hodgkin's lymphomas, B-cell acute and chronic lymphoid leukemias, Burkitt lymphoma, Hodgkin's lymphoma, hairy cell leukemia, acute and chronic myeloid leukemias, T-cell lymphomas and leukemias, multiple myeloma, glioma, Waldenstrom's macroglobulinemia, carcinomas (such as carcinomas of the oral cavity, gastrointestinal tract, colon, stomach, pulmonary tract, lung, breast, ovary, prostate, uterus, endometrium, cervix, urinary bladder, pancreas, bone, liver, gall bladder, kidney, skin, and testes), melanomas, sarcomas, gliomas, and skin cancers, acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, diabetes mellitus, Henoch-Schonlein purpura, post-streptococcal nephritis, erythema nodosum, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitis obliterans, Sjogren's syndrome, primary biliary cirrhosis, Hashimoto's thyroiditis, thyrotoxicosis, scleroderma, chronic active hepatitis, polymyositis/dermatomyositis, polychondritis, pemphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis, or fibrosing alveolitis.

A number of cell surface markers and their ligands are known. For example cancer cells have been reported to express at least one of the following cell surface markers and or ligands, including but not limited to, carbonic anhydrase IX, alpha-fetoprotein, alpha-ctinin-4, A3 (antigen specific for A33 antibody), ART-4, B7, Ba-733, BAGE, BrE3-antigen, CA125, CAMEL, CAP-1, CASP-8/m, CCCL19, CCCL21, CD1, CD1a, CD2, CD3, CD4, CDS, CD8, CD1-1A, CD14, CD15, CD16, CD18, CD19, CD20, CD21, CD22, CD23, CD25, CD29, CD30, CD32b, CD33, CD37, CD38, CD40, CD40L, CD45, CD46, CD54, CD55, CD59, CD64, CD66a-e, CD67, CD70, CD74, CD79a, CD80, CD83, CD95, CD126, CD133, CD138, CD147, CD154, CDC27, CDK-4/m, CDKN2A, CXCR4, CXCR7, CXCL12, HIF-1-alpha, colon-specific antigen-p (CSAp), CEA (CEACAM5), CEACAM6, c-met, DAM, EGFR, EGFRvIII, EGP-1, EGP-2, ELF2-M, Ep-CAM, Flt-1, Flt-3, folate receptor, G250 antigen, GAGE, GROB, HLA-DR, HM1.24, human chorionic gonadotropin (HCG) and its subunits, HER2/neu, HMGB-1, hypoxia inducible factor (HIF-1), HSP70-2M, HST-2or 1a, IGF-1R, IFN-gamma, IFN-alpha, IFN-beta, IL-2, IL-4R, IL-6R, IL-13R, IL-15R, IL-17R, IL-18R, IL-6, IL-8, IL-12, IL-15, IL-17, IL-18, IL- 25, insulin-like growth factor-1 (IGF-1), KC4-antigen, KS-1-antigen, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5, MUM-1/2, MUM-3, NCA66, NCA95, NCA90, pancreatic cancer mucin, placental growth factor, p53, PLAGL2, prostatic acid phosphatase, PSA, PRAME, PSMA, P1GF, ILGF, ILGF-1R, IL-6, IL-25, RS5, RANTES, T101, SAGE, S100, survivin, survivin-2B, TAC, TAG-72, tenascin, TRAIL receptors, TNF-alpha, Tn-antigen, Thomson-Friedenreich antigens, tumor necrosis antigens, VEGFR, ED-B fibronectin, WT-1, 17-lA-antigen, complement factors C3, C3a, C3b, C5a, C5, an angiogenesis marker, bcl-2, bcl-6, Kras, cMET, an oncogene marker and an oncogene product (see, e.g., Sensi et al., Clin. Cancer Res. 12 (2006) 5023-5032; Parmiani et al, J. Immunol. 178 (2007) 1975-1979; Novellino et al., Cancer Immunol. Immunother. 54 (2005) 187-207).

Thus, antibodies recognizing specific cell surface receptors including their ligands can be used for specific and selective targeting and binding to a number/multitude of cell surface markers that are associated with a disease. A cell surface marker is a polypeptide located on the surface of a cell (e.g. a disease-related cell) that is e.g. associated with signaling event or ligand binding.

In one example, for the treatment of cancer/tumors multispecific binding molecules/bispecific antibodies are used that target tumor-associated antigens, such as those reported in Herberman, "Immunodiagnosis of Cancer", in Fleisher ed., "The Clinical Biochemistry of Cancer", page 347 (American Association of Clinical Chemists, 1979) and in US 4,150,149; US 4,361,544; and US 4,444,744.

Reports on tumor associated antigens (TAAs) include Mizukami et al., Nature Med. 11 (2005) 992-997; Hatfield et al., Curr. Cancer Drug Targets 5 (2005) 229-248; Vallbohmer et al., J. Clin. Oncol. 23 (2005) 3536-3544; and Ren et al., Ann. Surg. 242 (2005) 55-63).

Where the disease involves a lymphoma, leukemia or autoimmune disorder, targeted antigens may be selected from the group consisting of CD4, CD5, CD8, CD14, CD15, CD19, CD20, CD21, CD22, CD23, CD25, CD33, CD37, CD38, CD40, CD40L, CD46, CD54, CD67, CD74, CD79a, CD80, CD126, CD138, CD154, CXCR4, B7, MUC1 or 1a, HM1.24, HLA-DR, tenascin, VEGF, P1GF, ED-B fibronectin, an oncogene, an oncogene product (e.g., c-met or PLAGL2), CD66a-d, necrosis antigens, IL-2, T101, TAG, IL-6, MIF, TRAIL-R1 (DR4) and TRAIL-R2 (DR5).

A number of bispecific antibodies are known directed against two different targets such as BCMA/CD3, different antigens of the HER family in combination (EGFR, HER2, HER3), CD19/CD3, IL17RA/IL7R, IL-6/IL-23, IL-1-beta/IL-8, IL-6 or IL-6R/ IL-21 or IL-21R, first specificity directed to a glycoepitope of an antigen selected from the group consisting of Lewis x-, Lewis b- and Lewis y-structures, Globo H-structures, KH1, Tn-antigen, TF-antigen and carbohydrate structures of Mucins, CD44, glycolipids and glycosphingolipids, such as Gg3, Gb3, GD3, GD2, Gb5, Gm1, Gm2, sialyltetraosylceramide and a second specificity directed to an ErbB receptor tyrosine kinase selected from the group consisting of EGFR, HER2, HER3 and HER4, GD2 in combination with a second antigen binding site is associated with an immunological cell chosen from the group consisting of T-lymphocytes NK cell, B-lymphocytes, dendritic cells, monocytes, macrophages, neutrophils, mesenchymal stem cells, neural stem cells, ANG2/VEGF, VEGF/PDGFR-beta, Vascular Endothelial Growth Factor (VEGF) acceptor 2/CD3, PSMA/CD3, EPCAM/CD3, combinations of antigens selected from a group consisting of VEGFR-1, VEGFR-2, VEGFR-3, FLT3, c-FMS/CSF1R, RET, c-Met, EGFR, Her2/neu, HER3, HER4, , IGFR, PDGFR, c-KIT, BCR, integrin and MMPs with a water-soluble ligand is selected from the group consisting of VEGF, EGF, PIGF, PDGF, HGF, and angiopoietin, ERBB-3/C-MET, ERBB-2/C-MET, EGF receptor 1/CD3, EGFR/HER3, PSCA/CD3, C-MET/CD3, ENDOSIALIN/CD3, EPCAM/CD3, IGF-1R/CD3, FAPALPHA/CD3, EGFR/IGF-1R, IL 17A/F, EGF receptor 1/CD3, and CD19/CD16.

Thus, it has been found that by using a modular approach as reported herein tailor-made bispecific therapeutic antibodies can be provided. These antibodies are tailor-made with respect to cell surface molecules actually present on the cells of an individual in need of a treatment or with respect to ligands interacting with such a cell surface molecule. By determining the cell surface molecule status of an individual a tailor-made combination of therapeutic targets can be chosen.

With this tailor-made generation of bispecific therapeutics by combining 2 single therapeutic molecules for simultaneous targeting and binding to two different epitopes an additive/synergistic effect can be expected in comparison to the single therapeutic molecules.

By using already available monospecific therapeutic binding entities, such as those derived from therapeutic antibodies, a fast and easy production of the required multispecific binding molecule can be achieved.

These avidity engineered binding molecules/antibodies can bind to two or more cell surface markers present on a single cell. This binding is only avid if all/both binding entities simultaneously bind to the cell. For this purpose medium to high affine antibodies are especially suited. This allows also on the other hand to exclude less specific combinations of binding specificities during a screening process.

### The "Combimatrix" approach

It is desirable to combine a first binding entity, such as an antibody Fab fragment, with another specific binding entity, such as a second antibody Fab fragment. In addition it is possible to screen, whether a first binding entity shows better properties when linking it to a number of different other binding entities. Using a so-called Combimatrix approach, a multitude of combinations of binding entities can be addressed in an easy way. It has to be pointed out that the second binding entities can either bind to different targets/epitopes/antigens, or can bind to the same antigen but to different epitopes, or can bind to the same epitope but be different variants of a single binding entity (e.g. humanization candidates).

In this scenario, an automated platform can perform the tasks to pipette, purify and combine the binding entities and their reactions or derivatives. Any platform that uses e.g. 96-well plates or other high throughput formats is suitable, such as an Eppendorf epMotion 5075vac pipetting robot.

First, cloning of the binding entity (such as an antibody Fab fragment) encoding constructs is performed. The plasmid with the binding entity encoding nucleic acid is usually obtained by gene synthesis, whereby the C-terminal region of the encoded binding entity contains a sortase-motive and a His-tag. The plasmids are individually transferred into a separate well of a multi-well plate (a whole plate can be loaded). Thereafter, the plasmids are digested with a restriction enzyme mix that cuts out the binding entity-coding region. It is desirable to design all gene synthesis in a way that only one restriction enzyme mix is needed for all plasmids. Afterwards, an optional cleaning step yields purified DNA fragments. These fragments are ligated into a plasmid backbone that had been cut out of an acceptor vector with the same restriction mix as mentioned above. Alternatively, the cloning procedure can be performed by a SLIC-mediated cloning step. After ligation, the automated platforms transfers all ligation mixes into a further multi-well plate with competent E. coli cells (e.g. Top10 Multi Shot, Invitrogen) and a transformation reaction is performed. The cells are cultivated to the desired density. From an aliquot of the cultivation mixture glycerol stocks can be obtained. From the culture plasmid is isolated (e.g. using a plasmid isolation mini kit (e.g. NucleoSpin 96 Plasmid, Macherey& Nagel)). Plasmid identity is checked by digesting an aliquot with an appropriate restriction mix and SDS-gel electrophoresis (e.g. E-Gel 48, Invitrogen). Afterwards, a new plate can be loaded with an aliquot of the plasmid for performing a control sequencing reaction.

In the next step the binding entities are expressed. Therefore, HEK cells are seeded onto a multi-well plate (e.g. a 48-well-plate) and are transfected with the isolated plasmids (containing the binding entity-coding region in an appropriate backbone vector). Transfected HEK cells are cultivated for several days and harvested (e.g. by filtrating through a 1.2 µm and a 0.22 µm filter plate by using a vacuum station). Titers can be monitored by performing e.g. an ELISA.

The binding entities can be covalently linked to the respective members of oligonucleotide binding pairs using a sortase-mediated transpeptidation reaction. The binding entity and the sortase reaction mix are combined in a multi-well format. After incubation at 37°C for 4-16 h, the binding entity-oligonucleotide conjugates are harvested by using a negative His-tag selection procedure (the mixture is applied onto e.g. His MultiTrap HP plates (GE Healthcare) and filtrated, whereby all molecules that still have a His-tag are bound on the chromatography column, whereas all other molecules like the oligonucleotide conjugates are found in the filtrate; with the filtrate a buffer exchange should be made, e.g. by applying the binding entity-oligonucleotide conjugate onto an ultrafiltration membrane or by using a plate containing an affinity medium that is specific for the binding entity; after buffer exchange, which also removes excess free oligonucleotide, the binding entity-oligonucleotide conjugates can be linked to become a multispecific binding molecule.

The multispecific binding molecules are made using the Combimatrix approach, see Table below).

| | 1 | 2 | 3 | 4 | 5 | 6 | 7 | 8 | 9 | 10 | 11 |
|---|---|---|---|---|---|---|---|---|---|---|---|
| A | 1A | 2A | 3A | 4A | 5A | 6A | 7A | 8A | 9A | 10A | 11A |
| B | 1B | ... | ... | ... | ... | ... | ... | ... | ... | ... | ... |
| C | 1C | ... | ... | ... | ... | ... | ... | ... | ... | ... | ... |
| D | 1D | ... | ... | ... | ... | ... | ... | ... | ... | ... | ... |
| E | IE | ... | ... | ... | ... | ... | ... | ... | ... | ... | ... |
| F | IF | ... | ... | ... | ... | ... | ... | ... | ... | ... | ... |
| G | 1G | ... | ... | ... | ... | ... | ... | ... | ... | 10G | 11G |

In the first row of a multi-well plate different binding entity-oligonucleotide conjugates of equal molar concentrations are pipetted into each well (excluding first well of the first row), designated in arabic numbers (e.g. 1 to 11). In the first column of the same plate, different binding entity-oligonucleotide conjugates of equal molar concentrations are pipetted into each well (excluding first well of the first column), designated in letters (e.g. A to G). Thereafter all binding entity-oligonucleotide conjugates of the first row are combined with all binding entity-oligonucleotide conjugates of the first column (e.g. resulting in 77 combinations in a 96-well plate), designated by a combination of number and letter (e.g. 1A to 11 G). To all combinations a linker molecule in equal molar ratios to the binding entity-oligonucleotide conjugates and an appropriate buffer (e.g. PBS with 150 mM NaCl, 1.5 mM MgCl₂) is added. The linking reaction can be performed at room temperature or by denaturing the mixture at 60°C and then cooling down slowly. Afterwards, an optional purification step by e.g. size exclusion chromatography can be performed. The multispecific binding molecules are then ready for evaluation in cell-based assays.

### Methods as reported herein

One aspect as reported herein is a method for producing a bispecific antibody comprising the following steps
(i) determining surface makers present on the surface of a cell in a sample and selecting thereof a first surface marker and a second surface marker,
(ii) incubating (a) an antibody Fab fragment or a scFv antibody fragment conjugated to a first partner or member of a first binding pair, whereby the Fab fragment or scFv specifically binds to the first surface marker, (b) an antibody Fab fragment or a scFv antibody fragment conjugated to a first member of a second binding pair, whereby the Fab fragment or scFv antibody fragment specifically binds to the second surface marker, and (c) an enantiomeric DNA polynucleotide linker comprising at one of its termini the second member of the first binding pair and at the respective other terminus the second member of the second binding pair, and thereby producing the bispecific antibody.

One aspect as reported herein is a method for determining a combination of antigen binding sites comprising the following steps
(i) determining the binding specificity and/or affinity of a multitude of bispecific antibodies prepared by combining each member of a first multitude of antibody Fab fragments or scFv antibody fragments each linked to the same first member of a first binding pair with each member of a second multitude of antibody Fab fragments or scFv antibody fragments each linked to the same first member of a second binding pair, and an enantiomeric DNA polynucleotide linker comprising at one of its termini the second member of the first binding pair and at the respective other terminus the second member of the second binding pair,
   whereby the first multitude specifically binds to a first cell surface molecule and the second multitude specifically binds to a second cell surface molecule,
   and
(ii) choosing the bispecific antibody with suitable binding specificity and/or affinity and thereby determining a combination of antigen binding sites.

In one embodiment the bispecific antibody is a complex comprising
a) a first Fab fragment or scFv antibody fragment
   i) that specifically binds to a first cell surface marker, and
   ii) that is conjugated to a first member of a first binding pair,
b) a second Fab fragment or scFv antibody fragment
   i) that specifically binds to a second cell surface marker, and
   ii) that is conjugated to a first member of a second binding pair, and
c) a polynucleotide linker
   i) that is conjugated to the second member of the first binding pair, and
   ii) that is conjugated to the second member of the second binding pair.

The following are embodiments of all aspects as reported herein.

In one embodiment the complex is a non-covalent complex.

In one embodiment the complex further comprises an effector moiety that is conjugated to a polynucleotide that is complementary to at least a part of the polynucleotide linker.

In one embodiment the complex further comprises a further polypeptide i) that specifically binds to a second target, and ii) that is conjugated to a first member of a second binding pair, and the polynucleotide linker is conjugated to the second member of the second binding pair.

In one embodiment the complex further comprises an effector moiety conjugated to a polynucleotide that is i) complementary to at least a part of the polynucleotide that is conjugated to the first effector moiety and ii) not complementary to the polynucleotide linker.

In one embodiment the first and second Fab fragment or scFv antibody fragment bind to the same target and to non-overlapping epitopes thereon.

In one embodiment the polynucleotide linker comprises of from 8 to 1000 nucleotides. In one embodiment the polynucleotide linker comprises of from 10 to 500 nucleotides.

In one embodiment the enantiomeric DNA is L-DNA. In one embodiment the L-DNA is single stranded L-DNA (ss-L-DNA).

In one embodiment the effector moiety is selected from the group consisting of a binding moiety, a labeling moiety, and a biologically active moiety.

In one embodiment the polynucleotide linker is conjugated to the Fab fragment or scFv antibody fragment at its first or second terminus.

In one embodiment the polynucleotide linker is conjugated to two second members of two binding pairs, whereby the second member of the first binding pair is conjugated to the first terminus of the polynucleotide linker and the second member of the second binding pair is conjugated to the second terminus of the polynucleotide linker.

In one embodiment the first and second members of the first binding pair comprise the nucleic acid sequences of SEQ ID NO: 05 and SEQ ID NO: 08, respectively.

In one embodiment the first and second members of the second binding pair comprise the nucleic acid sequences of SEQ ID NO: 06 and SEQ ID NO: 07, respectively.

In one embodiment the method comprises the steps of:
a) synthesizing the first Fab fragment or scFv antibody fragment that specifically binds to a first cell surface marker and that is conjugated to a first member of a first binding pair,
b) synthesizing the second Fab fragment or scFv antibody fragment that specifically binds to a second cell surface marker and that is conjugated to a first member of a second binding pair,
c) synthesizing the polynucleotide linker that is conjugated to the second member of the first binding pair and that is conjugated to the second member of the second binding pair, and
d) forming the complex by combining the synthesized components.

### Polypeptide-polynucleotide-complex

Herein is reported a multispecific binding molecule, such as a bispecific antibody, that is a complex that comprises at least two components that are connected by a non-covalent interaction, whereby the components are more resistant to proteolytic and enzymatic degradation in vivo than isolated RNA or DNA, especially D-DNA. The complex has a high affinity for its target exploiting binding avidity and has a good solubility. The complex can be used for the delivery of one or more effector moieties to a target.

It has been found that a complex comprising a mixture of polypeptidic and polynucleotidic parts, especially L-polynucleotidic parts, fulfills these requirements and is especially suited for the delivery of an effector moiety in vivo.

If the cell to be targeted has at least two cell surface molecules the multispecific binding molecule (e.g. a bispecific antibody) as reported herein comprises a linker polynucleotide and two or more polypeptides (binding entities) that specifically bind to non-overlapping epitopes and it is constructed such that the linker polynucleotide has the optimal length for synergistic binding of the polypeptides specifically binding to these cell surface molecules.

Reported herein is a polypeptide-polynucleotide-complex of the formula:

(A - a':a - S- b:b' - B) - X(n)

or

(A - a':a - S- b:b' - B) : X(n),

wherein A as well as B is a binding entity that specifically binds to a target,
wherein a':a as well as b:b' is a binding pair, wherein a' and a and do not interfere with the binding of b to b' and vice versa,
wherein S is a linker polynucleotide,
wherein (: X) denotes an effector moiety bound either covalently or via a binding pair to at least one of a', a, b, b' or S,
wherein (n) is an integer,
wherein - represents a covalent bond, and
wherein: represents a non-covalent bond.

Also reported herein is a method for producing a polypeptide-polynucleotide-complex of the formula:

(A - a':a - S- b:b' - B) - X(n)

or

(A - a':a - S- b:b' - B) : X(n),

as outlined above comprising the steps of:
a) synthesizing A-a' and b'-B, respectively,
b) synthesizing the linker a - S - b, and
c) forming the complex of the formula,
wherein the effector moiety X is bound to at least one of a', a, b, b' or S in step a), b), or c).

Based on its individual components the complex as reported herein can be obtained according to standard procedures by hybridization between the members of the binding pair conjugated to the individual components of the complex.

In order to obtain a complex e.g. with 1:1:1 stoichiometry the complex can be separated by chromatography from other conjugation side-products. This procedure can be facilitated by using a dye labeled binding pair member and/or a charged linker. By using this kind of labeled and highly negatively charged binding pair member, mono conjugated binding entities/polypeptides are easily separated from non-labeled binding entities/polypeptides and binding entities/polypeptides which carry more than one linker, since the difference in charge and molecular weight can be used for separation. The fluorescent dye can be useful for purifying the complex from non-bound components, like a labeled monovalent binder.

Reported a method of producing a binding entity-polynucleotide-complex comprising the components
a) a binding entity, such as a polypeptide, that specifically binds to a target and that is conjugated to a first member of a binding pair,
b) a polynucleotide linker conjugated at its first terminus to the second member of the binding pair, and
c) an effector moiety conjugated to a polynucleotide that is complementary to at least a part of the polynucleotide linker,
comprising the steps of: a) synthesizing i) the binding entity specifically binding to a target and conjugated to a first member of a binding pair and ii) an effector moiety conjugated to a polynucleotide that is complementary to at least a part of the polynucleotide linker, respectively, b) synthesizing the polynucleotide linker conjugated at its first terminus to the second member of the binding pair, and c) forming the binding entity-polynucleotide-complex by hybridizing the synthesized components.

Reported herein is a method of producing a binding entity-polynucleotide-complex comprising the components
a) a first binding entity, such as a polypeptide, that specifically binds to a first target which is conjugated to a first member of a first binding pair,
b) a second binding entity, such as a polypeptide, that specifically binds to a second target which is conjugated to a first member of a second binding pair, and
c) a polynucleotide linker conjugated at its first terminus to the second member of the first binding pair and conjugated at its second terminus to the second member of the second binding pair,
comprising the steps of: a) synthesizing the first binding entity specifically binding to a first target which is conjugated to a first member of a first binding pair, and the second binding entity specifically binding to a second target which is conjugated to a first member of a second binding pair, respectively, and b) synthesizing the polynucleotide linker conjugated at its first terminus to the second member of the first binding pair and conjugated at its second terminus to the second member of the second binding pair, and c) forming the binding entity-polynucleotide-complex by hybridizing the synthesized components.

The complex can additionally contain one or several counter ions Y to equalize the charge. Examples of suitable negatively charged counter ions are halogenides, OH-, carbonate, alkylcarboxylate, e.g. trifluoroacetate, sulphate, hexafluorophosphate and tetrafluoroborate groups. Hexafluorophosphate, trifluoroacetate and tetrafluoroborate groups are especially suited. Other suited positively charged counter ions are monovalent cations such as alkaline metal ions and/or ammonium ions.

A full library of complexes as reported herein can easily be provided, analyzed and a suitable binding agent out of such library can be produced at large scale, as required.

The library refers to a set of complexes as reported herein, wherein the binding entity, the length of the polynucleotide linker is adjusted to best meet the requirements set out for the binding agent.

It is easily possible e.g. to first use a polynucleotide linker ladder spanning the whole spectrum of 1 nm to 100 nm and having steps that are about 10 nm apart. The linker length is then again easily further refined around the most appropriate length identified in the first round.

Herein is also reported a method for the selection of a binding entity-polynucleotide-complex from a library comprising a multitude of complexes with different polynucleotide linker length. In one example of this method several linker molecules with polynucleotide linkers of various lengths are synthesized and used in the formation of a complex as reported herein comprising polynucleotide linkers of variable length and those complexes are selected having an improvement in the K_{diss} of at least 5-fold over the better of the two monovalent polypeptide binders. Selection of a bivalent binding agent with the desired K_{diss} in one embodiment is performed by BIAcore-analysis as disclosed in the Examples.

Reported herein is a complex comprising
a) a binding entity (e.g. a polypeptide) that specifically binds to a first target and that is conjugated to a first single stranded L-DNA moiety,
b) a second binding entity (e.g. a polypeptide) that specifically binds to a second target and that is conjugated to a second single stranded L-DNA moiety, and
c) a linker that comprises at its first (or 3') terminus a first single stranded L-DNA linker moiety that is complementary to the first single stranded L-DNA moiety and that comprises at its second (or 5') terminus a second single stranded L-DNA linker moiety that is complementary to the second single stranded L-DNA moiety.

Reported herein is a complex comprising
a) an antibody FAB fragment or a scFv that specifically binds to a first target and that is conjugated to a first single stranded L-DNA moiety,
b) an antibody FAB fragment or a scFv that specifically binds to a second target and that is conjugated to a second single stranded L-DNA moiety, and
c) a linker that comprises at its first (or 3') terminus a first single stranded L-DNA linker moiety that is complementary to the first single stranded L-DNA moiety and that comprises at its second (or 5') terminus a second single stranded L-DNA linker moiety that is complementary to the second single stranded L-DNA moiety.

The first single stranded L-DNA moiety does not hybridize with the second single stranded L-DNA moiety and does not hybridize with the second single stranded L-DNA linker moiety. In turn, the second single stranded L-DNA moiety does not hybridize with the first single stranded L-DNA moiety and does not hybridize with the first single stranded L-DNA linker moiety.

In the following embodiments of all aspects as presented herein are given:
In one example the binding entity that specifically binds to a target is an antibody or antibody fragment. In one embodiment the antibody fragment is a Fab.

In one embodiment the first and/or second single stranded L-DNA moiety has a length of from 10 to 50 nucleotides. In one embodiment the length is of from 15 to 35 nucleotides. In one embodiment the length is of from 20 to 30 nucleotides.

In one embodiment the linker comprises a first single stranded L-DNA linker moiety, a second single stranded L-DNA linker moiety, and a single stranded docking moiety. In one embodiment the linker further comprises a linear non-nucleotide moiety. In one embodiment the linear non-nucleotide moiety is a polypeptide or a non-ionic polymer. In one embodiment the non-ionic polymer is linear poly (ethylene glycol). In one embodiment the linear poly (ethylene glycol) comprises of from 1 to 100 ethylene glycol units. In one embodiment the linear poly (ethylene glycol) comprises of from 1 to 50 ethylene glycol units. In one embodiment the linear poly (ethylene glycol) comprises of from 1 to 25 ethylene glycol units.

In one example the complex comprises
a) a polypeptide that specifically binds to a first target and that is conjugated to a first single stranded L-DNA moiety,
b) a polypeptide that specifically binds to a second target and that is conjugated to a second single stranded L-DNA moiety, and
c) a linker that comprises at its first (or 3') terminus a first single stranded L-DNA linker moiety that is complementary to the first single stranded L-DNA moiety, that comprises at its second (or 5') terminus a second single stranded L-DNA linker moiety that is complementary to the second single stranded L-DNA moiety, and that comprises a third single stranded L-DNA linker moiety between the first and second single stranded L-DNA moieties.

In one embodiment the linker comprises in 3' to 5' orientation
- a first single stranded L-DNA linker moiety that is complementary to the first single stranded L-DNA moiety,
- a docking single stranded L-DNA moiety, and
- a second single stranded L-DNA linker moiety that is complementary to the second single stranded L-DNA moiety.

The docking single stranded L-DNA moiety does not hybridize with the first single stranded L-DNA moiety or its complementary first single stranded linker moiety and it does not hybridize with the second single stranded L-DNA moiety or its complementary second single stranded L-DNA linker moiety.

In one embodiment the linker comprises in 3' to 5' orientation
- a first single stranded L-DNA linker moiety that is complementary to the first single stranded L-DNA moiety,
- a linear non-nucleotide moiety,
- a docking single stranded L-DNA moiety, and
- a second single stranded L-DNA linker moiety that is complementary to the second single stranded L-DNA moiety.

In one embodiment the linker comprises in 3' to 5' orientation
- a first single stranded L-DNA linker moiety that is complementary to the first single stranded L-DNA moiety,
- a docking single stranded L-DNA moiety,
- a non-nucleotide moiety, and
- a second single stranded L-DNA linker moiety that is complementary to the second single stranded L-DNA moiety.

In one embodiment the linker comprises in 3' to 5' orientation
- a first single stranded L-DNA linker moiety that is complementary to the first single stranded L-DNA moiety,
- a non-nucleotide moiety,
- a docking single stranded L-DNA moiety, and
- a second single stranded L-DNA linker moiety that is complementary to the second single stranded L-DNA moiety.

In one embodiment the linker comprises in 3' to 5' orientation
- a first single stranded L-DNA linker moiety that is complementary to the first single stranded L-DNA moiety,
- a first non-nucleotide moiety,
- a docking single stranded L-DNA moiety,
- a second non-nucleotide moiety,
- a second single stranded L-DNA linker moiety that is complementary to the second single stranded L-DNA moiety.

In one embodiment the first non-nucleotide moiety and the second non-nucleotide moiety are the same or different. In one embodiment the linear non-nucleotide moiety is a polypeptide or a non-ionic polymer. In one embodiment the non-ionic polymer is linear poly (ethylene glycol). In one embodiment the linear poly (ethylene glycol) comprises of from 1 to 100 ethylene glycol units. In one embodiment the linear poly (ethylene glycol) comprises of from 1 to 50 ethylene glycol units. In one embodiment the linear poly (ethylene glycol) comprises of from 1 to 25 ethylene glycol units.

### The binding entity component

Monoclonal antibody techniques allow for the production of specifically binding agents in the form of specifically binding monoclonal antibodies or fragments thereof. For creating monoclonal antibodies, or fragments thereof, techniques such as immunizing mice, rabbits, hamsters, or any other mammal with a polypeptide, i.e. the target of the antibody, or/and nucleic acid encoding the polypeptide can be used. Alternatively monoclonal antibodies, or fragments thereof, can be obtained by the use of phage libraries of scFv (single chain variable region), specifically human scFv (see e.g. US 5,885,793, WO 92/01047, WO 99/06587).

In one embodiment the binding entity that specifically binds to a target is a monovalent antibody fragment. In one embodiment the monovalent antibody fragment is derived from a monoclonal antibody.

Monovalent antibody fragments include, but are not limited to Fab, Fab'-SH, single domain antibody, F(ab')₂, Fv, and scFv fragments. Thus, in one embodiment the monovalent antibody fragment is selected from the group comprising Fab, Fab'-SH, single domain antibody, F(ab')₂, Fv, and scFv fragments.

In one example at least one of the binding entities of the complex as reported herein is a single domain antibody, or a Fab-fragment, or a Fab'-fragment of a monoclonal antibody.

In one embodiment both of the binding entities of the complex as reported herein are independently of each other a single domain antibody, or a Fab-fragment, or a Fab'-fragment of a monoclonal antibody.

In one embodiment both of the binding entities of the complex as reported herein are single domain antibodies, or Fab-fragments, or Fab'-fragments.

In one embodiment the targets or epitopes specifically bound by the binding entities do not overlap.

Diabodies are antibody fragments with two antigen-binding sites that may be bivalent or bispecific (see e.g. EP 0 404 097, WO 93/01161, Hudson, P.J., et al., Nat. Med. 9 (2003) 129-134, and Holliger, P., et al., Proc. Natl. Acad. Sci. USA 90 (1993) 6444-6448). Triabodies and tetrabodies are also described in Hudson, P.J., et al., Nat. Med. 9 (2003) 129-134.

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; US 6,248,516).

An Fv is a minimum antibody fragment that contains a complete antigen-binding site and is devoid of constant region. For a review of scFv, see, e.g., Plueckthun, in The Pharmacology of Monoclonal Antibodies, vol. 113, Rosenburg and Moore (eds.), (Springer-Verlag, New York, 1994), pp. 269-315, WO 93/16185, US 5,571,894, US 5,587,458. Generally, six hyper variable regions (HVRs) confer antigen-binding specificity to an antibody. However, even a single variable domain (or half of an Fv comprising only three HVRs specific for an antigen) has the ability to recognize and bind its antigen.

In one example the monovalent antibody fragments is a two-chain Fv species consisting of a dimer of one heavy- and one light-chain variable domain in tight, non-covalent association.

In one embodiment the monovalent antibody fragments is a single-chain Fv (scFv) species consisting of one heavy-chain and one light-chain variable domain covalently linked by a flexible peptide linker.

A Fab fragment of an antibody contains the heavy-chain and light-chain variable domains as well as the constant domain of the light chain and the first constant domain (CH1) of the heavy chain.

A Fab' fragments differ from a Fab fragment by the addition of a few residues at the carboxy terminus of the heavy chain CH1 domain including one or more cysteines from the antibody hinge region.

Fab'-SH denotes a Fab' in which the cysteine residue(s) of the constant domains bear a free thiol group.

Various techniques have been developed for the production of antibody fragments. Traditionally, antibody fragments can be obtained via proteolytic digestion of full length antibodies (see, e.g., Morimoto, K., et al., J. Biochem. Biophys. Meth. 24 (1992) 107-117, Brennan, M., et al., Science 229 (1985) 81-83). For example, papain digestion of full length antibodies results in two identical antigen-binding fragments, called "Fab" fragments, each with a single antigen-binding site, and a residual "Fc" fragment, whose name reflects its ability to crystallize readily. For a review of certain antibody fragments, see Hudson, P.J., et al., Nat. Med. 9 (2003) 129-134.

Antibody fragments can also be produced directly by recombinant means. Fab, Fv and scFv antibody fragments can all be expressed in and secreted from e.g. E. coli, thus, allowing the facile production of large amounts of these fragments. Antibody fragments can be isolated from antibody phage libraries according to standard procedures. Alternatively, Fab'-SH fragments can be directly recovered from E. coli. (Carter, P., et al., Bio/Technology 10 (1992) 163-167). Mammalian cell systems can be also used to express and, if desired, secrete antibody fragments.

In one example the binding entity that specifically binds to an antigen is a single-domain antibody. In a certain example a single-domain antibody is a human single-domain antibody (see, e.g., US 6,248,516). In one example a single-domain antibody consists of all or a portion of the heavy chain variable domain of an antibody.

A single-domain antibody is a single polypeptide chain comprising all or a portion of the heavy chain variable domain or all or a portion of the light chain variable domain of an antibody.

In certain embodiments, the binding entity binds to its target with a dissociation constant (KD) of ≤ 10 nM, ≤ 1 nM, ≤ 0.1 nM, ≤ 0.01 nM, or ≤ 0.001 nM (e.g. 10⁻⁸M or less, e.g. from 10⁻⁸ M to 10⁻¹³ M, e.g., from 10⁻⁹ M to 10⁻¹³ M).

In certain embodiments, the binding entity binds to its target with a dissociation constant (KD) of 10⁻⁵ M to 10⁻¹³ M, or of 10⁻⁵ M to 10⁻¹⁰ M, or of 10⁻⁵ M to 10⁻⁸ M.

In one embodiment in which the binding entity is an antibody or an antibody fragment, the dissociation constant is determined by a radiolabeled antigen binding assay (RIA) performed with the Fab fragment of the antibody and its antigen as described by the following assay.

Solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen, Y., et al., J. Mol. Biol. 293 (1999) 865-881). To establish conditions for the assay, MICROTITER^{®} multi-well plates (Thermo Scientific) are coated overnight with 5 µg/ml of a capturing anti-FAB antibody (Cappel Labs) in 50 mM sodium carbonate (pH 9.6), and subsequently blocked with 2 % (w/v) bovine serum albumin in PBS for two to five hours at room temperature (approximately 23 °C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta, L.G., et al., Cancer Res. 57 (1997) 4593-4599). The FAB of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1 % polysorbate 20 (TWEEN-20^{®}) in PBS. When the plates have dried, 150 µl/well of scintillant (MICROSCINT-20^{™}; Packard) is added, and the plates are counted on a TOPCOUNT^{™} gamma counter (Packard) for ten minutes. Concentrations of each FAB that give less than or equal to 20 % of maximal binding are chosen for use in competitive binding assays.

According to another embodiment, the dissociation constant is determined using surface plasmon resonance assays using a BIACORE^{®}-2000 or a BIACORE ^{®}-3000 or a BIACORE ^{®} A-100 (BIAcore, Inc., Piscataway, NJ) at 25 °C with immobilized antigen CM5 chips at ~10 response units (RU).

Briefly, carboxymethylated dextran biosensor chips (CM5, BIACORE, Inc.) are activated with *N-*ethyl-*N*'-(3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 µg/ml (~ 0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of FAB (0.78 nM to 500 nM) are injected in PBS with 0.05 % polysorbate 20 (TWEEN-20^{™}) surfactant (PBST) at 25 °C at a flow rate of approximately 25 µl/min. Association rates (kₒₙ) and dissociation rates (k_{off}) are calculated using a simple one-to-one Langmuir binding model (BIACORE^{®} Evaluation Software version 3.2) by simultaneously fitting the association and dissociation sensorgrams. The equilibrium dissociation constant (KD) is calculated as the ratio koff/kon (see e.g. Chen, Y., et al., J. Mol. Biol. 293 (1999) 865-881). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25 °C of a 20 nM anti-antigen antibody (FAB form) in PBS, pH 7.2, in the presence of increasing concentrations of antigen as measured in a spectrometer, such as a stop-flow equipped spectrophotometer (Aviv Instruments) or a 8000-series SLM-AMINCO^{™} spectrophotometer (ThermoSpectronic) with a stirred cuvette.

In case, two binding molecules recognize two independent binding sites, a cooperative binding event can be generated, which can be in dependence of the polynucleotide linker length.

A cooperative binding effect is physically characterized in that the free Gibbs binding energies ΔG°₁ and ΔG°₂ summarize to ΔG°_{coop} : ΔG°₁ + ΔG°₂ = ΔG°_{coop}. According to the Gibbs Equation ΔG°_{coop} = -RTlnK_{Dcoop}, ΔG°_{coop} forms the product from the affinities K_{D1} and K_{D2}.

Enhancement of the free Gibbs binding energy by cooperativity dramatically increases binding affinity (K_{Dcoop}) and binding specificity.

Binding specificity is further increased, when the addressed binding sites are independently located on two different target molecules, which e.g. might be co-localized on the surface of a tumor cell.

The binding entity specifically binding to a target likely carries one or more free OH, COOH, NH₂ and/or SH groups, which could potentially react with certain coupling reagents. To avoid (side-)reactions during the conjugation reaction one of the coupling chemistries as given in the following Table 1 can be chosen.

Table 1 provides an overview over reactive groups for covalently binding the polypeptides to the respective member of a binding pair as well as for covalently binding the linker to the respective members of a binding pair.

**Table 1.**

| **reactive site within the first polypeptide** | **first reactive site of the linker L** | **second reactive site of the linker** | **reactive site within the second polypeptide** |
|---|---|---|---|
| ONH₂ (aminoxy) | C(H)=O (aldehyde) | -C≡C (alkyne) or triphenylphosphin carboxylic ester | N₃ (azide) |
| C(H)=O (aldehyde) | ONH₂ (aminoxy) | N₃ (azide) | -C=C (alkyne) or triphenylphosphin carboxylic ester |
| ONH2 ( aminoxy) | C(H)=O (aldehyde) | Diene | Dienophile |
| C(H)=O (aldehyde) | ONH₂ (aminoxy) | Dienophile | Diene |
| Dien | Dienophile | N₃ (azide) | -C=C (alkyne) or triphenylphosphin carboxylic ester |
| Dienophile | Diene | N₃ (azide) | -C=C (alkyne) or triphenylphosphin carboxylic ester |
| Dienophile | Diene | -C=C (alkyne) or triphenylphosphin carboxylic ester | N3 (azide) |
| Dien | Dienophile | -C=C (alkyne) or triphenylphosphin carboxylic ester | N₃ (azide) |

The above bi-orthogonal coupling chemistries are especially appropriate for the conjugation of the monovalent binding polypeptides. If the two binding partners are not carrying certain reactive functional groups, e.g. in the case of combination of two aptamers there is more freedom in selection of the reactive sites. Therefore in addition or in combination with the pairs of corresponding reactive sites given in the above table, amino/active ester (e.g. NHS ester), and SH/SH or SH/maleinimido can be used for orthogonal coupling.

The monovalent binding polypeptide may also be a synthetic peptide or peptide mimic. In case a polypeptide is chemically synthesized, amino acids with orthogonal chemical reactivity can be incorporated during such synthesis (see e.g. de Graaf, A.J., et al., Bioconjug. Chem. 20 (2009) 1281-1295). Since a great variety of orthogonal functional groups is at stake and can be introduced into a synthetic peptide, conjugation of such peptide to a linker is standard chemistry.

### The polynucleotide component

The complex as reported herein comprises a (polynucleotide) linker. The linker can either be covalently bound to the polypeptide(s) or the (polynucleotide) linker and the polypeptide(s) can be bound to each other by a specific binding pair.

When (polynucleotide) linkers of different length are used resulting complex constructs with different distances in between the first and second polypeptide specifically binding to a target can be obtained. This allows for optimal distance and/or flexibility.

The term polynucleotide is to be understood broadly and includes DNA and RNA as well as analogs and modifications thereof.

In one embodiment the polynucleotide linker is composed of a mixture of different types of monomers as long as more than 20 % of the monomers are nucleosides. In one embodiment the polynucleotide linker is composed of a mixture of different types of monomers as long as more than 30 % of the monomers are nucleosides. In one embodiment the polynucleotide linker is composed of a mixture of different types of monomers as long as more than 40 % of the monomers are nucleosides. In one embodiment the polynucleotide linker is composed of a mixture of different types of monomers as long as more than 50 % of the monomers are nucleosides.

For example, the linker can be composed exclusively of nucleosides or it can be a mixture of nucleosides and amino acids, and/or sugar residues, and/or diols, and/or phospho-sugar units, and/or non-ionic polymer building blocks.

An oligonucleotide may for example contain a substituted nucleotide carrying a substituent at the standard bases deoxyadenosine (dA), deoxyguanosine (dG), deoxycytosine (dC), deoxythymidine (dT), deoxyuracil (dU). Examples of such substituted nucleobases are 5-substituted pyrimidines (like 5-methyl-dC, aminoallyl-dU or -dC, 5-(aminoethyl-3-acrylimido)-dU, 5-propinyl-dU or -dC), 5-halogenated-dU or -dC, N-substituted pyrimidines (like N4-ethyl-dC), N-substituted purines (like N6-ethyl-dA, N2-ethyl-dG), 8-substituted purines (like 8-[6-amino)-hex-1-yl]-8-amino-dG or -dA), 8-halogenated-dA or -dG, 8-alkyl-dG or -dA, and 2-substituted-dA (like 2-amino-dA).

An oligonucleotide may contain a nucleotide or a nucleoside analog. I.e. the naturally occurring nucleobases can be exchanged by using nucleobase analogs like 5-nitroindol-D-riboside, 3-nitro-pyrrole-D-riboside, deoxyinosine (dI), deoyxanthosine (dX), 7-deaza-dG, -dA, -dI or -dX, 7-deaza-8-aza-dG, -dA, -dI or - dX, 8-aza-dA, -dG, -dI or -dX, D-Formycin, pseudo-dU, pseudo-iso-dC, 4-thio-dT, 6-thio-dG, 2-thio-dT, iso-dG, 5-methyl-iso-dC, N8-linked-8-aza-7-deaza-dA, 5,6-dihydro-5-aza-dC, etheno-dA, or pyrollo-dC. As obvious to the skilled artisan, the nucleobase in the complementary strand has to be selected in such manner that duplex formation is specific. If, for example, 5-methyl-iso-dC is used in one strand (e.g. (a)) iso-dG has to be in the complementary strand (e.g. (a')).

In one embodiment the oligonucleotide backbone of the linker is modified to contain substituted sugar residues, sugar analogs, modifications in the inter-nucleoside phosphate moiety, and/or is a PNA (having a backbone without phosphate and d-ribose).

An oligonucleotide may for example contain a nucleotide with a substituted deoxy ribose like 2'-methoxy-, 2'-fluoro-, 2'-methylseleno-, 2'-allyloxy-, 4'-methyl-dN (wherein N is a nucleobase, e.g., A, G, C, T or U).

Sugar analogs are for example xylose, 2',4'-bridged ribose like (2'-O, 4'-C methylene) (oligomer known as LNA), or (2'-O, 4'-C ethylene) (oligomer known as ENA), L-ribose, L-D-ribose, hexitol (oligomer known as HNA), cyclohexenyl (oligomer known as CeNA), altritol (oligomer known as ANA), a tricyclic ribose analog where C3' and C5' atoms are connected by an ethylene bridge that is fused to a cyclopropane ring (oligomer known as tricyclo DNA), glycerin (oligomer known as GNA), glucopyranose (oligomer known as Homo DNA), carbaribose (with a cyclopentane instead of a tetrahydrofurane subunit), hydroxymethyl-morpholine (oligomers known as morpholino DNA).

A great number of modification of the inter-nucleosidic phosphate moiety are also known not to interfere with hybridization properties and such backbone modifications can also be combined with substituted nucleotides or nucleotide analogs. Examples are phosphorthioate, phosphordithioate, phosphoramidate and methylphosphonate oligonucleotides.

The above mentioned modified nucleotides, nucleotide analogs as well as polynucleotide backbone modifications can be combined as desired in a polynucleotide comprised in the complex as reported herein.

The (polynucleotide) linker has a length of from 1 nm to 100 nm. In one embodiment the (polynucleotide) linker has a length of from 4 nm to 80 nm. In one embodiment the (polynucleotide) linker has a length of from 5 nm to 50 nm or of from 6 nm to 40 nm. In one embodiment the (polynucleotide) linker has a length of 10 nm or longer or of 15 nm or longer. In one embodiment the (polynucleotide) linker has a length between 10 nm and 50 nm.

In one embodiment the members of a binding pair conjugated to the (polynucleotide) linker have a length of at least 2.5 nm each.

The length of the (polynucleotide) linker can be calculated by using known bond distances and bond angles of components which are chemically similar to the entities. Such bond distances are summarized for some molecules in standard text books (see e.g. CRC Handbook of Chemistry and Physics, 91st edition (2010-2011), Section 9).

In the calculation of a spacer or a linker length the following approximations apply: a) for calculating lengths of non-nucleosidic entities an average bond length of 130 pm with an bond angle of 180° independently of the nature of the linked atoms is used, b) one nucleotide in a single strand is calculated with 500 pm, and c) one nucleotide in a double strand is calculated with 330 pm.

The value of 130 pm is based on calculation of the distance of the two terminal carbon atoms of a C(sp3)-C(sp3)-C(sp3) chain with a bond angle of 109°28' and a distance of 153 pm between two C(sp3) which is approx. 250 pm which translates with an assumed bond angle of 180° and bond distance between two C(Sp3) with 125 pm. Taking in account that heteroatoms like P and S and sp2 and sp1 C atoms could also be part of the linker the value 130 pm is taken. If the linker comprises a cyclic structure like cycloalkyl or aryl the distance is calculated in analogous manner by counting the number of the bonds of the cyclic structure which are part of the overall chain of atoms which are defining the distance.

The length of the (polynucleotide) linker in a complex as reported herein can be varied as desired. In order to easily make available linkers of variable length, i.e. a library, it is suitable to have a simple synthetic access to the different linkers of such library. A combinatorial solid phase synthesis of the linker is suited. Since linkers have to be synthesized up to a length of about 100 nm, the synthesis strategy is chosen in such a manner that the monomeric synthetic building blocks are assembled during solid phase synthesis with high efficiency. The synthesis of deoxy oligonucleotides based on the assembly of phosphoramidite as monomeric building blocks meet this requirement. In such a linker monomeric units within a linker are linked in each case via a phosphate or phosphate analog moiety.

The (polynucleotide) linker can contain as in one embodiment free positively or/and negatively charged groups of polyfunctional amino-carboxylic acids, e.g. amino, carboxylate or phosphate. For example the charge carriers can be derived from trifunctional aminocarboxylic acids which contain a) an amino group and two carboxylate groups, or b) two amino groups and one carboxylate group. Examples of such trifunctional aminocarboxylic acids are lysine, ornithine, hydroxylysine, α,β-diamino propionic acid, arginine, aspartic acid and glutamic acid, carboxy glutamic acid and symmetric trifunctional carboxylic acids like those described in EP 0 618 192 or US 5,519,142. Alternatively one of the carboxylate groups in the trifunctional aminocarboxylic acids of a) can be replaced by a phosphate, sulphonate or sulphate group. An example of such a trifunctional amino acid is phosphoserine.

The (polynucleotide) linker can also contain as in one embodiment uncharged hydrophilic groups. Suited examples of uncharged hydrophilic groups are ethylene oxide or poly (ethylene oxide) groups comprising especially at least three building blocks, such as ethylene oxide, sulphoxide, sulphone, carboxylic acid amide, carboxylic acid ester, phosphonic acid amide, phosphonic acid ester, phosphoric acid amide, phosphoric acid ester, sulphonic acid amide, sulphonic acid ester, sulphuric acid amide and sulphuric acid ester groups. The amide groups are in one embodiment primary amide groups, especially carboxylic acid amide residues in amino acid side groups, e.g. of the amino acids asparagine and glutamine. The esters are especially derived from hydrophilic alcohols, in particular C1-C3 alcohols, or diols, or triols.

Enantiomeric L-DNA is known for its orthogonal hybridization behavior, its nuclease resistance, and for ease of synthesis of polynucleotides of variable length.

In one embodiment all polynucleotides in the complex are enantiomeric L-DNA or L-RNA. In one embodiment all polynucleotides in the complex are enantiomeric L-DNA.

Enantiomeric, single stranded L-DNA (ss-L-DNA) combines high molecular flexibility and stability in body fluids. When single stranded L-DNA is used as a linker between two or more independent binding molecules, these binding molecules can get adjusted to virtually any binding angle and binding distance, which are just dependent from the ss-L-DNA linker length.

In one embodiment the (polynucleotide) linker is synthesized in segments that can hybridize with each other.

In this case the linker can be formed by hybridization of the segments with one another. The resulting linker construct comprises oligonucleotide duplex portions. In case the linker is constructed that way the sequence of the hybridizable polynucleotide entity forming the duplex is chosen in such a manner that no hybridization or interference with the binding pair nucleic acids can occur.

In one embodiment the polynucleotide linker is synthesized in ss-L-DNA segments, e.g. A and B, which can hybridize with each other.

In this case the polynucleotide linker can be build up by the hybridization of the segments with one another. Therefore, the linker length can be self-adjusted to the distance between two binding sites simply by sequential application of the concatemer forming building blocks, i.e. A and B as exemplified. The linker is characterized in that the nucleic acid termini of the established linker hybridize with lower melting point temperature (i.e. TM1) to the ss-L-DNA labeled binding molecules than the inter-concatemeric melting point temperature (i.e. TM2, thus with TM2 > TM1). To analyze the final length of the full length linker, the obtained complex is incubated at a third temperature (i.e. TM3) that is above the first melting point temperature but below the second melting point temperature (i.e. TM3 > TM1 and TM3 < TM2). The temperature-eluted linker can be analyzed by standard methods e.g. using ethidiumbromide stained agarose gel. The linker length can also be calculated, because the length of each concatemer is known. The individual concatemers can be labeled in one embodiment.

The duplex portions can rigidize the oligonucleotide linker. This can be used to reduce the linker mobility and flexibility.

In one embodiment one or more L-DNA oligonucleotides are hybridized to the oligonucleotide L-DNA linker.

In this embodiment the oligonucleotide linker is rigidized via L-DNA duplex formation.

In one embodiment an L-DNA/poly (ethylene glycol) hybrid is used as (oligonucleotide) linker.

In one embodiment an L-DNA/D-DNA/poly (ethylene glycol) hybrid is used as (oligonucleotide) linker.

In one embodiment an L-DNA/D-DNA/poly (ethylene glycol)/polypeptide hybrid is used as (oligonucleotide) linker.

In one embodiment one or more L-DNA oligonucleotides are hybridized to the L-DNA/poly (ethylene glycol) hybrid (oligonucleotide) linker.

In one embodiment one or more L-DNA oligonucleotides, which are covalently coupled to a poly (ethylene glycol) molecule of varying length, are hybridized to the oligonucleotide L-DNA poly (ethylene glycol) hybrid (oligonucleotide) linker.

In one embodiment an L-DNA/D-DNA hybrid is used as (oligonucleotide) linker.

In one embodiment an L-DNA/D-DNA hybrid is used as (oligonucleotide) linker, wherein one or more D-DNA oligonucleotides are hybridized to the oligonucleotide D-DNA portion of the (oligonucleotide) linker to form double stranded D-DNA.

In one embodiment an L-DNA/D-DNA hybrid is used as linker, wherein one or more L-DNA oligonucleotides are hybridized to the oligonucleotide L-DNA portion of the (oligonucleotide) linker to form double stranded L-DNA.

The formation of double stranded, i.e. helical, DNA-duplexes can be used to modify or adjust the in vivo half-life of the complex making it available for the enzymatic action of nucleases.

A simple way to build the (polynucleotide) linker is to use standard D or L nucleoside phosphoramidite building blocks.

In one embodiment a single strand stretch of dT is used.

This is advantageous, because dT does not require carrying a base protecting group.

Hybridization can be used in order to vary the (polynucleotide) linker length (distance between the binding pair members at the termini of the polynucleotide linker) and the flexibility of the spacer, because the double strand length is reduced compared to the single strand and the double strand is more rigid than a single strand.

For hybridization in one embodiment oligonucleotides modified with a functional moiety are used.

The oligonucleotide used for hybridization can have one or two terminal extensions not hybridizing with the linker and/or is branched internally. Such terminal extensions that are not hybridizing with the linker (and not interfering with the members of the binding pairs) can be used for further hybridization events.

In one embodiment an oligonucleotide hybridizing with a terminal extension is oligonucleotide comprising an effector moiety.

This labeled oligonucleotide again may comprise terminal extensions or being branched in order to allow for further hybridization, thereby a polynucleotide aggregate or dendrimer can be obtained. A poly-oligonucleic acid dendrimer is especially used in order to produce a polylabel, or in order to get a high local concentration of an effector moiety.

Modified nucleotides which do not interfere with the hybridization of polynucleotides can be incorporated into those polynucleotides. Suited modified nucleotides are C5-substituted pyrimidines or C7-substituted 7-deaza purines. Polynucleotides can be modified internally or at the 5' or 3' terminus with non-nucleotidic entities which are used for the introduction of the effector moiety.

In one embodiment such non-nucleotidic entities are located within the (polynucleotide) linker between the two binding pair members conjugated to its ends.

Many different non-nucleotidic building blocks for construction of a polynucleotide are known in literature and a great variety is commercially available. For the introduction of an effector moiety either non-nucleosidic bifunctional building blocks or non-nucleosidic trifunctional building blocks can either be used as CPG for terminal labeling or as phosphoramidite for internal labeling (see e.g. Wojczewski, C., et al., Synlett 10 (1999) 1667-1678).

Bifunctional spacer building blocks in one embodiment are non-nucleosidic components. For example, such linkers are C2 - C18 alkyl, alkenyl, alkinyl carbon chains, whereas the alkyl, alkenyl, alkinyl chains may be interrupted by additional ethyleneoxy and/or amide moieties or quarternized cationic amine moieties in order to increase hydrophilicity of the linker. Cyclic moieties like C5-C6-cycloalkyl, C4N-, C5N-, C4O-, C5O-heterocycloalkyl, phenyl which are optionally substituted with one or two C1-C6 alkyl groups can also be used as non-nucleosidic bifunctional linkers. Suited bifunctional building blocks comprise C3-C6 alkyl moieties and tri- to hexa- ethylene glycol chains. Tables 2a and 2b show some examples of nucleotidic bifunctional spacer building blocks with different hydrophilicity, different rigidity and different charges. One oxygen atom is connected to an acid labile protecting group preferably dimethoxytrityl and the other is part of a phosphoramidite.

**Table 2a.**

| Non-nucleotidic bifunctional spacer building blocks | Reference |
|---|---|
| 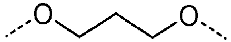 | Seela, F., Nucleic Acids Research 15 (1987) 3113-3129. |
| 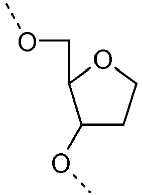 | Iyer, R.P., Nucleic Acids Research 18 (1990) 2855-2859. |
| 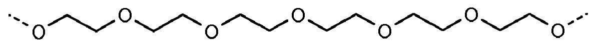 | WO 89/02931 A1 |
| 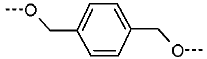 | EP 1 538 221 |
| 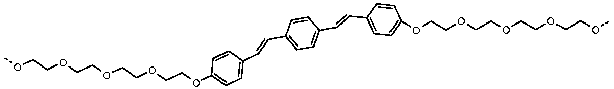 | US 2004/224372 |
| 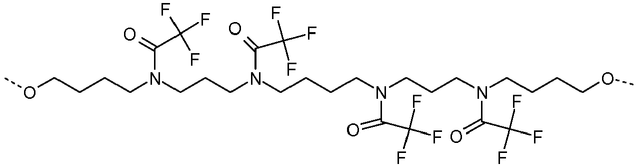 | WO 2007/069092 |

**Table 2b.**

| Bifunctional non-nucleosidic modifierIntroduction of building blocks | Reference |
|---|---|
| 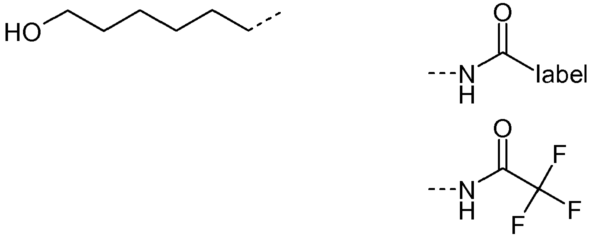 | Pon, R.T., Tetrahedron Letters 32 (1991) 1715-1718. |
| | Theisen, P., et al., Nucleic Acids Symposium Series 27 (Nineteenth Symposium on Nucleic Acids Chemistry (1992)) 99-100. |
| | EP 0 292 128 |
| 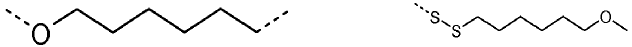 | EP 0 523 978 |
| 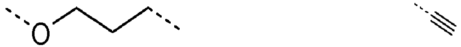 | Meyer, A., et al., Journal of Organic Chemistry 75 (2010)3927-3930. |
| 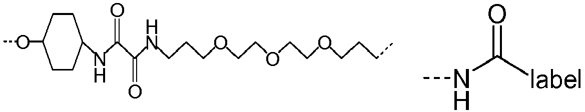 | Morocho, A.M., et al., Nucleosides, Nucleotides & Nucleic Acids 22(2003)1439-1441. |
| 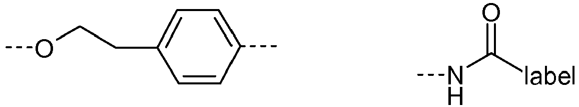 | Cocuzza, A., Tetrahedron Letters 30 (1989) 6287-6290. |

Therefore trifunctional building blocks allow for positioning of a functional moiety to any location within a polynucleotide. Trifunctional building blocks are also a prerequisite for synthesis using solid supports, e.g. controlled pore glass (CPG), which are used for 3' terminal labeling of polynucleotides. In this case, the trifunctional linkers is connected to a functional moiety or a - if necessary - a protected functional moiety via an C2 - C18 alkyl, alkenyl, alkinyl carbon chains, whereas the alkyl, alkenyl, alkinyl chains may be interrupted by additional ethyleneoxy and/or amide moieties in order to increase hydrophilicity of the linker and comprises a hydroxyl group which is attached via a cleavable spacer to a solid phase and a hydroxyl group which is protected with an acid labile protecting group. After removal of this protecting group a hydroxyl group is liberated that could thereafter react with a phosphoramidite.

Trifunctional building blocks may be non-nucleosidic or nucleosidic.

Non-nucleosidic trifunctional building blocks are C2 - C18 alkyl, alkenyl, alkinyl carbon chains, whereas the alkyl, alkenyl, alkinyl are optionally interrupted by additional ethyleneoxy and/or amide moieties in order to increase hydrophilicity of the linker. Other trifunctional building blocks are cyclic groups like C5-C6-cycloalkyl, C4N-, C5N-, C4O-, C5O-heterocycloalkyl, phenyl which are optionally substituted with one or two C1-C6 alkyl groups. Cyclic and acyclic groups may be substituted with one (C1-C18)alkyl-O-PG group, whereas the C1-C18 alkyl comprises (Ethyleneoxy)ₙ, (Amide)ₘ moieties with n and m independently from each other = 0 - 6 and PG is an acid labile protecting group. Preferred trifunctional building blocks are C3-C6 alkyl, cycloalkyl, C5O-heterocycloalkyl moieties optionally comprising one amide bond and substituted with a C1 - C6 alkyl O-PG group, wherein PG is an acid labile protecting group, preferably monomethoxytrityl, dimethoxytrityl, pixyl, xanthyl most preferred dimethoxytrityl.

Non-limiting, yet suited examples for non-nucleosidic trifunctional building blocks are e.g. summarized in Table 3.

**Table 3.**

| Trifunctional | introduction of | Reference |
|---|---|---|
| 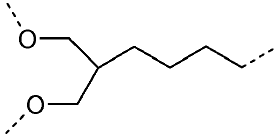 | 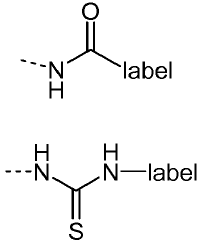 | Nelson, P.S., et al., Nucleic Acids Research 20 (1992) 6253-6259. |
| | | EP 0 313 219; US 5,585,481; US 5,451,463; EP 0 786 468; WO 92/11388, WO 89/02439 |
| 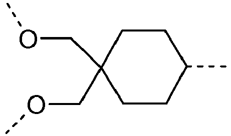 | 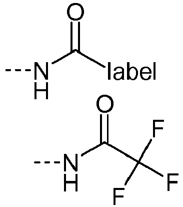 | Su, Sheng-Hui, et al., Bioorganic & Medicinal Chemistry Letters 7 (1997) 1639-1644. |
| | | WO 97/43451 |
| 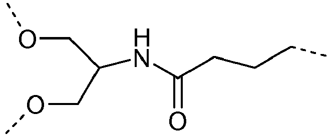 | 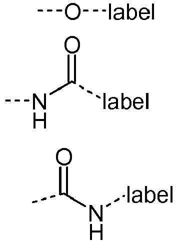 | Putnam, W.C. and Bashkin, J.K., Nucleosides, Nucleotides & Nucleic Acids 24 (2005) 1309-1323. |
| | | US 2005/214833, EP 1 186 613 |
| 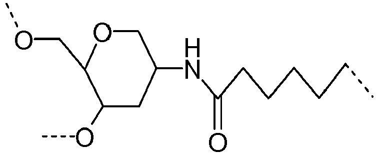 | 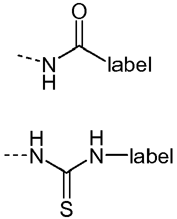 | EP 1 431 298 |
| 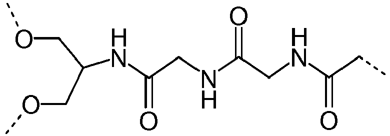 | 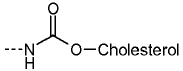 | WO 94/04550 |
| | | Huynh Vu, et al., Nucleic Acids Symposium Series (1993) 29 (Second International Symposium on Nucleic Acids Chemistry) 19-20. |
| 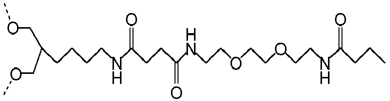 | 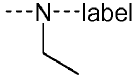 | WO 2003/019145 |
| 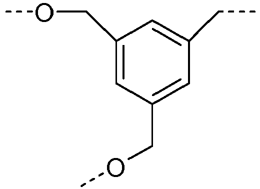 | 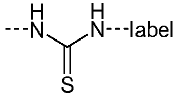 | Behrens, C. and Dahl, O., Nucleosides & Nucleotides 18 (1999) 291-305. |
| | | WO 97/05156 |
| 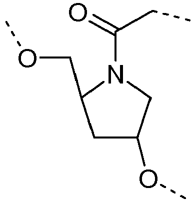 | 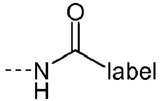 | Prokhorenko, I.A., et al., Bioorganic & Medicinal Chemistry Letters 5 (1995) 2081-2084. |
| | | WO 2003/104249 |
| 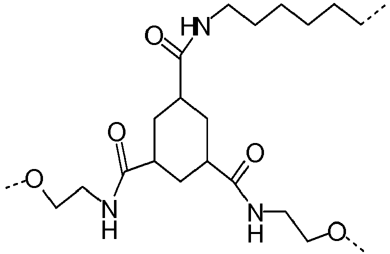 | 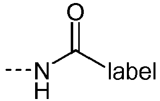 | US 5,849,879 |

Nucleosidic trifunctional building blocks are used for internal labeling whenever it is necessary not to influence the polynucleotide hybridization properties compared to a non-modified polynucleotide. Therefore nucleosidic building blocks comprise a base or a base analog which is still capable of hybridizing with a complementary base. The general formula of a labeling compound for labeling a nucleic acid sequence of one or more of a, a', b, b' or S comprised in a complex as reported herein is given in Formula II. 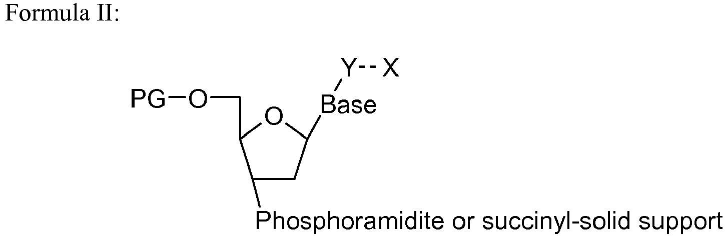 wherein PG is an acid labile protecting group, especially monomethoxytrityl, dimethoxytrityl, pixyl, xanthyl, especially dimethoxytrityl, wherein Y is C2 - C18 alkyl, alkenyl alkinyl, wherein the alkyl, alkenyl, alkinyl may comprise ethyleneoxy and/or amide moieties, wherein Y preferably is C4 - C18 alkyl, alkenyl or alkinyl and contains one amide moiety and wherein X is a functional moiety.

Specific positions of the base may be chosen for such substitution to minimize the influence on hybridization properties. Therefore the following positions are especially suited for substitution: a) with natural bases: uracil substituted at C5, cytosine substituted at C5 or at N4, adenine substituted at C8 or at N6, and guanine substituted at C8 or at N2, and b) with base analogs: 7-deaza-A and 7-deaza-G substituted at C7, 7-deaza-8-aza-A and 7-deaza-8-aza-G substituted at C7, 7-deaza-aza-2-amino-A substituted at C7, pseudouridine substituted at N1 and formycin substituted at N2.

**Table 4.**

| Trifunctional nucleosidic | introduction of | Reference |
|---|---|---|
| 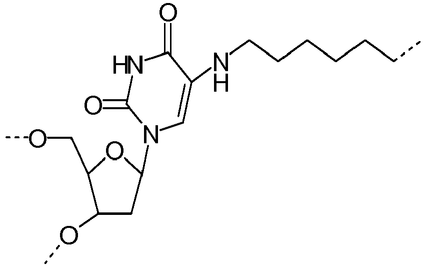 | 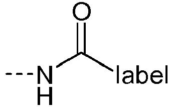 | Roget, A., et al., Nucleic Acids Research 17 (1989) 7643-7651. |
| | | WO 89/12642, WO 90/08156, WO 93/05060 |
| 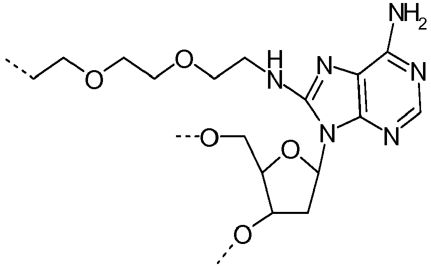 | 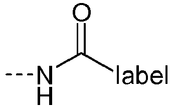 | Silva, J.A., et al., Biotecnologia Aplicada 15 (1998)1154-158. |
| 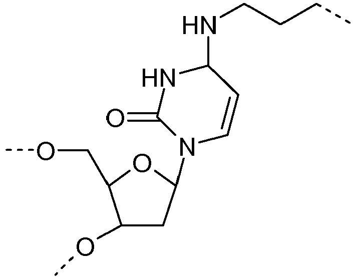 | 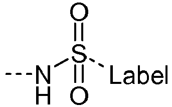 | US 6,531,581 EP 0 423 839 |
| 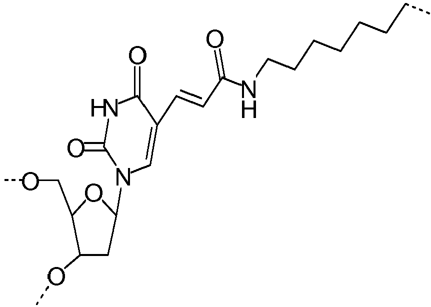 | 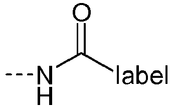 | US 4,948,882; US 5,541,313; US 5,817,786 |
| 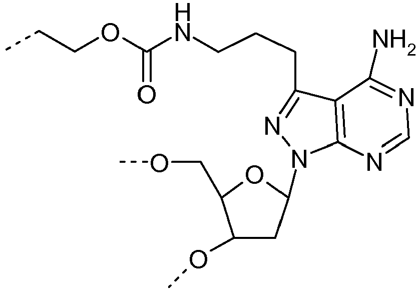 | 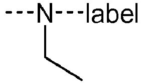 | WO 2001/042505 |
| 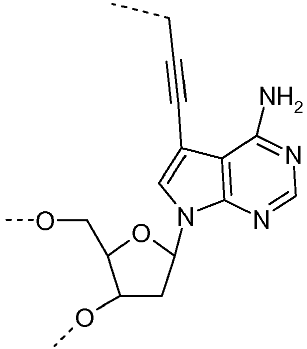 | 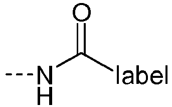 | McKeen, C.M., et al., Organic & Biomol. Chem. 1 (2003) 2267-2275. |
| 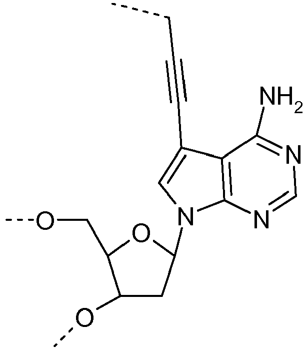 | 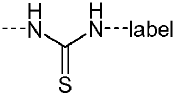 | Ramzaeva, N., et al., Helv. Chim. Acta 83 (2000) 1108-1126. |

In Table 4 the terminal oxygen atom of bifunctional moiety or one of the terminal oxygen atoms of a trifunctional moiety are part of a phosphoramidite that is not shown in full detail but obvious to the skilled artisan. The second terminal oxygen atom of trifunctional building block is protected with an acid labile protecting group PG, as defined for Formula II above.

Post-synthetic modification is another strategy for introducing a covalently bound functional moiety into a linker. In this approach an amino group is introduced by using bifunctional or trifunctional building blocks during solid phase synthesis. After cleavage from the support and purification of the amino modified linker the linker is reacted with an activated ester of a functional moiety or with a bifunctional reagent wherein one functional group is an active ester. Especially suited active esters are NHS ester or pentafluor phenyl esters.

Post-synthetic modification is especially useful for introducing a functional moiety which is not stable during solid phase synthesis and deprotection. Examples are modification with triphenylphosphincarboxymethyl ester for Staudinger ligation (Wang, Charles C.-Y., et al., Bioconjugate Chemistry 14 (2003) 697-701), modification with digoxigenin or for introducing a maleinimido group using commercial available sulfo SMCC.

### The binding pair component

In one embodiment each member of a binding pair is of/has a molecular weight of 10 kDa or less. In one embodiment the molecular weight of each member of a binding pair is 8 kDa, or 7 kDa, or 6 kDa, or 5 kDa, or 4 kDa or less.

The dissociation constant, i.e. the binding affinity, for (within) a binding pair is at least 10⁻⁸ M (= 10⁻⁸ mol/l = 10⁸ 1/mol). The members of both binding pairs in the complex as reported herein are different. The difference between the binding pairs a:a' and b:b' is e.g. acknowledged if the dissociation constant for the reciprocal binding, e.g. binding of a as well as a' to b or b', is 10 times the dissociation constant of the pair a:a' or more.

In one embodiment dissociation constant for the reciprocal binding, i.e. binding of a as well as a' to b or b', respectively, is 20 times the dissociation constant of the pair a:a' or more. In one embodiment the dissociation constant is 50 times the dissociation constant within the pair a:a' or more. In one embodiment the reciprocal (cross-reactive) binding dissociation constant is 100 times or more the dissociation constant within a binding pair.

In one embodiment the members of the binding pairs are selected from the group consisting of leucine zipper domain dimers and hybridizing nucleic acid sequences. In one embodiment both binding pairs are leucine zipper domain dimers.

In one embodiment both binding pairs are hybridizing nucleic acid sequences. In one embodiment all binding pair members are L-DNA sequences. In one embodiment both binding pairs are hybridizing L-DNAs.

In one embodiment both member of the binding pairs represent leucine zipper domains.

The term "leucine zipper domain" denotes a dimerization domain characterized by the presence of a leucine residue at every seventh residue in a stretch of approximately 35 residues. Leucine zipper domains are peptides that promote oligomerization of the proteins in which they are found. Leucine zippers were originally identified in several DNA-binding proteins (Landschulz, W.H., et al., Science 240 (1988) 1759-1764). Among the known leucine zippers are naturally occurring peptides and derivatives thereof that dimerize or trimerize. Examples of leucine zipper domains suitable for producing soluble multimeric proteins are those reported in WO 94/10308, and the leucine zipper derived from lung surfactant protein D (SPD) as reported in Hoppe, H.J., et al., FEBS Lett. 344 (1994) 191-195.

Leucine zipper domains form dimers (binding pairs) held together by an alpha-helical coiled coil. A coiled coil has 3.5 residues per turn, which means that every seventh residue occupies an equivalent position with respect to the helix axis. The regular array of leucines inside the coiled coil stabilizes the structure by hydrophobic and Van der Waals interactions.

If leucine zipper domains form the first binding pair and the second binding pair, both leucine zipper sequences are different, i.e. the members of the first binding pair do not bind to the members of the second binding pair. Leucine zipper domains may be isolated from natural proteins known to contain such domains, such as transcription factors. One leucine zipper domain may e.g. come from the transcription factor fos and a second one from the transcription factor jun. Leucine zipper domains may also be designed and synthesized artificially, using standard techniques for synthesis and design known in the art.

In one embodiment both binding pairs are hybridizing nucleic acid sequences.

Thus, the members of each binding pair, i.e. a and a' as well as b and b', hybridize to one another, respectively. The nucleic acid sequences comprised in the first binding pair on the one hand and in the second binding pair on the other hand are different, i.e. do not hybridize with each other.

In one embodiment the binding pairs are both hybridizing nucleic acid pairs, wherein the hybridizing nucleic acid sequences of the different binding pairs do not hybridize with one another.

With other words the nucleic acids of the first binding pair hybridize to each other but do not bind to any of the nucleic acids of the second binding pair or interfere with their hybridization and vice versa. Hybridization kinetics and hybridization specificity can easily be monitored by melting point analyses. Specific hybridization of a binding pair and non-interference is acknowledged, if the melting temperature for the binding pair as compared to any possible combination with other binding pairs or combination of binding pair members is at least 20 °C higher.

The nucleic acid sequences forming a binding pair may comprise in principle any naturally occurring nucleobase or an analogue thereto and may have in principle a modified or a non-modified backbone as described above provided it is capable of forming a stable duplex via multiple base pairing. Stable denotes that the melting temperature of the duplex is higher than 30 °C, especially higher than 37 °C.

The double strand is in one embodiment consisting of two fully complementary single stranded polynucleotides.

However mismatches or insertions are possible as long as the stability at 37 °C is given.

A nucleic acid duplex can be further stabilized by inter-strand crosslinking. Several appropriate cross-linking methods are known, e.g. methods using psoralen or based on thionucleosides.

The nucleic acid sequences representing the members of a binding pair in one embodiment consist of from 12 to 50 nucleotides. In one embodiment such nucleic acid sequences consist of from 15 to 35 nucleotides.

RNAses are ubiquitous and special care has to be taken to avoid unwanted digestion of RNA-based binding pairs and/or linker sequences. While RNA-based binding pairs and/or linkers can be used, binding pairs and/or linkers based on DNA are especially suited.

Appropriate hybridizing nucleic acid sequences can easily be designed to provide for more than two pairs of orthogonal complementary polynucleotides, allowing for an easy generation and use of more than two binding pairs. Another advantage of using hybridizing nucleic acid sequences in a complex as reported herein is that modifications can be easily introduced. Modified building blocks are commercially available which e.g. allow for an easy synthesis of a polynucleotide comprising a functional moiety. Such functional moiety can be easily introduced at any desired position and in any of the members of the first and/or second binding pair and/or the polynucleotide linker, provided they all represent a polynucleotide.

The (polynucleotide) linker comprising members of bindings pairs at its termini can be provided for and synthesize as a single polynucleotide. The polypeptides specifically binding to a target can each be coupled to hybridizing nucleic acid sequences, i.e. members of binding pairs. The length of the (polynucleotide) linker can easily be varied in any desired manner.

Depending on the biochemical nature of the polypeptide that specifically binds to a target different strategies for the conjugation to the member of a binding pair are at hand. In case the polypeptide is naturally occurring or recombinantly produced and between 50 to 500 amino acid residues in length, standard procedures as reported in text books can be easily followed by the skilled artisan (see e.g. Hackenberger, C.P.R., and Schwarzer, D., Angew. Chem. Int. Ed. 47 (2008) 10030-10074).

In one embodiment for the conjugation the reaction of a maleinimido moiety with a cysteine residue within the polypeptide is used.

This is an especially suited coupling chemistry in case e.g. a FAB or FAB'-fragment of an antibody is used a monovalent binding polypeptide.

In one embodiment coupling of a member of a binding pair to the C-terminal end of the polypeptide is performed.

C-terminal modification of a protein, e.g. of a FAB-fragment can e.g. be performed as described (Sunbul, M. and Yin, J., Org. Biomol. Chem. 7 (2009) 3361-3371).

In general site specific reaction and covalent coupling of a binding pair member to a monovalent binding polypeptide is based on transforming a natural amino acid into an amino acid with a reactivity which is orthogonal to the reactivity of the other functional groups present in a polypeptide.

For example, a specific cysteine within a rare sequence context can be enzymatically converted in an aldehyde (see e.g. Frese, M-A. and Dierks, T., ChemBioChem 10 (2009) 425-427). It is also possible to obtain a desired amino acid modification by utilizing the specific enzymatic reactivity of certain enzymes with a natural amino acid in a given sequence context (see e.g.: Taki, M., et al., Prot. Eng. Des. Sel. 17 (2004) 119-126, Gautier, A., et al., Chem. Biol. 15 (2008) 128-136; Bordusa, F., in Highlights in Bioorganic Chemistry (2004), Schmuck, C. and Wennemers, H., (eds.), Wiley VCH, Weinheim, pp. 389-403).

Site specific reaction and covalent coupling of a binding pair member to a monovalent binding polypeptide can also be achieved by the selective reaction of terminal amino acids with appropriate modifying reagents.

The reactivity of an N-terminal cysteine with benzonitrils (see Ren, H., et al., Angew. Chem. Int. Ed. 48 (2009) 9658-9662) can be used to achieve a site-specific covalent coupling.

Native chemical ligation can also rely on C-terminal cysteine residues (Taylor, E., et al., Nucl. Acids Mol. Biol. 22 (2009) 65-96).

EP 1 074 563 reports a conjugation method which is based on the faster reaction of a cysteine within a stretch of negatively charged amino acids with a cysteine located in a stretch of positively charged amino acids.

### The effector component

The effector moiety can be selected from the group consisting of a binding moiety, a labeling moiety, a biologically active moiety, and a reactive moiety. If more than one effector moiety is present in the complex, each such effector moiety can in each case be independently a binding moiety, a labeling moiety, a biologically active moiety, or a reactive moiety. The binding moiety will be selected to have no interference with each of the binding pairs.

In one embodiment the effector moiety is selected from the group consisting of a binding moiety, a labeling moiety, and a biologically active moiety.

In one embodiment the effector moiety is a binding moiety.

Examples of binding moieties are the members of a bioaffine binding pair which can specifically interact with each other. Suitable bioaffine binding pairs are hapten or antigen and antibody; biotin or biotin analogues such as aminobiotin, iminobiotin or desthiobiotin and avidin or streptavidin; sugar and lectin, polynucleotide and complementary polynucleotide, receptor and ligand, e.g., steroid hormone receptor and steroid hormone; and the pair of an 104-aa fragment of bovine ribonuclease A (known as S-protein) and a 15-aa fragment of bovine ribonuclease A (known as S-peptide).

In one embodiment the effector moiety is a binding moiety and is covalently bound to at least one of the components of the complex.

In one embodiment the smaller partner of a bioaffine binding pair, e.g. biotin or an analogue thereto, a receptor ligand, a hapten or a polynucleotide is covalently bound to at least one of the polynucleotides comprised in the complex as reported herein.

In one embodiment the effector moiety is a binding moiety selected from hapten, biotin or biotin analogues such as aminobiotin, iminobiotin or desthiobiotin; polynucleotide and steroid hormone.

In one embodiment the effector moiety is a labeling group.

The labeling group can be selected from any known detectable group.

In one embodiment the labeling group is selected from dyes like luminescent labeling groups such as chemiluminescent groups e.g. acridinium esters or dioxetanes or fluorescent dyes e.g. fluorescein, coumarin, rhodamine, oxazine, resorufin, cyanine and derivatives thereof, luminescent metal complexes such as ruthenium or europium complexes, enzymes as used for CEDIA (Cloned Enzyme Donor Immunoassay, e.g. EP 0 061 888), microparticles or nanoparticles e.g. latex particles or metal sols, and radioisotopes.

In one embodiment the labeling group is a luminescent metal complex and the compound has a structure of the general formula (I):

[M(L₁L₂L₃)]ₙ - Y - XₘA (I)

in which M is a divalent or trivalent metal cation selected from rare earth or transition metal ions, L₁, L₂ and L₃ are the same or different and denote ligands with at least two nitrogen-containing heterocycles in which L₁, L2 and L3 are bound to the metal cation via nitrogen atoms, X is a reactive functional group which is covalently bound to at least one of the ligands L₁, L2 and L3 via a linker Y, n is an integer from 1 to 10, especially 1 to 4, m is 1 or 2, or especially 1 and A denotes the counter ion which may be required to equalize the charge.

The metal complex is in one embodiment a luminescent metal complex i.e. a metal complex which undergoes a detectable luminescence reaction after appropriate excitation.

The luminescence reaction can for example be detected by fluorescence or by electrochemiluminescense measurement. The metal cation in this complex is for example a transition metal or a rare earth metal.

The metal is in one embodiment ruthenium, osmium, rhenium, iridium, rhodium, platinum, indium, palladium, molybdenum, technetium, copper, chromium or tungsten. Ruthenium, iridium, rhenium, chromium and osmium are especially suited. Ruthenium is most suited.

Gold nanorods (GNRs) can also be used as labeling moiety in the complexes as reported herein. The nanorods can have a length of from 10 to 100 nm, inclusive, and including all integers there between.

In one embodiment, the GNRs have an average length of from 70-75 nm.

The GNRs can have a diameter of from 5 to 45 nm inclusive, and including all integers there between.

In one embodiment, the GNRs have an average diameter of 25-30 nm. The GNRs can be pure gold, or may be from 90 % to 99 %, inclusive, including all integers there between, pure gold.

In various embodiments, the GNRs may contain up to 1 % silver on their surfaces, and may contain cetyltrimethylammonium bromide (CTAB).

In this regard, GNRs can be made by any suitable method. For example, electrochemical synthesis in solution, membrane templating, photochemical synthesis, microwave synthesis, and seed mediated growth are all suitable and non-limiting examples of methods of making the GNRs.

In one embodiment, the gold nanorods are made using the seed-mediated growth method in cetyltrimethylammonium bromide (CTAB).

In order to form complexes of the gold nanorods and the RNA polynucleotides, the surfaces of the gold nanorods can be functionalized so as impart a positive zeta potential suitable for electrostatically complexing the GNRs with DNA or RNA polynucleotides. Any suitable method of creating a positive zeta potential on the gold nanorods may be used. For example, the surfaces of the gold nanorods can be functionalized with bifunctional molecules, such as thiolated-PEG-NH2 or thiolated-PEG-COOH.

In one embodiment, the surface functionalization is achieved by coating the CTAB-coated gold nanorods first with the anionic polyelectrolyte poly (3,4-ethylenedioxythi-6-phene)/poly (styrene sulfate) (PEDT/PSS), then with the cationic polyelectrolyte poly (diallyl dimethyl ammonium chloride) (PDDAC).

This results in gold nanorods with a cationic surface charge (positive zeta potential), and also masks the CTAB layer (see, e.g., Ding, H., et al., J. Phys. Chem. C 111 (2007) 12552-12557).

The positively charged gold nanorods are electrostatically complexed to the DNA polynucleotides using electrostatic interactions.

The formation of nanoplexes can be confirmed from an observed red-shift in localized longitudinal plasmon resonance peak of the gold nanorods, as well as from restricted electrophoretic mobility of the nanoplexes using gel electrophoresis.

In one embodiment the effector moiety X is a therapeutically active substance.

Therapeutically active substances have different ways in which they are effective, e.g. in inhibiting cancer, damaging the DNA template by alkylation, by cross-linking, or by double-strand cleavage of DNA. Other therapeutically active substances can block RNA synthesis by intercalation. Some agents are spindle poisons, such as vinca alkaloids, or anti-metabolites that inhibit enzyme activity, or hormonal and anti-hormonal agents. The effector moiety may be selected from alkylating agents, antimetabolites, antitumor antibiotics, vinca alkaloids, epipodophyllotoxins, nitrosoureas, hormonal and anti-hormonal agents, and toxins.

Suited alkylating agents are cyclophosphamide, chlorambucil, busulfan, melphalan, thiotepa, ifosphamide, or nitrogen mustard.

Suited antimetabolites are methotrexate, 5-Fluorouracil, cytosine arabinoside, 6-thioguanine, 6-mercaptopurin.

Suited antitumor antibiotics are doxorubicin, daunorubicin, idorubicin, nimitoxantron, dactinomycin, bleomycin, mitomycin, and plicamycin.

Suited spindle poisons are maytansine and maytansinoids, vinca alkaloids and epipodophyllotoxins may be exemplified by vincristin, vinblastin, vindestin, Etoposide, Teniposide.

Furthermore, suited taxane agents may be exemplified by Paclitaxel, Docetaxel, SB-T-1214.

Suited nitrosoureas are carmustine, lomustine, semustine, streptozocin.

Suited hormonal and anti-hormonal agents are adrenocorticoids, estrogens, antiestrogens, progestins, aromatase inhibitors, androgens, anti-androgens.

Suited random synthetic agents are dacarbazine, hexamethylmelamine, hydroxyurea, mitotane, procarbazide, cisplatin, carboplatin.

Suited monocytes chemotactic factors are f-Met-Leu-Phe (fMLP), f-Met-Leu-Phe-o-methyl ester, formyl-norleucyl-phenylalanine, formyl-methionyl-phenylalanine.

Suited NK cell attracting factors are IL-12, IL-15, IL-18, IL-2, and CCL5, the FC portion of an antibody.

In one embodiment the effector moiety X is an antibody Fc-region or fragment thereof.

In one embodiment the human antibody Fc-region is of human IgG1 subclass, or of human IgG2 subclass, or of human IgG3 subclass, or of human IgG4 subclass.

In one embodiment the antibody Fc-region is a human antibody Fc-region of the human IgG1 subclass, or of the human IgG4 subclass.

In one embodiment the human antibody Fc-region comprises a mutation of the naturally occurring amino acid residue at least at one of the following amino acid positions 228, 233, 234, 235, 236, 237, 297, 318, 320, 322, 329, and/or 331 to a different residue, wherein the residues in the antibody Fc-region are numbered according to the EU index of Kabat.

In one embodiment the human antibody Fc-region comprises a mutation of the naturally occurring amino acid residue at position 329 and at least one further mutation of at least one amino acid residue selected from the group comprising amino acid residues at position 228, 233, 234, 235, 236, 237, 297, 318, 320, 322 and 331 to a different residue, wherein the residues in the Fc-region are numbered according to the EU index of Kabat. The change of these specific amino acid residues results in an altering of the effector function of the Fc-region compared to the non-modified (wild-type) Fc-region.

In one embodiment the human antibody Fc-region has a reduced affinity to the human FcγRIIIA, and/or FcγRIIA, and/or FcγRI compared to a conjugate comprising the corresponding wild-type IgG Fc-region.

In one embodiment the amino acid residue at position 329 in the human antibody Fc-region is substituted with glycine, or arginine, or an amino acid residue large enough to destroy the proline sandwich within the Fc-region.

In one embodiment the mutation in the human antibody Fc-region of the naturally occurring amino acid residue is at least one of S228P, E233P, L234A, L235A, L235E, N297A, N297D, P329G, and/or P331S.

In one embodiment the mutation is L234A and L235A if the antibody Fc-region is of human IgG1 subclass, or S228P and L235E if the antibody Fc-region is of human IgG4 subclass.

In one embodiment the antibody Fc-region comprises the mutation P329G.

The effector moiety can be bound either covalently or via an additional binding pair to at least one of the components of the complex. The effector moiety can be comprised for one to several (n) times in the complex as reported herein, whereby (n) is an integer and 0 or 1 or more than one. In one embodiment (n) is between 1 and 1,000,000. In one embodiment (n) is between 1,000 and 300,000. In one embodiment (n) is 1 to 50. In one embodiment (n) is 1 to 10, or 1 to 5. In one embodiment (n) is 1 or 2

For covalent binding of the effector moiety to at least one of the components in the complex any appropriate coupling chemistry can be used. It is also possible to incorporate a functional moiety by use of appropriate building blocks when synthesizing the members of the first and/or second binding pair and/or the (polynucleotide) linker, especially in the members of the binding pairs conjugated to the polypeptide or the (polynucleotide) linker.

Conjugation methods resulting in linkages which are substantially (or nearly) non-immunogenic are especially suited. Therefore, peptide- (i.e. amide-), sulfide-, (sterically hindered), disulfide-, hydrazone-, or ether linkage are especially suited. These linkages are nearly non-immunogenic and show reasonable stability within serum (see e.g. Senter, P.D., Curr. Opin. Chem. Biol. 13 (2009) 235-244; WO 2009/059278; WO 95/17886).

In one embodiment the effector moiety is bound to the (polynucleotide) linker of the complex as reported herein.

In one embodiment the effector moiety is covalently bound to a member of a binding pair conjugated to the polypeptide or the (polynucleotide) linker of the complex as reported herein.

If an effector moiety is located within a hybridizing polynucleotide it is especially suited to bind it to a modified nucleotide or is attached to the internucleosidic P atom (see e.g. WO 2007/059816).

Bifunctional building blocks (as described above) can be used to connect an effector moiety or a - if necessary - a protected effector moiety to a phosphoramidite group for attaching the building block at the 5'-end (regular synthesis) or at the 3'-end (inverted synthesis) to the terminal hydroxyl group of a growing polynucleotide chain.

Trifunctional building blocks (as described above) can be used to connect (i) a effector moiety or a - if necessary - a protected effector moiety, (ii) a phosphoramidite group for coupling the reporter or the effector moiety or a - if necessary - a protected effector moiety, during the polynucleotide synthesis to a hydroxyl group of the growing polynucleotide chain and (iii) a hydroxyl group which is protected with an acid labile protecting group especially with a dimethoxytrityl protecting group. After removal of this acid labile protecting group a hydroxyl group is liberated which can react with further phosphoramidites.

The effector moiety is bound in one embodiment to at least one of the members of the first and/or second binding pair or to the polynucleotide linker via an additional third binding pair. In one embodiment the third binding pair is a pair of hybridizing nucleic acid sequences. The members of the third binding pair do not interfere with the binding of the members of the other binding pairs to each other.

The additional binding pair to which an effector moiety can be bound is especially a leucine zipper domain or a hybridizing nucleic acid. In case the effector moiety is bound to at least one of the members of the first and/or second binding pair or the (polynucleotide) linker via an additional binding pair member, the binding pair member to which the effector moiety is bound and the first and second binding pairs members, respectively, all are selected to have different specificity. The members of the first and second binding pair and the binding pair to which the effector moiety is bound each bind to (e.g. hybridize with) their respective partner without interfering with the binding of any of the other binding pairs.

In one embodiment the complementary nucleic acids of the binding pairs and/or the (polynucleotide) linker is made at least partly of L-DNA, or L-RNA, or LNA, or iso-C nucleic acid, or iso-G nucleic acid, or any combination thereof. In one embodiment the (polynucleotide) linker is made at least to 50 % of L-DNA, or L-RNA, or LNA, or iso-C nucleic acid, or iso-G nucleic acid, or any combination thereof. In one embodiment the (polynucleotide) linker is an L-polynucleotide (a spiegelmer). In one embodiment the L-polynucleotide is L-DNA.

In one embodiment the (polynucleotide) linker is DNA. In one embodiment the (polynucleotide) linker is the L-stereoisomer of DNA also known as beta-L-DNA or L-DNA or mirror image DNA.

This stereoisomeric DNA features advantages like orthogonal hybridization behavior, which means that a duplex is formed only between two complementary single strands of L-DNA but no duplex is formed between a single strands of L-DNA and the complementary D-DNA strand, nuclease resistance and ease of synthesis even of a long linker. The ease of synthesis and variability in spacer length are important for providing a linker library. (Polynucleotide) Linkers of variable length are useful in identifying complexes as reported herein having a polynucleotide linker of optimal length, thus, providing for the optimal distance between two polypeptide specifically binding a target.

In one embodiment the complex is a non-covalent complex. In one embodiment the non-covalent complex is formed via binding pairs.

In some embodiments, the effector moiety is a therapeutic drug.

For instance, the effector moiety can be a therapeutic radionuclide, hormone, cytokine, interferon, antibody or antibody fragment, nucleic acid aptamer, enzyme, polypeptide, toxin, cytotoxin, a chemotherapeutic agent, or a radiation sensitizer.

Reported herein is a method of using the complex as reported herein.

For example, herein is reported a method of killing a cell, wherein a complex as reported herein is administered to the cell in an amount sufficient to kill the cell.

In one example, the cell is a cancer cell.

Herein is also reported a method of retarding or stopping the growth of a cancer cell in a mammal, wherein a complex as reported herein is administered to the mammal in an amount sufficient to retard or stop growth of the cancer cell.

In one example the method is a method for inhibiting the growth or proliferation of a cancer cell.

In one embodiment the polypeptide specifically binding to a target is specifically binding to a cell surface molecule of a cell. In one embodiment the cell surface molecule is specifically present on cancer cells.

In one example the first and second polypeptide specifically binding to a target are independently from each other selected from the group consisting of an antibody, an antibody fragment, a single-chain variable region antibody, a small peptidic molecule, a cyclic polypeptide, a peptidomimetic, and an aptamer.

In one example the first and the second polypeptide specifically binding to a target are monovalent binding polypeptides.

In one embodiment the polypeptide is an antibody fragment. In one embodiment the antibody fragment is from an internalizing antibody that specifically binds to a cell surface molecule.

The conjugation of an effector moiety to a complex as reported herein allows for specific localization of the effector moiety at the desired site on a cell. The localization increases the effective concentration of the effector moiety on the target cell and thereby optimizes the effect of the effector moiety. Furthermore, the complex can be administered at a lower dose compare to a non-targeted effector moiety. This can be particularly relevant if the effector moiety has associated toxicities or if it is to be used in the treatment of chronic diseases.

L-DNA is a useful nucleotide in the formation of complexes as reported herein. L-DNA does not, by itself, hybridize to the naturally occurring form of DNA (D-DNA) or RNA. Since L-DNA is not a natural substrate for many enzymes, the stability of an L-DNA in vivo can be greater than that of D-DNA. L-DNA duplexes have the same physical characteristics in terms of solubility, duplex stability and selectivity as D-DNA but form a left-helical double-helix. It is to be understood that the L-polynucleotide as used herein may also comprise some D-polynucleotides.

Due to the chemical nature of the L-polynucleotides these are not metabolized so that the pharmacokinetics underlying the use of L-nucleotides is not or at least not to such an extend affected by DNA specific degradation processes. In view of the increased stability of the L-polynucleotides the in vivo half-life of the complex as reported herein in a mammal is, thus, factually unlimited. Of particular importance is the fact that the L-polynucleotides are not nephrotoxic.

In one example the mammal is selected from humans, monkeys, dogs, cats, horses, rats, or mice. In one embodiment the polynucleotide linker comprises D-DNA and L-DNA nucleotides, i.e. the polynucleotide linker is a mixture of D-DNA and L-DNA.

With this linker it is possible to engineer the half-life of the polynucleotide linker, i.e. the in vivo half-life of the oligonucleotide linker can be tailor made and adjusted to the intended application of the complex.

Each of the polynucleotides present in the complex as reported herein can comprise one or more effector moieties. Effector moieties allow the use of the complex as reported herein in the treatment of a disease. The effector moieties can be used e.g. for carrier purposes, i.e. the delivery of an effector function, and/or modulation of pharmacokinetic behavior, and/or modulation of the physico-chemical properties.

In one embodiment the effector moiety is selected from lipophilic moieties, peptides, proteins, carbohydrates and liposomes.

In one embodiment the polynucleotide is an L-polynucleotide.

The L-poly (deoxy) nucleotides can be present either as single- or as double-stranded polynucleotide. Typically, the L-poly (deoxy) nucleotide is present as single-stranded nucleic acid, which may form (defined) secondary structures and also tertiary structures. In such secondary structures also double-stranded stretches can be present. The L-poly (deoxy) nucleotide, however, can also be present at least partly as double-stranded molecule in the meaning that two strands, which are complementary to each other, are hybridized. The L-polynucleotide(s) can also be modified. The modification can be related to the individual nucleotides of the polynucleotide.

In order to avoid secondary structure formation 2,4-Dihydroxy-5-methylpyrimidin (T) can be used as nucleobase in one embodiment.

The L-polynucleotides in the complex as reported herein are in one embodiment susceptible to "self-hybridization".

Thus, the L-polynucleotides are more readily able to hybridize with complementary L-polynucleotide sequences but do not form a stable duplex with natural DNA or RNA.

In one embodiment, the nucleotides in the L-DNA segment have a conformation of 1'S, 3'R, and 4' S.

In one embodiment, the L-DNA polynucleotide linker is conjugated through hybridization of the members of the binding pairs at its termini with the polypeptide(s) of the complex.

In one embodiment the polynucleotide linker has a length of at least 1 nm. In one embodiment the polynucleotide linker has a length of from 6 nm to 100 nm. In one embodiment the polynucleotide linker has a length of at least 70 nucleotides.

The polynucleotide linker may also comprise a tag sequence. The tag sequence may be selected from commonly used protein recognition tags such as YPYDVPDYA (HA-Tag, SEQ ID NO: 64) or GLNDIFEAQKIEWHE (Avi-Tag, SEQ ID NO: 65).

Thus, in one example of the methods as reported herein, the complex as reported herein not comprising an effector moiety is administered first and allowed to bind to its target(s) and afterwards the effector moiety conjugated to a polynucleotide complementary to at least a part of the (polynucleotide) linker is administered. Thereby the effector moiety is co-located to the complex bound to its target by hybridizing to the complex as reported herein in situ.

It is to be understood that the complex as reported herein is not limited to any specific nucleic acid sequence, or any binding entity (polypeptide) specifically binding to a target, or to specific cell types, or to specific conditions, or to specific methods, etc., as such may vary and the numerous modifications and variations therein will be apparent to those skilled in the art.

In one embodiment of the methods as reported herein the complex binds to the cell surface of a tumor cell and locally enriches to a high density or high local concentration of the effector moiety.

In one example the effector moiety is labeled ss-L-DNA, which is administered simultaneously or subsequently to the initial target association of the complex.

The labeled ss-L-DNA effector moiety hybridizes to ss-L-DNA (oligonucleotide) linker of the complex.

The target bound complex is used to activate the innate immune response, namely to attract cytotoxic lymphocytes, also called natural killer cells (NK cells). NK cells play a major role in the rejection of tumors and cells infected by viruses. They kill cells by releasing small cytoplasmic granules of proteins called perforin and granzyme that cause the target cell to die by apoptosis.

In one example the complex as reported herein is used to attract NK cells into close proximity of the bound complex. In one embodiment ss-L-DNA conjugated to a cytokine is used as effector moiety.

This cytokine labeled effector moiety can be used to attract NK cells. Cytokines involved in NK activation include IL-12, IL-15, IL-18, IL-2, and CCL5.

In one embodiment ss-L-DNA conjugated to an Fc portion of an antibody is used as effector moiety.

NK cells, along with macrophages and several other cell types, express the Fc receptor (FcR) molecule (FC-gamma-RIII = CD16), an activating biochemical receptor that binds the Fc portion of antibodies. This allows NK cells to target cells against which a humoral response has been mobilized and to lyse cells through antibody-dependent cellular cytotoxicity (ADCC).

In one embodiment, one or more or a combination of ss-L-DNA conjugated to one or more Fc parts is / are used as effector moieties.

In this embodiment the complex can be used to modulate the ADCC and/or the complement activation (CDC).

In one example this complex is used in a method to screen engineered Fc compartments for their efficacy in engaging ADCC and CDC.

In one example the complex is used to inhibit seminal fluid phosphatase.

In this example the complex can be used to avoid NK cell inactivation.

In one embodiment the polypeptide specifically binding to a target, such as an antibody or antibody fragment specifically binding to a cell surface molecule, is conjugated to a ligand for a target receptor or large molecule that is more easily engulfed by the endocytotic mechanisms of a cell in order to increase the uptake of the complex into the cell presenting the target.

The target bound complex can then be internalized by endocytosis and the effector moiety released inside the cell.

The binding entity (polypeptide) specifically binding to a target is in one embodiment an antibody fragment.

The term "single-chain variable region fragment" or "scFv" denotes a variable, antigen-binding region of a single antibody light chain and single antibody heavy chain linked together by a covalent linkage having a length sufficient to allow the light and heavy chain portions to form an antigen binding site. Such a linker may be as short as a covalent bond. Especially suited linkers comprise of from 2 to 50 amino acid residues, and especially of from 5 to 25 amino acid residues.

Other antibody fragments are diabodies, first described by Holliger, P., et al. (PNAS (USA) 90 (1993) 6444-6448). These may be constructed using heavy and light chains of an antibody, as well as by using individual CDR regions of an antibody. Typically, diabodies comprise a heavy chain variable domain (VH) connected to a light chain variable domain (VL) by a linker which is too short to allow pairing between the two domains on the same chain. Accordingly, the VH and VL domains of one fragment are forced to pair with the complementary VH and VL domains of another fragment, thereby forming two antigen-binding sites. Triabodies can be similarly constructed with three antigen-binding sites.

An Fv antibody fragment contains a complete antigen-binding site which includes a VL domain and a VH domain held together by non-covalent interactions. Fv fragments also include constructs in which the VH and VL domains are crosslinked through glutaraldehyde, intermolecular disulfide bonds, or other linkers. The variable domains of the heavy and light chains can be fused together to form a single chain variable fragment (scFv), which retains the original specificity of the parent antibody. Single chain Fv (scFv) dimers, first described by Gruber, M., et al., J. Immunol. 152 (1994) 5368-5374, may be constructed using heavy and light chains of an antibody, as well as by using individual CDR regions of an antibody. Many techniques known in the art can be used to prepare the specific binding constructs suitable in the complex as reported herein (see e.g., US 2007/0196274, US 2005/0163782).

Bispecific antibodies can be generated by chemical cross-linking or by the hybrid hybridoma technology. Alternatively, bispecific antibody molecules can be produced by recombinant techniques. Dimerization can be promoted by reducing the length of the linker joining the VH and the VL domain from about 15 amino acids, routinely used to produce scFv fragments, to about 5 amino acids. These linkers favor intrachain assembly of the VH and VL domains. A suitable short linker is SGGGS (SEQ ID NO: 66) but other linkers can be used. Thus, two fragments assemble into a dimeric molecule. Further reduction of the linker length to zero to two amino acid residues can generate trimeric (triabodies) or tetrameric (tetrabodies) molecules.

In one embodiment the binding entity (polypeptide) specifically binding to a target, e.g. an antibody specifically binding to a cell surface receptor, can be linked to a ligand for a target receptor or large molecule that is more easily engulfed by the cell's endocytotic mechanisms.

In this embodiment the complex can be used to increase the uptake of the complex into the cell presenting the target. The target bound complex can then be internalized by endocytosis and the effector moiety released by acid hydrolysis or enzymatic activity when the endocytotic vesicle fuses with lysosomes.

The complexes as reported herein can be used to deliver the effector moiety intracellularly and extracellularly. The complex can be used to recognize cancer cells in situ making them attractive candidates for the development of targeted therapeutics.

When the non-covalent association of a component to another component (or to a particle or capsule) is desired, appropriate associative interactions that may be employed include, but are not limited to, antibody-antigen, receptor-hormone, avidin-biotin pairs, streptavidin-biotin, metal-chelate, small molecule/polynucleotide (see, e.g., Dervan, P.B., Bioorg. Med. Chem. 9 (2001) 2215-2235; Zahn, Z.Y. and Dervan, P.B., Bioorg. Med. Chem. 8 (2000) 2467-2474); polynucleotide/complementary polynucleotide (e.g., dimeric and trimeric helices), aptamer/small molecule, aptamer/polypeptide, coiled-coil, and polynucleotide/polypeptide (e.g. zinc finger, helix-tum-helix, leucine zipper, and helix-loop-helix motifs that bind to DNA sequences).

The complex as reported herein can be used to deliver a variety of effector moieties such as cytotoxic drugs including therapeutic drugs, components emitting radiation, molecules of plants, fungal, or bacterial origin, biological proteins, and mixtures thereof to a cell. The cytotoxic drug, e.g., can be an intracellularly acting cytotoxic drug, such as short-range radiation emitters, including, for example, short-range, high-energy α-emitters.

In one embodiment the effector moiety is a liposome encapsulating a drug (e.g. an anti-cancer drug such as abraxane, doxorubicin, pamidronate disodium, anastrozole, exemestane, cyclophosphamide, epirubicin, toremifene, letrozole, trastuzumab, megestroltamoxifen, paclitaxel, docetaxel, capecitabine, goserelin acetate, zoledronic acid, vinblastine, etc.), an antigen that stimulates recognition of the bound cell by components of the immune system, an antibody that specifically binds immune system components and directs them to the cell, and the like.

In one embodiment the effector moiety can comprise a radiosensitizer that enhances the cytotoxic effect of ionizing radiation (e.g., such as might be produced by ⁶⁰Co or an X-ray source) on a cell.

In one embodiment the effector moiety is selected from monocytes chemotactic factors, or f-Met-Leu-Phe (fMLP), or f-Met-Leu-Phe-o-methyl ester, or formyl-norleucyl-phenylalanine, or formyl-methionyl-phenylalanine, or derivatives thereof.

In one embodiment the effector moiety is a reactive group.

The reactive group can be selected from any known reactive group, like Amino, Sulflrydryl, Carboxylate, Hydroxyl, Azido, Alkinyl or Alkenyl.

In one embodiment the reactive group is selected from Maleinimido, Succinimidyl, Dithiopyridyl, Nitrophenylester, Hexafluorophenylester.

If the mode of action depends on creating on a target a high local concentration of an effector like in the case of fMLP as effector moiety, the L-DNA nature of the linker entities allows specific hybridization with a second L-DNA oligonucleotide modified with the same or a different effector moiety.

The number of effector moieties which are bound to the second L-DNA has to be limited in order that there is no response induced by the single effector modified L-DNA. If desired, the second L-DNA compromises a further site which is capable of specifically hybridizing with a third L-DNA oligonucleotide modified with the same or a different effector moiety. Since it is easy to select many different sequences which form specifically a duplex in the presence of other duplexes a multimeric complex can be built up easily.

Multimeric complexes can be built up by using oligonucleotides with overlapping sequences to form a linear multimeric complex or by using branched oligonucleotides, wherein the branches are capable of hybridizing with a third oligonucleotide which results in formation of dendritic, multimeric complexes.

In one embodiment the effector moiety is an alpha emitter, i.e. a radioactive isotope that emits alpha particles. Suitable alpha emitters include, but are not limited to Bi, ²¹³Bi, ²¹¹At, and the like.

The effector moiety can also comprise a ligand, an epitope tag, an antibody Fc-region, or an antibody.

Enzymatically active toxins and fragments thereof can be selected from diphtheria toxin A fragment, non-binding active fragments of diphtheria toxin, exotoxin A (from Pseudomonas aeruginosa), ricin A chain, abrin A chain, modeccin A chain, .alpha.-sacrin, certain Aleurites fordii proteins, certain Dianthin proteins, Phytolacca americana proteins (PAP, PAPII and PAP-S), Morodica charantia inhibitor, curcin, crotin, Saponaria officinalis inhibitor, gelonin, mitogillin, restrictocin, phenomycin, and enomycin.

In one embodiment one or more L-DNA oligonucleotides, modified with a high density of caged effector moieties are hybridized to the L-DNA linker.

Cancer cells differ from normal cells in a variety of ways, one of which is the molecular composition of the cell surface. The altered surface chemistry allows cancer cells to respond efficiently to external signals for growth and survival and to interact directly with a variety of host tissue elements to migrate, enter the circulation, extravasate, and become colonized at a distant site. Besides serving as markers for malignant cells, tumor cell surface molecules are valuable targets for therapy due to their relatively easy accessibility to targeting molecules administered to the bloodstream or extracellular space (Feng, A., et al., Mol. Cancer Ther. 7 (2008) 569-578).

Contemplated tumor specific antigens include, but are not limited, to CEA, CD20, HER1, HER2/neu, HER4, PSCA, PSMA, CA-125, CA-19-9, c-Met, MUC1, RCAS1, Ep-CAM, Melan-A/MART1, RHA-MM, VEGF, EGFR, integrins, ED-B of fibronectin, ChL6, Lym-1, CD1b, CD3, CD5, CD14, CD20, CD22, CD33, CD56, TAO-72, interleukin-2 receptor (IL-2R), ferritin, neural cell adhesion molecule (NCAM), melanoma-associated antigen, ganglioside Gm, EOF receptor, tenascin, c-Met (HGFR).

In one embodiment the antibody is specifically binding to a post-translationally modified target on a cell surface receptor. In one embodiment the post-translationally target is modified by phosphorylation or glycosylation.

In one embodiment the first polypeptide and the second polypeptide bind to the same or an overlapping epitope.

It has been found that a posttranslationally modified target polypeptide can be detected by a complex consisting of two monovalent polypeptides specifically binding to a target that are linked to each other via a polynucleotide linker, wherein the first polypeptide binds to a polypeptide epitope of the target, the second polypeptide binds to a posttranslational polypeptide modification, wherein each monovalent binder has a Kdiss in the range of 10⁻²/sec to 10⁻³/sec, and wherein the complex has a Kdiss of 10⁻⁴/sec or less.

Different types of covalent amino acid modifications are known. (Mann, M. and Jensen, O.N., Biochemistry 21 (2003) 255-261; Seo, J. and Lee, K.-J., Biochemistry and Molecular Biology 37/1 (2004) 35-44).

In one embodiment the posttranslational modification is selected from the group consisting of acetylation, phosphorylation, acylation, methylation, glycosylation, ubiquitinylation, sumoylation, sulfatation and nitration.

Acetylation (+42 Da molecular weight change) is a rather stable secondary modification. Examples are the acetylation which is found on the N-termini of many proteins or the acetylation on lysine or serine residues. Usually acetylation of a lysine residue is found at one or more well-defined position(s) within a polypeptide chain, while other lysine residues are acetylated less frequently or not at all.

Phosphorylation and de-phosphorylation (the net balance of which may be referred to as phosphorylation status) of a protein is known to be one of the key elements in regulating a proteins biological activity. A low percentage of phosphorylated amino acid residues may already be sufficient to trigger a certain biological activity.

Phosphorylation results in a mass increase of 80 Da (molecular weight increase). The amino acids tyrosine (Y), serine (S), threonine (T), histidine (H), and aspartic acid (D) can be phosphorylated. The more complex the biological function of a polypeptide is the more complex the corresponding pattern of possible sites of phosphorylation is. This is especially known and true for membrane-bound receptors, especially the so-called receptor tyrosine kinases (RTKs). As the nomenclature already suggests, at least part of the intracellular signaling of the RTKs is mediated by the phosphorylation status of certain tyrosine of the intracellular domain of such RTKs.

Polypeptides may be acylated by farnesyl, myristoyl or palmitoyl groups. Acylation usually occurs on the side chain of a cysteine residue.

Methylation as a secondary modification occurs via the side chain of a lysine residue. It has been shown that the binding properties of regulatory proteins that are able to bind to a nucleic acid can e.g. be modulated via methylation.

Glycosylation is a common secondary modification. It has a major influence on protein-protein interactions, on solubilization of proteins, their stability, also. Two different types of glycosylation are known: the N-linked (via the amino acid N (asparagine)) side chains and the O-linked side chains (via serine (S) or threonine (T)). Many different polysaccharides (linear or with branched side chains), some containing sugar derivatives like O-Glc-NAc, have been identified.

Ubiquitinylation and sumoylation, respectively, are known to influence the half-life of proteins in the circulation. Ubiquitinylation may serve as a destruction signal, resulting in cleavage and/or removal of ubiquitinylated polypeptides.

Sulfatation via a tyrosine residue (Y) appears to be important in the modulation of protein-protein (cell-cell) interaction as well as in protein ligand-interaction.

Nitration of tyrosine residues (Y) appears to be a hall-mark of oxidative damage as e.g. in inflammatory processes.

The L-deoxynucleoside phosphoramidite units L-dT, L-dC, L-dA and L-dG can be prepared according to the literature (see e.g. Urata, H., et al., Nucl. Acids Res. 20 (1992) 3325-3332; Shi, Z.D., et al., Tetrahed. 58 (2002) 3287-3296). The L-deoxyribose derivative can be synthesized from L-arabinose through 8 steps. The L-deoxynucleosides can be obtained by a glycosylation of appropriate nucleobase derivatives with the L-deoxyribose derivative. After derivatization to nucleoside phosphoramidites, they can be incorporated into oligodeoxynucleosides by a solid phase DNA synthesis method. The oligomer can be purified by reverse phase HPLC and poly acrylamide gel electrophoresis (PAGE).

L-DNA can be synthesized like DNA in large scales by using standard synthesis protocols.

For expression and purification of scFv antibody fragments the scFv encoding nucleic acid can be cloned into an expression and/or secretion vector, such as pUC119mycHis, which would result in the addition of a c-myc epitope tag and hexahistidine tag at the C-terminus of the scFv. To create the (scFv')₂ dimer for immunohistochemistry (Adams, G.P., et al., Cancer Res. 53 (1993) 4026-4034), the c-myc epitope tag can be genetically removed from pUC119mycHis, and a free cysteine can be introduced at the C-terminus of the scFv preceding the hexahistidine tag. scFv or (scFv')₂ dimer protein can be harvested from the bacterial periplasm and purified by immobilized metal affinity chromatography and gel filtration (Nielsen, U.B., et al., Biochim. Biophys. Acta 1591 (2002) 109-118).

Alternatively scFvs can be produced by introducing the structural genes encoding scFvs an expression vector imparting a c-myc and a hexahistidine tag at the C-terminus (Liu, B., et al., Cancer Res. 64 (2004) 704-710). To produce soluble (scFv)₂, a second vector can be used to impart a cysteine and a hexahistidine tag at the C-terminus. Following IPTG induction, antibody fragments can be purified from bacterial periplasmic space on nitrilotriacetic acid-nickel beads. For FACS and immunohistochemistry studies, scFvs can be biotinylated using EZ-Link Sulfo-NHS-LC-Biotin (Pierce) according to the manufacturer's instructions.

For dissociation constant (KD) determination the a cell line expressing the respective target surface molecule can be grown to 90 % confluency in suitable medium such as RPMI 1640 supplemented with 10 % FCS. The cells can be harvested by brief digestion with trypsin (0.2 %) in 2 mmol/l EDTA/PBS. Biotinylated scFvs can be incubated with 10⁵ cells for 4 h at 4 °C in PBS/0.25 % bovine serum albumin. Bound scFvs can be detected by streptavidin-phycoerythrin and analyzed by FACS. Data can be curve fitted and KD values can be calculated using GraphPad Prism (Graph-Pad Software).

For immunohistochemistry tissue sections from frozen and/or paraffin-embedded blocks can be used. For immunohistochemical analysis, tissue sections can be incubated with purified dimeric scFv (e.g. 50 µg/ml in 2 % milk/PBS) at 4 °C for four hours, washed with PBS, incubated with an anti-(His)6 antibody diluted 1:400 (Santa Cruz Biotechnology), followed by biotinylated anti-anti-(His)6 antibody antibody diluted 1:400 (Vector Lab) and horseradish peroxidase-conjugated streptavidin diluted 1:400 (Sigma). Binding can be detected using diaminobenzidine as the substrate (Sigma).

Alternatively frozen sections of test and control tissues can be stained with biotinylated scFvs (250 nmol/l) at room temperature for one hour. A scFv that does not bind to the test cell lines by FACS can be used as a control for all experiments. Bound scFvs can be detected by streptavidin-horseradish peroxidase using 3,3'-diaminobenzidine substrate. The stained tissues can be counter-stained with hematoxylin, dried in 70 %, 95 % and 100 % ethanol, mounted and analyzed.

Specifically see US 2003/0152987 concerning immunohistochemistry (IHC) and fluorescence in situ hybridization (FISH) for detecting HER2 overexpression and amplification.

For the determination of internalization the following procedure can be used. For fluorescence microscopy experiments, cells can be grown to about 80 % confluency in 24-well plates and co-incubated with non-targeted or targeted complexes labeled with 1,1'-dioctadecyl-3,3,3',3'-tetramethylindocarbocyanine-5,5'-disulfonic acid for four hours at 37 °C. The cells can be washed with PBS and examined with a Nikon Eclipse TE300 fluorescence microscope. For FACS analysis, cells can be incubated with 1,1'-dioctadecyl-3,3,3',3'-tetramethylindocarbocyanine-5,5'-disulfonic acid-labeled complexes at 37 °C for two hours, removed from the dish by trypsin digestion, exposed to glycine buffer (pH 2.8; 150 mmol/l NaCl) at room temperature for 5 min. to remove surface-bound liposomes, and analyzed by FACS (LSRII; BD Biosciences). Mean fluorescence intensity values can be used to calculate the percentage of internalized liposomes (resistant to glycine treatment) over total cell associated liposomes (before glycine treatment).

For a growth inhibition and internalization assays cancer cells at about 30 % - 80 % confluence can be incubated with various concentrations of affinity-purified complex at 37 °C for 72 h in medium containing 1 % FCS. Growth status can be assessed using the tetrazolium salt 3-(4,5-dimethylthizaol-2-yl)-2,5-diphenyltetrazolium bromide assay (Promega), and the IC₅₀ can be calculated using KaleidaGraph 3.5 (Synergy Software). For internalization assays, the complex can be biotinylated with sulfo-NHS-LC-biotin (Pierce) and incubated with target cells at 37 °C for various amounts of time. Cells can be washed with 100 mM glycine buffer (pH 2.8), fixed with 2 % formaldehyde, permeabilized with ice-cold 100 % methanol, and incubated with streptavidin-FITC. The stained cells can be first examined with an Axiophot fluorescence microscope (Zeiss) and further studied with a Leica TCS NT confocal laser fluorescence microscope (Leica).

For toxicity determination cells can be plated at 6,000 per well in 96-well plates and incubated with a complex as reported herein at varying concentrations (0-10 µg/ml) for two hours at 37 °C. After removal of the complex, the cells can be washed once with RPMI 1640 supplemented with 10 % FCS and incubated for an additional 70 h at 37 °C. The cell viability can be assayed using Cell Counting Kit-8 (Dojindo) according to the manufacturer's instructions. The data can be expressed as the percent of viable cells compared with that of untreated control cells.

For in vitro cytotoxicity determination cancer cells can be seeded in 96-well plates (6,000 cells per well for e.g. PC3 and Du-145 cells or 10,000 cells per well for e.g. LNCaP cells) and incubated with the complex as reported herein (0 - 10 µg/ml) for 4 h at 37 °C. Cells can be washed twice with supplemented RPMI 1640 to remove drugs and incubated with fresh medium for an additional 72 h at 37 °C. Cell viabilities can be assayed using the 3-(4,5-dimethylthiazol-2-yl)-2,5-diphenyltetrazolium bromide staining (Carmichael, J., et al., Cancer Res. 47 (1987) 936-942), and the results can be read at 570 nm using a microtiter plate reader (SpectraMax 190, Molecular Devices). The cell viability can be determined using the Cell counting kit-8 (Dojindo) according to the manufacturer's instructions.

For an assay of intracellular delivery the following method can be used. To assess intracellular complex delivery, the complex can be added to cells along with 1 µg/ml of purified (His)6-tagged scFv, incubated at 37 °C for 30 min, and washed three times with saline containing 1 mM EDTA to remove cell surface-bound complexes that failed to internalize. Uptake of the complex can be determined by microfluorimetry with a Gemini microfluorometer (Molecular Devices) and by an inverted fluorescence microscope (Nikon).

### Recombinant Methods and Compositions

Generally binding entities such as antibody fragments or members of a binding pair may be produced using recombinant methods and compositions, e.g., as described in US 4,816,567.

In one example isolated nucleic acid encoding each binding entity of the complex as reported herein is provided.

Such nucleic acid may encode an amino acid sequence comprising the VL and/or an amino acid sequence comprising the VH of an antibody (e.g., the light and/or heavy chains of the antibody) and/or an amino acid sequence of a member of a binding pair.

In one example a host cell comprising such nucleic acid is provided. In one embodiment a host cell is provided that comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one example the host cell is a eukaryotic cell, e.g. a Chinese Hamster Ovary (CHO) cell or lymphoid cell (e.g., Y0, NS0, Sp20 cell). For expression of antibody fragments and polypeptides in bacteria, see, e.g., US 5,648,237, US 5,789,199, and US 5,840,523; Charlton, Methods in Molecular Biology, Vol. 248, B.K.C. Lo, (ed.), Humana Press, Totowa, NJ, (2004) pp. 245-254. After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors (see Gerngross, T.U., Nat. Biotech. 22 (2003) 1409-1414), and Li, H., et al., Nat. Biotech. 24 (2006) 210-215).

Plant cell cultures can also be utilized as hosts (see, e.g., US 5,959,177, US 6,040,498, US 6,420,548, US 7,125,978, and US 6,417,429).

Vertebrate cells may also be used as hosts. For example, mammalian cell lines that are adapted to grow in suspension may be useful. Other examples of useful mammalian host cell lines are monkey kidney CV1 line transformed by SV40 (COS-7), human embryonic kidney line (HEK 293), baby hamster kidney cells (BHK), mouse sertoli cells (TM4 cells as described, e.g., in Mather, J.P., Biol. Reprod. 23 (1980) 243-252), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells, as described, e.g., in Mather, J.P., et al., Annals N.Y. Acad. Sci. 383 (1982) 44-68, MRC 5 cells, and FS4 cells. Other useful mammalian host cell lines include Chinese hamster ovary (CHO) cells, including DHFR⁻ CHO cells (Urlaub, G., et al., Proc. Natl. Acad. Sci. USA 77 (1980) 4216-4220), and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

The host cells used to produce the polypeptides of the complex as reported herein can be cultured in a variety of media. Commercially available media such as Ham's F10 (Sigma), Minimal Essential Medium ((MEM), (Sigma), RPMI-I640 (Sigma), and Dulbecco's Modified Eagle's Medium ((DMEM), Sigma) are suitable for culturing the host cells. In addition, any of the media described in Ham, R.G., et al., Meth. Enzymol. 58 (1979) 44-93, Barnes, D., et al., Anal. Biochem. 102 (1980) 255-270, US 4,767,704, US 4,657,866, US 4,927,762, US 4,560,655, US 5,122,469, WO 90/03430, WO 87/00195, and US Re 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN^{™} drug), trace elements (defined as inorganic components usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

When using recombinant techniques, the binding entity/polypeptide can be produced intracellularly, in the periplasmic space, or directly secreted into the medium. If the polypeptide is produced intracellularly, as a first step, the particulate debris, either host cells or lysed fragments, is removed, for example, by centrifugation or ultrafiltration. For example, Carter, P., et al., Bio/Technology 10 (1992) 163-167 describe a procedure for isolating antibodies which are secreted to the periplasmic space of E. coli. Briefly, cell paste is thawed in the presence of sodium acetate (pH 3.5), EDTA, and phenylmethylsulfonylfluoride (PMSF) over about 30 min. Cell debris can be removed by centrifugation. Where the polypeptide is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants. The polypeptide composition prepared from the cells can be purified using, for example, hydroxylapatite chromatography, gel electrophoresis, dialysis, and affinity chromatography, with affinity chromatography being the preferred purification technique for antibodies. The suitability of protein A as an affinity ligand depends on the species and subclass of any immunoglobulin Fc domain that is present in the antibody. Protein A can be used to purify antibodies that are based on human γ1, γ2, or γ4 heavy chains (Lindmark, R., et al., J. Immunol. Meth. 62 (1983) 1-13). Protein G is recommended for all mouse subclasses and for human γ3 (Guss, B., et al., EMBO J. 5 (1986) 1567-1575). The matrix to which the affinity ligand is attached is most often agarose, but other matrices are available. Mechanically stable matrices such as controlled pore glass or poly (styrene divinyl) benzene allow for faster flow rates and shorter processing times than can be achieved with agarose. Where the antibody comprises a CH3 domain, the Bakerbond ABX^{™}resin (J.T. Baker, Phillipsburg, NJ, USA) is useful for purification. Other techniques for protein purification such as fractionation on an ion-exchange column, ethanol precipitation, Reverse Phase HPLC, chromatography on silica, chromatography on heparin SEPHAROSE^{™} chromatography on an anion or cation exchange resin (such as a poly aspartic acid column), chromatofocusing, SDS-PAGE, and ammonium sulfate precipitation are also available depending on the antibody to be recovered.

Following any preliminary purification step(s), the mixture comprising the polypeptide of interest and contaminants may be subjected to low pH hydrophobic interaction chromatography using an elution buffer at a pH between about 2.5-4.5, especially performed at low salt concentrations (e.g., from about 0-0.25 M salt).

The complex and its individual components as reported herein can be isolated and purified as desired. Unwanted components of a reaction mixture in which the complex is formed are e.g. polypeptides and polynucleotides that did not end up in the desired complex but constitute its building blocks. In one embodiment the complex is purified to greater than 80 % purity as determined by analytical size exclusion chromatography. In some embodiments, the complex is purified to greater than 90 %, 95 %, 98 %, or 99 % purity by weight as determined by analytical size exclusion chromatography, respectively. Purity can alternatively e.g. be easily determined by SDS-PAGE under reducing or non-reducing conditions using, for example, Coomassie blue or silver stain in protein detection. In case purity is assessed on the complex level, size exclusion chromatography can be applied to separate the complex from side products and the OD at 260 nm is monitored to assess its purity.

### Immunoconjugates

Herein are also provided complexes in which at least one of the binding entities that specifically binds to a target or the linker is further conjugated to one or more effector moieties, e.g. cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment the effector moiety is a drug, including but not limited to a maytansinoid (see US 5,208,020, US 5,416,064, EP 0 425 235), an auristatin such as monomethylauristatin drug moieties DE and DF (MMAE and MMAF, see US 5,635,483, US 5,780,588, US 7,498,298), a dolastatin, a calicheamicin or derivative thereof (see US 5,712,374, US 5,714,586, US 5,739,116, US 5,767,285, US 5,770,701, US 5,770,710, US 5,773,001, US 5,877,296, Hinman, L.M., et al., Cancer Res. 53 (1993) 3336-3342, Lode, H.N., et al., Cancer Res. 58 (1998) 2925-2928), an anthracycline such as daunomycin or doxorubicin (see Kratz, F., et al., Current Med. Chem. 13 (2006) 477-523, Jeffrey, S.C., et al., Bioorg. Med. Chem. Letters 16 (2006) 358-362, Torgov, M.Y., et al., Bioconjug. Chem. 16 (2005) 717-721, Nagy, A., et al., Proc. Natl. Acad. Sci. USA 97 (2000) 829-834, Dubowchik, G.M., et al., Bioorg. Med. Chem. Lett. 12 (2002) 1529-1532, King, H.D., et al., J. Med. Chem. 45 (2002) 4336-4343, and US 6,630,579), methotrexate, vindesine, a taxane such as docetaxel, paclitaxel, larotaxel, tesetaxel, and ortataxel, a trichothecene, and CC1065.

In one embodiment the effector moiety is an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, Saponaria officinalis inhibitor, gelonin, mitogellin, restrictocin, phenomycin, enomycin, and the tricothecenes.

In one embodiment the effector moiety is a radioactive atom. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹², and radioactive isotopes of Lu. When the radioconjugate is used for detection, it may comprise a radioactive atom for scintigraphic studies, for example Tc⁹⁹m or I¹²³, or a spin label for nuclear magnetic resonance (NMR) imaging (also known as magnetic resonance imaging, MRI) such as I¹²³ again, I¹³¹, In¹¹¹, F¹⁹, C¹³, N¹⁵, O¹⁷, gadolinium, manganese or iron.

The effector moiety can be conjugated to any component of the complex as reported herein using a variety of bifunctional protein coupling agents such as N-succinimidyl-3-(2-pyridyldithio) propionate (SPDP), succinimidyl-4-(N-maleiimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido components (such as bis (p-azidobenzoyl) hexane diamine), bis-diazonium derivatives (such as bis-(p-diazoniumbenzoyl)-ethylene diamine), diisocyanates (such as toluene 2,6-diisocyanate), and bis-active fluorine components (such as 1,5-difluoro-2,4-dinitrobenzene). For example, a ricin immunotoxin can be prepared as described in Vitetta, E.S., et al., Science 238 (1987) 1098-1104. Carbon-14-labeled 1-isothiocyanatobenzyl-3-methyldiethylene triamine penta acetic acid (MX-DTPA) is an exemplary chelating agent for conjugation of radionucleotide to the complex (see WO 94/11026). The linker for conjugating the toxic moiety to the complex as reported herein can be a "cleavable linker" facilitating release of a cytotoxic drug in the cell. For example, an acid-labile linker, peptidase-sensitive linker, photolabile linker, dimethyl linker, or disulfide-containing linker (Chari, R.V., et al., Cancer Res. 52 (1992) 127-131, US 5,208,020) can be used.

The effector moiety may be conjugated to a compound of the complex as reported herein, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### Pharmaceutical Formulations

Pharmaceutical formulations of a multispecific binding molecule (such as a bispecific antibody) as reported herein are prepared by mixing such multispecific binding molecule having the desired degree of purity with one or more optional pharmaceutically acceptable carriers (Osol, A. (ed.), Remington's Pharmaceutical Sciences, 16th edition, Mack Publishing Company (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride, hexamethonium chloride, benzalkonium chloride, benzethonium chloride, phenol, butyl or benzyl alcohol, alkyl parabens such as methyl or propyl paraben, catechol, resorcinol, cyclohexanol, 3-pentanol, and m-cresol), low molecular weight (less than about 10 residues) polypeptides, proteins, such as serum albumin, gelatin, or immunoglobulins, hydrophilic polymers such as poly vinylpyrrolidone, amino acids such as glycine, glutamine, asparagine, histidine, arginine, or lysine, monosaccharides, disaccharides, and other carbohydrates including glucose, mannose, or dextrins, chelating agents such as EDTA, sugars such as sucrose, mannitol, trehalose or sorbitol, salt-forming counter-ions such as sodium, metal complexes (e.g. Zn-protein complexes), and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US 2005/0260186 and US 2006/0104968. In one aspect, a sHASEGP is combined with one or more additional glycosaminoglycanases such as chondroitinases.

Exemplary lyophilized antibody formulations are described in US 6,267,958. Aqueous antibody formulations include those described in US 6,171,586 and WO 2006/044908, the latter formulations including a histidine-acetate buffer.

The formulation herein may also contain more than one active ingredients as necessary for the particular indication being treated, especially those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. (ed.), (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for in vivo administration are generally sterile. Sterility may be readily accomplished, e.g., by filtration through sterile filtration membranes.

### Therapeutic Methods and Compositions

Any of the multispecific binding molecule (e.g. bispecific antibodies) as reported herein may be used in therapeutic methods.

Reported herein a pharmaceutical formulation comprising any of the multispecific binding molecules or bispecific antibodies as provided herein, e.g., for use in any of the above therapeutic methods is provided. In one example a pharmaceutical formulation comprises any of the multispecific binding molecules or bispecific antibodies provided herein and a pharmaceutically acceptable carrier. In another example a pharmaceutical formulation comprises any of the multispecific binding molecules or bispecific antibodies as reported herein and at least one additional therapeutic agent.

The multispecific binding molecules or bispecific antibodies as reported herein can be used either alone or in combination with other agents in a therapy. For instance, a multispecific binding molecule or bispecific antibody as reported herein may be co-administered with at least one additional therapeutic agent.

Such combination therapies noted above encompass combined administration (where two or more therapeutic agents are included in the same or separate formulations), and separate administration, in which case, administration of the multispecific binding molecule or bispecific antibody of the invention can occur prior to, simultaneously, and/or following, administration of the additional therapeutic agent and/or adjuvant. Multispecific binding molecules or bispecific antibodies as reported herein can also be used in combination with radiation therapy.

A multispecific binding molecule or bispecific antibody as reported herein can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Multispecific binding molecule and bispecific antibodies as reported herein would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The multispecific binding molecule or bispecific antibody need not be, but is optionally formulated with one or more agents currently used to prevent or treat the disorder in question. The effective amount of such other agents depends on the amount of multispecific binding molecule or bispecific antibody present in the formulation, the type of disorder or treatment, and other factors discussed above. These are generally used in the same dosages and with administration routes as described herein, or about from 1 % to 99 % of the dosages described herein, or in any dosage and by any route that is empirically/clinically determined to be appropriate.

For the prevention or treatment of disease, the appropriate dosage of a multispecific binding molecule or bispecific antibody as reported herein (when used alone or in combination with one or more other additional therapeutic agents) will depend on the type of disease to be treated, the type of multispecific binding molecule or bispecific antibody, the severity and course of the disease, whether the multispecific binding molecule or bispecific antibody is administered for preventive or therapeutic purposes, previous therapy, the patient's clinical history and response to the multispecific binding molecule or bispecific antibody, and the discretion of the attending physician. The multispecific binding molecule or bispecific antibody is suitably administered to the patient at one time or over a series of treatments. Depending on the type and severity of the disease, about 1 µg/kg to 15 mg/kg (e.g. 0.1 mg/kg-10 mg/kg) of multispecific binding molecule or bispecific antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the multispecific binding molecule or bispecific antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the multispecific binding molecule or bispecific antibody). An initial higher loading dose, followed by one or more lower doses may be administered. The progress of this therapy can be easily monitored by conventional techniques and assays.

### Articles of Manufacture

Reported herein an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a complex as reported herein. The label or package insert indicates that the composition is used for treating the condition of choice. Moreover, the article of manufacture may comprise (a) a first container with a composition contained therein, wherein the composition comprises a complex as reported herein; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The following examples, figures and sequences are provided to aid the understanding of the present invention, the true scope of which is set forth in the appended claims.

**Sequences**

| | |
|---|---|
| SEQ ID NO: 01 | VH (mAb 1.4.168) |
| SEQ ID NO: 02 | VL (mAb 1.4.168) |
| SEQ ID NO: 03 | VH (mAb 8.1.2) |
| SEQ ID NO: 04 | VL (mAb 8.1.2) |
| SEQ ID NO: 05 | 17mer ss-DNA (covalently bound with 5' end to FAB' of anti-TroponinT MAB a and FAB' 1.4.168 to IGF-1R, respectively) |
| SEQ ID NO: 06 | 19mer ss-DNA (covalently bound with 3' end to FAB' of anti-TroponinT MAB b and FAB' 8.1.2 to phosphorylated IGF-1R, respectively) |
| SEQ ID NO: 07 | complementary 19mer ss-DNA (used as part of a linker) |
| SEQ ID NO: 08 | complementary 17mer ss-DNA (used as part of a linker) |
| SEQ ID NO: 09 | Epitope "A" for anti-Troponin antibody a. |
| SEQ ID NO: 10 | Epitope "B" for anti-Troponin antibody b. |
| SEQ ID NO: 11 | IGF-1R (1340-1366) |
| SEQ ID NO: 12 | hInsR (1355-1382) |
| SEQ ID NO: 13 | 35-mer L-DNA polynucleotide linker |
| SEQ ID NO: 14 | 75-mer L-DNA polynucleotide linker |
| SEQ ID NO: 15 | 95-mer L-DNA polynucleotide linker |
| SEQ ID NO: 16 | 4D5 FAB' heavy chain amino acid sequence |
| SEQ ID NO: 17 | 4D5 FAB' light chain amino acid sequence |
| SEQ ID NO: 18 | 2C4 FAB' heavy chain amino acid sequence |
| SEQ ID NO: 19 | 2C4 FAB' light chain amino acid sequence |
| SEQ ID NO: 20 | Residues 22-645 within the extracellular domain (ECD) of ErbB2 |
| SEQ ID NO: 21 | 5'-AGT CTA TTA ATG CTT CTG C-XXX-Y-Z-3', wherein X = propylene-phosphate introduced via phosphoramidite C3 (3-(4,4'-dimethoxytrityloxy)propyl-1-[(2-cyanoethyl)-(N,N-diisopropyl)]-phosphoramidite (Glen Research), wherein Y = 5'-amino-modifier C6 introduced via (6-(4-monomethoxytritylamino)hexyl-(2-cyanoethyl)-(N,N-diisopropyl)-phosphoramidite (Glen Research), and wherein Z = 4[N-maleinimidomethyl] cyclohexane-1-carboxy introduced via sulfosuccinimidyl 4-[N-maleimidomethyl]cyclohexane-1-carboxylate (ThermoFischer). |
| SEQ ID NO: 22 | 5'-Y-Z-XXX-AGT TCT ATC GTC GTC CA-3', wherein X = propylene-phosphate introduced via Phosphoramidite C3 (3-(4,4'-Dimethoxytrityloxy)propyl-1-[(2-cyanoethyl)-(N,N-diisopropyl)]-phosphoramidite (Glen Research), wherein Y = 5'-Amino-Modifier C6 introduced via (6-(4-Monomethoxytritylamino)hexyl-(2-cyanoethyl)-(N,N-diisopropyl)-phosphoramidite (Glen Research), and wherein Z = 4[N-maleinimidomethyl] cyclohexane-1-carboxy introduced via Sulfosuccinimidyl 4-[N-maleimidomethyl]cyclohexane-1-carboxylate (ThermoFischer). |
| SEQ ID NO: 23 | |
| SEQ ID NO: 24 | |
| SEQ ID NO: 25 | |
| | |
| SEQ ID NO: 26 | anti-HER2 antibody 4D5 heavy chain variable domain |
| SEQ ID NO: 27 | VH CDR1 |
| SEQ ID NO: 28 | VH CDR2 |
| SEQ ID NO: 29 | VH CDR3 |
| SEQ ID NO: 30 | anti-HER2 antibody 4D5 heavy light variable domain |
| SEQ ID NO: 31 | VL CDR1 |
| SEQ ID NO: 32 | VL CDR2 |
| SEQ ID NO: 33 | VL CDR3 |
| SEQ ID NO: 34 | anti-HER2 antibody 2C4 heavy chain variable domain |
| SEQ ID NO: 35 | VH CDR1 |
| SEQ ID NO: 36 | VH CDR2 |
| SEQ ID NO: 37 | VH CDR3 |
| SEQ ID NO: 38 | anti-HER2 antibody 2C4 light chain variable domain |
| SEQ ID NO: 39 | VL CDR1 |
| SEQ ID NO: 40 | VL CDR2 |
| SEQ ID NO: 41 | VL CDR3 |
| SEQ ID NO: 42 | 5'-X-AGT CTA TTA ATG CTT CTG C-ZZZ-Y-; X= Fluorescein Y=C7Aminolinker Z=C3 spacer |
| SEQ ID NO: 43 | 5'-X AGT CTA TTA ATG CTT CTG C-ZZZ-Y-; X=Cy5 Y=C7Aminolinker Z=C3 spacer |
| SEQ ID NO: 44 | 5'-X-ZZZ-AGT TCT ATC GTC GTC CA-Y-3'; X=aminolinker Y= Fluorescein Z=C3 spacer |
| SEQ ID NO: 45 | 5'-X-(AGT CTA TTA ATG CTT CTG C)-(ZZZ)-y-; X=Fluorescein Y= C7Aminolinker Z=C3 spacer |
| SEQ ID NO: 46 | 5'-X-(ZZZ-(AGT TCT ATC GTC GTC CA)-Y-3'; X= aminolinker Y= Fluorescein Z=C3 spacer |
| SEQ ID NO: 47 | |
| SEQ ID NO: 48 | |
| SEQ ID NO: 49 | |
| SEQ ID NO: 50 | |
| SEQ ID NO: 51 | |
| SEQ ID NO: 52 | |
| SEQ ID NO: 53 | |
| SEQ ID NO: 54 | |
| SEQ ID NO: 55 | |
| SEQ ID NO: 56 | |
| SEQ ID NO: 57 | |
| SEQ ID NO: 58 | |
| SEQ ID NO: 59 | |
| SEQ ID NO: 60 | |
| SEQ ID NO: 61 | |
| SEQ ID NO: 62 | |
| SEQ ID NO: 63 | |
| SEQ ID NO: 64 | YPYDVPDYA |
| SEQ ID NO: 65 | GLNDIFEAQKIEWHE |
| SEQ ID NO: 66 | SGGGS |
| SEQ ID NO: 67 | f-Met-Leu-Phe (fMLP) |
| SEQ ID NO: 68 | f-Met-Leu-Phe-o-methyl ester |
| SEQ ID NO: 69 | IgG1 constant domain |
| SEQ ID NO: 70 | IgG2 constant domain |
| SEQ ID NO: 71 | IgG4 constant domain |
| SEQ ID NO: 72 | Cy5-Y- ATG CGA-GTA CCT TAG AGT C -Z-Cy5 |
| SEQ ID NO: 73 | |
| SEQ ID NO: 74 | Sortase tag |
| SEQ ID NO: 75 | Binding pair member oligonucleotide. |
| SEQ ID NO: 76 | L-DNA linker. |

### Figures

- **Figure 1**: Scheme of the BIAcore assay setup. ss-L-DNA-bi linkers were presented on a BIAcore SA sensor. Flow cell 1 served as a control (not shown).
- **Figure 2**: BIAcore sensorgrams for the HER2-ECD interaction with ss-D-DNA labeled FAB fragments.
- **Figure 3**: BIAcore sensorgrams showing concentration dependent interaction measurements of the complex as reported herein comprising a 35-mer (= 35 nucleotide length) as linker polynucleotide with HER2-ECD.
- **Figure 4**: BIAcore sensorgrams showing concentration dependent interaction measurements of the complex as reported herein comprising a 75-mer (= 75 nucleotide length) as linker polynucleotide with HER2-ECD.
- **Figure 5**: BIAcore sensorgrams showing concentration dependent interaction measurements of the complex as reported herein comprising a 95-mer (= 95 nucleotide length) as linker polynucleotide with HER2-ECD.

- **Figure 6**: Scheme of the BIAcore assay setup: polyclonal goat anti human IgG-Fc gamma antibody was presented on a BIAcore SA sensor. Flow cell 1 served as a control (not shown).
- **Figure 7**: The BIAcore sensorgram shows an overlay plot of interaction signals upon 50 nM injections of anti-HER2 antibody 2C4-FAB'-ss-L-DNA (2C4), anti-HER2 antibody 4D5-FAB'-ss-L-DNA (4D5) and fully established complex (2C4-75mer-4D5) connected by a 75mer ss-L-DNA linker.
- **Figure 8**: BIAcore sensorgram showing an overlay plot of concentration-dependent measurements of the fully established 75-mer complex as analyte in solution interacting with the surface presented huFc chimera HER2 ECD
- **Figure 9**: Plot of the response levels of Figure 8 versus the analyte concentration of the fully established complex.

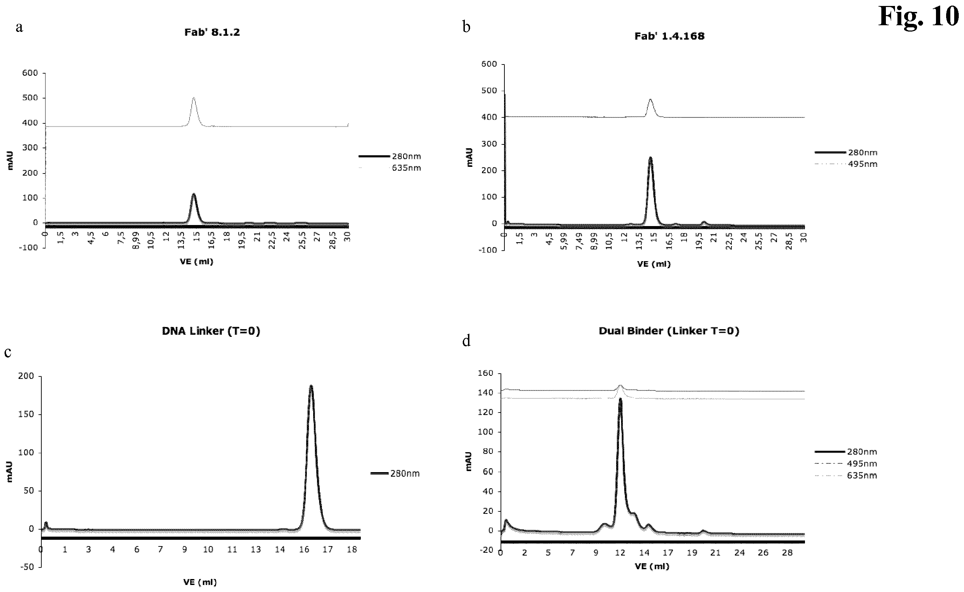

- **Figure 10**: Analytical gel filtration experiments assessing efficiency of the anti-pIGF1-R complex assembly. Diagrams a, b and c show the elution profile of the individual complex components (fluorescein-ss-FAB' 1.4.168, Cy5-ssFab' 8.1.2 and Linker DNA (T=0)). Diagram d shows the elution profile after the 3 components needed to form the bivalent binding agent had been mixed in a 1:1:1 molar ratio. The thicker (bottom) curve represents absorbance measured at 280nm indicating the presence of the ss-FAB' proteins or the linker DNA, respectively. The thinner top curve in b) and d) (absorbance at 495 nm) indicates the presence of Cy5 and the thinner top curve in a) and the middle curve in d) (absorbance at 635 nm) indicates the presence of fluorescein. Comparison of the elution volumes of the single complex components (VE_{ssFab' 1.4.168} ~15 ml; VE_{ssFab' 8}.₁.₂ ~15 ml; VEₗᵢₙₖₑᵣ ~ 16 ml) with the elution volume of the reaction mix (VEₘᵢₓ ~ 12 ml) demonstrates that the complex assembly reaction was successful (rate of yield: ~ 90%). The major 280nm peak that represents the eluted complex nicely overlaps with the major peaks in the 495nm and 695nm channel, proving the presence of both ss-FAB' 8.1.2 and ssFab'1.4.168 in the peak representing the bivalent binding agent.
- **Figure 11**: Scheme of the BIAcore experiment: schematically and exemplarily, two binding molecules in solution are shown. The T=0-Dig, bivalent binding agent and the T=40-Dig, bivalent binding agent. Both these bivalent binding agents only differ in their linker-length (no additional T versus 40 additional Ts, separating the two hybridizing nucleic acid sequences). Furthermore, ss-FAB' fragments 8.1.2 and 1.4.168 were used.
- **Figure 12**: BIAcore sensorgram with overlay plot of three kinetics showing the interaction of 100 nM bivalent binding agent (consisting of ss-FAB 8.1.2 and ss-FAB 1.4.168 hybridized on the T40-Dig ss-DNA-Linker) with the immobilized peptide pIGF-1R compared to the binding characteristics of 100 nM ss-FAB 1.4.168 or 100 nM ss-FAB 8.1.2 to the same peptide. Highest binding performance is only obtained with the complex construct, clearly showing, that the cooperative binding effect of the Complex increases affinity versus the target peptide pIGF-1R.
- **Figure 13**: BIAcore sensorgram with overlay plot of three kinetics showing the interactions of the bivalent binding agent consisting of ss-FAB 8.1.2 and ss-FAB 1.4.168 hybridized on the T40-Dig ss-DNA-Linker with immobilized peptides pIGF-1R (phosphorylated IGF-1R), IGF-1R or IR (phosphorylated insulin receptor). Highest binding performance is obtained with the pIGF-1R peptide, clearly showing, that the cooperative binding effect of the Complex increases specificity versus the target peptide pIGF-1R as compared to e.g. the phosphorylated insulin receptor peptide (IR).
- **Figure 14**: BIAcore sensorgram with overlay plot of two kinetics showing the interactions of 100 nM bivalent binding agent consisting of ss-FAB' 8.1.2 and ss-FAB' 1.4.168 hybridized on the T=40-Dig ss-DNA-Linker and a mixture of 100 nM ss-FAB' 8.1.2 and 100 nM ss-FAB' 1.4.168 without linker DNA. Best binding performance is only obtained with the bivalent binding agent, whereas the mixture of the ss-FAB's without linker doesn't show an observable cooperative binding effect, despite the fact that the total concentration of these ss-FAB's had been at 200 nM.
- **Figure 15**: Schematic drawing of a BIAcore sandwich assay. This assay has been used to investigate the epitope accessibility for both antibodies on the phosphorylated IGF-1R peptide. <MIgGFcy>R presents a rabbit anti-mouse antibody used to capture the murine antibody M-1.4.168. M-1.4.168 then is used to capture the pIGF-1R peptide. M-8.1.2 finally forms the sandwich consisting of M-1.4.168, the peptide and M-8.1.2.

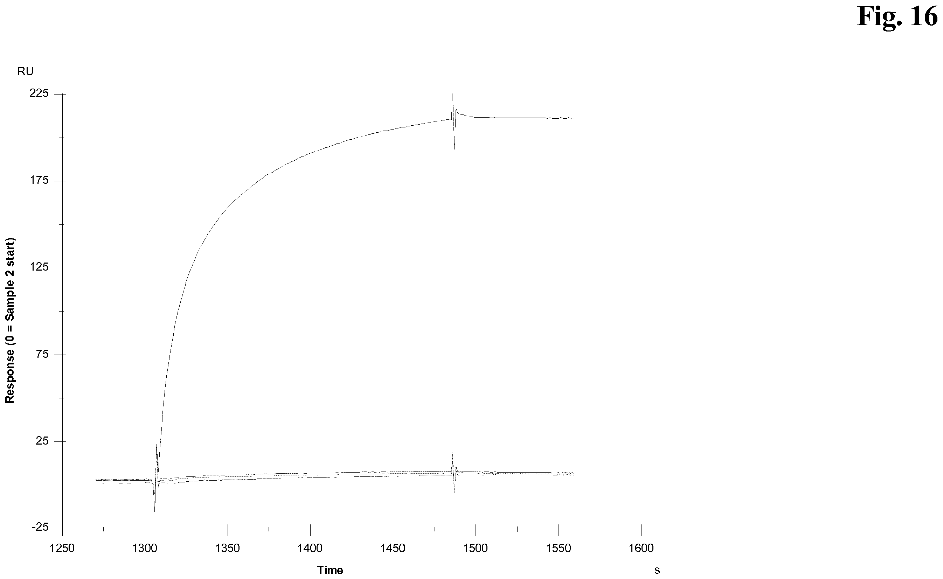

- **Figure 16**: BIAcore sensorgram showing the binding signal (thick line) of the secondary antibody 8.1.2. to the pIGF-1R peptide after this was captured by antibody 1.4.168 on the BIAcore chip. The other signals (thin lines) are control signals: given are the lines for a homologous control with 500 nM 1.4.168, 500 nM target unrelated antibody <CKMM>M- 33-IgG; and 500 nM target unrelated control antibody <TSH>M-1.20-IgG, respectively. No binding event could be detected in any of these controls.

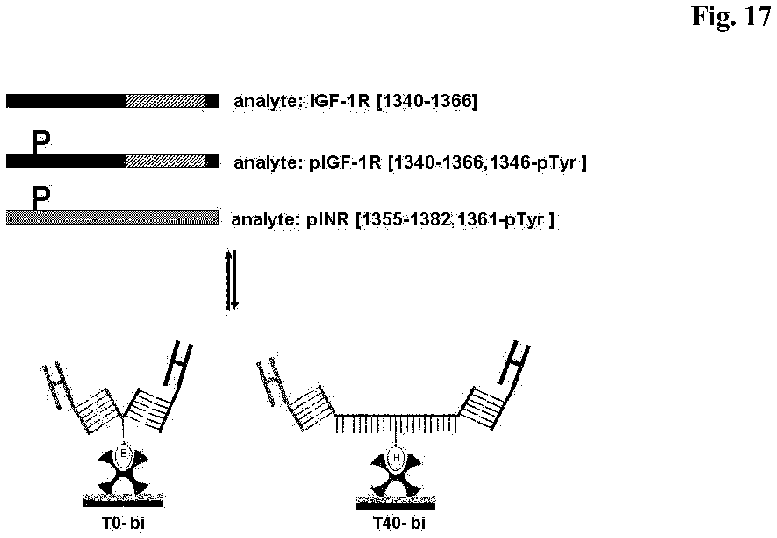

- **Figure 17**: Schematic drawing of the BIAcore assay, presenting the complex s on the sensor surface. On Flow Cell 1 (=FC1) (not shown) amino-PEO-biotin was captured. On FC2, FC3 and FC4 bivalent binding agents with increasing linker length were immobilized. Analyte 1: IGF-1R-peptide containing the M-1.4.168 ss-FAB epitope (thin line) - the M-8.1.2 ss-FAB phospho-epitope is not present, because this peptide is not phosphorylated; analyte 2: pIGF-1R peptide containing the M-8.1.2 ss-FAB phospho-epitope (P) and the M-1.4.168 ss-FAB epitope (thin line). Analyte 3: pINR peptide, containing the cross reacting M-8.1.2 ss-FAB phospho-epitope, but not the epitope for M-1.4.168.
- **Figure 18**: Kinetic data of the Complex experiment. T40-bi complex with ss-FAB 8.1.2 and ss-FAB 1.4.168 shows a 1300-fold lower off-rate (KD = 2.79E-05/s) versus pIGF-1R when compared to pINR (KD = 3.70E-02).
- **Figure 19**: BIAcore sensorgram, showing concentration dependent measurement of the T40-bi complex vs. the pIGF-1R peptide (the phosphorylated IGF-1R peptide).
- **Figure 20**: BIAcore sensorgram, showing concentration dependent measurement of the T40-bi complex vs. the IGF-1R peptide (the non-phosphorylated IGF-1R peptide).
- **Figure 21**: BIAcore sensorgram, showing concentration dependent measurement of the T40-bi complex vs. the pINR peptide (the phosphorylated insulin receptor peptide).
- **Figure 22**: Staining of tumor cells with Cy5 labeled Xolair® and Herceptin®.
- **Figure 23**: NIRF imaging of KPL-4 cells.
- **Figure 24**: Ex vivo staining of KPL-4 xenografts.
- **Figure 25**: Size exclusion profile of freshly prepared 4D5-95mer-2C4 complex. Upper signal: 260 nm signal, lower signal: 280 nm signal. No aggregates can be detected between start at 0.0 min and the elution peak at 5.64 min.
- **Figure 26**: Size exclusion profile of the 4D5-95mer-2C4 complex after a freezing and thawing cycle. Upper signal: 260 nm signal, lower signal: 280 nm signal. No aggregates can be detected between start at 0.0 min and the elution peak at 5.71 min.
- **Figure 27**: Typical chromatogram (analytical SEC) of a bispecific binding molecule.
- **Figure 28**: Electropherograms of a LabChip system (Perkin Elmer) of the monitoring of an antibody Fab fragment-oligonucleotide conjugate in different stages of the workflow.

### Example 1

### Formation of FAB-ss-DNA-conjugates

Two monoclonal antibodies binding to human cardiac Troponin T at different, non-overlapping epitopes, epitope a and epitope b, respectively, were used. Both these antibodies are used in the current Roche Elecsys™ Troponin T assay, wherein Troponin T is detected in a sandwich immunoassay format.

Purification of the monoclonal antibodies from culture supernatant was carried out using state of the art methods of protein chemistry.

The purified monoclonal antibodies are protease digested with either pre-activated papain (anti-epitope a MAb) or pepsin (anti-epitope b MAb) yielding F(ab')₂ fragments that are subsequently reduced to FAB'-fragments with a low concentration of cysteamine at 37 °C. The reaction is stopped by separating the cysteamine on a Sephadex G-25 column (GE Healthcare) from the polypeptide-containing part of the sample.

The FAB'-fragments are conjugated with the below described activated ss-DNAa and ss-DNAb oligonucleotides.

### a) anti-Troponin T (epitope A) antibody FAB-ss-DNA-conjugate A

For preparation of the anti-Troponin T <epitope a> antibody FAB-ss-DNAa-conjugate A a derivative of SEQ ID NO: 05 is used, i.e. 5'-AGT CTA TTA ATG CTT CTG C(=SEQ ID NO:5)-XXX-Y-Z-3', wherein X = propylene-phosphate introduced via Phosphoramidite C3 (3-(4,4'-Dimethoxytrityloxy)propyl-1-[(2-cyanoethyl)-(N,N-diisopropyl)]-phosphoramidite (Glen Research), wherein Y = 3 "-Amino-Modifier C6 introduced via 3'-Amino Modifier TFA Amino C-6 lcaa CPG (ChemGenes) and wherein Z = 4[N-maleinimidomethyl]cyclohexane-1-carboxy introduced via Sulfosuccinimidyl 4-[N-maleimidomethyl]cyclohexane-1-carboxylate (ThermoFischer).

### b) Anti-Troponin T (epitope B) antibody FAB-ss-DNA-conjugate B

For the preparation of the anti-Troponin T <epitope b> antibody FAB-ss-DNAb-conjugate B a derivative of SEQ ID NO: 06 is used, i.e. 5'-Y-Z-XXX-AGT TCT ATC GTC GTC CA-3', wherein X = propylene-phosphate introduced via Phosphoramidite C3 (3-(4,4'-Dimethoxytrityloxy)propyl-1-[(2-cyanoethyl)-(N,N-diisopropyl)]-phosphoramidite (Glen Research), wherein Y = 5'-Amino-Modifier C6 introduced via (6-(4-Monomethoxytritylamino)hexyl-(2-cyanoethyl)-(N,N-diisopropyl)-phosphoramidite (Glen Research), and wherein Z = 4[N-maleinimidomethyl]cyclohexane-1-carboxy introduced via Sulfosuccinimidyl 4-[N-maleimidomethyl]cyclohexane-1-carboxylate (ThermoFischer).

The oligonucleotides of SEQ ID NO: 05 or 06, respectively, have been synthesized by state of the art oligonucleotide synthesis methods. The introduction of the maleinimido group was done via reaction of the amino group of Y with the succinimidyl group of Z which was incorporated during the solid phase oligonucleotide synthesis process.

The single-stranded DNA constructs shown above bear a thiol-reactive maleimido group that reacts with a cysteine of the FAB' hinge region generated by the cysteamine treatment. In order to obtain a high percentage of single-labeled FAB'-fragments the relative molar ratio of ss-DNA to FAB'-fragment is kept low. Purification of single-labeled FAB'-fragments (ss-DNA:FAB' = 1:1) occurs via anion exchange chromatography (column: MonoQ, GE Healthcare). Verification of efficient labeling and purification is achieved by analytical gel filtration chromatography and SDS-PAGE.

### Example 2

### Formation of biotinylated linker molecules

The oligonucleotides used in the ss-DNA linkers L1, L2 and L3, respectively, have been synthesized by state of the art oligonucleotide synthesis methods and employing a biotinylated phosphoramidite reagent for biotinylation.

Linker 1 (=L1), a biotinylated ss-DNA linker 1 with no spacer has the following composition:
5'-GCA GAA GCA TTA ATA GAC T (Biotin-dT)-GG ACG ACG ATA GAA CT-3'. It comprises ss-DNA oligonucleotides of SEQ ID NO: 7 and 8, respectively, and was biotinylated by using Biotin-dT (5'-Dimethoxytrityloxy-5-[N-((4-t-butylbenzoyl)-biotinyl)-aminohexyl)-3-acrylimido]-2'-deoxyUridine-3'-[(2-cyanoethyl)-(N,N-diisopropyl)]-phosphoramidite (Glen Research).

Linker 2 (=L2), a biotinylated ss-DNA linker 2 with a 10mer spacer has the following composition:
5'-GCA GAA GCA TTA ATA GAC T T5-(Biotin-dT)-T5 GG ACG ACG ATA GAA CT-3'. It comprises ss-DNA oligonucleotides of SEQ ID NO: 7 and 8, respectively, twice oligonucleotide stretches of five thymidines each and was biotinylated by using Biotin-dT (5'-Dimethoxytrityloxy-5-[N-((4-t-butylbenzoyl)-biotinyl)-aminohexyl)-3-acrylimido]-2'-deoxyUridine-3'-[(2-cyanoethyl)-(N,N-diisopropyl)]-phosphoramidite (Glen Research).

Linker 3 (=L3), a biotinylated ss-DNA linker 3 with a 30mer spacer has the following composition:
5'-GCA GAA GCA TTA ATA GAC T T15-(Biotin-dT)-T15 GG ACG ACG ATA GAA CT-3'. It comprises ss-DNA oligonucleotides of SEQ ID NO: 7 and 8, respectively, twice oligonucleotide stretches of fifteen thymidines each and was biotinylated by using Biotin-dT (5'-Dimethoxytrityloxy-5-[N-((4-t-butylbenzoyl)-biotinyl)-aminohexyl)-3-acrylimido]-2'-deoxyUridine-3'-[(2-cyanoethyl)-(N,N-diisopropyl)]-phosphoramidite (Glen Research).

### Example 3

### Epitopes for monovalent Troponin T binders a and b, respectively

Synthetic peptides have been construed that individually only have a moderate affinity to the corresponding FAB'-fragment derived from the anti-Troponin T antibodies a and b, respectively.

### a) The epitope "A" for antibody a is comprised in:

SEQ ID NO: 9 = ERAEQQRIRAEREKEUUSLKDRIEKRRRAERAEamide, wherein U represents ß-Alanine.

### b) The epitope "B" for antibody b is comprised in:

SEQ ID NO: 10 = SLKDRIERRRAERAEOOERAEQQRIRAEREKEamide, wherein O represents Amino-trioxa-octanoic-acid

As the skilled artisan will appreciate it is possible to combine these two epitope-containing peptides two ways and both variants have been designed and prepared by linear combining the epitopes "A" and "B". The sequences of both variants, the linear sequences of epitopes "A"-"B" (= TnT 1) and "B"-"A" (=TnT 2), respectively have been prepared by state of the art peptide synthesis methods.

The sequences for epitopes "A" and "B", respectively, had been modified compared to the original epitopes on the human cardiac Troponin T sequence (P45379/UniProtKB) in order to reduce the binding affinity for each of the FABs thereto. Under these circumstances the dynamics of the effect of hetero-bivalent binding is better visible, e.g. by analyzing binding affinity with the BIAcore Technology.

### Example 4

### Biomolecular Interaction Analysis

For this experiment a BIAcore 3000 instrument (GE Healthcare) was used with a BIAcore SA sensor mounted into the system at T = 25° C. Preconditioning was done at 100 µl/min with 3x1 min injection of 1 M NaCl in 50 mM NaOH and 1 min 10 mM HCl.

HBS-ET (10 mM HEPES pH 7.4, 150 mM NaCl, 1mM EDTA, 0.05% Tween® 20 was used as system buffer. The sample buffer was identical to the system buffer.

The BIAcore 3000 System was driven under the control software V1.1.1. Flow cell 1 was saturated with 7 RU D-biotin. On flow cell 2, 1063 RU biotinylated ss-DNA linker L1 was immobilized. On flow cell 3, 879 RU biotinylated ss-DNA linker L2 was immobilized. On flow cell 4, 674 RU biotinylated ss-DNA linker L3 was captured.

Thereafter, FAB fragment DNA conjugate A was injected at 600 nM. FAB fragment DNA conjugate B was injected into the system at 900 nM. The conjugates were injected for 3 min at a flow rate of 2 µl/min. The conjugates were consecutively injected to monitor the respective saturation signal of each FAB fragment DNA conjugate on its respective linker. FAB combinations were driven with a single FAB fragment DNA conjugate A, a single FAB fragment DNA conjugate B and both FAB fragment DNA conjugates A and B present on the respective linker. Stable baselines were generated after the linkers have been saturated by the FAB fragment DNA conjugates, which was a prerequisite for further kinetic measurements.

The peptidic analytes TnT1 and TnT2 were injected as analytes in solution into the system in order to interact with the surface presented FAB fragments.

TnT1 was injected at 500 nM, TnT2 was injected at 900 nM analyte concentration. Both peptides were injected at 50 µl/min for 4 min association time. The dissociation was monitored for 5 min. Regeneration was done by a 1 min injection at 50 µl/min of 50 mM NaOH over all flow cells.

Kinetic data was determined using the BIAevaluation software (V.4.1). The dissociation rate KD (1/s) of the TnT1 and TnT2 peptides from the respective surface presented FAB fragment combinations was determined according to a linear Langmuir 1:1 fitting model. The complex halftime in min were calculated according to the solution of the first order kinetic equation: ln(2)/ (60*kD).

### Results:

The experimental data given in Tables 5 and 6, respectively demonstrate an increase in complex stability between analyte (TnT1 or TnT2), respectively, and the various heterobivalent FAB-FAB conjugates A-B as compared to the monovalent dsDNA FAB A or B conjugate, respectively. This effect is seen in each Table in line 1 compared to lines 2 and 3.

**Table 5: Analysis data using TnT1 with linkers of various length**

| **a) Linker L1** | | | |
|---|---|---|---|
| **FAB fragment DNA conjugate A** | **FAB fragment DNA conjugate B** | **kD (1/s)** | **t1/2 diss (min)** |
| x | x | 6.6E-03 | 1.7 |
| x | - | 3.2E-02 | 0.4 |
| - | x | 1.2E-01 | 0.1 |

| **b) Linker L2** | | | |
|---|---|---|---|
| **FAB fragment DNA conjugate A** | **FAB fragment DNA conjugate B** | **kD (1/s)** | **t1/2 diss (min)** |
| x | x | 4.85E-03 | 2.4 |
| x | - | 2.8E-02 | 0.4 |
| - | x | 1.3E-01 | 0.1 |

| **c) Linker L3** | | | |
|---|---|---|---|
| **FAB fragment DNA conjugate A** | **FAB fragment DNA conjugate B** | **kD (/1/s)** | **t1/2 diss (min)** |
| x | x | 2.0E-03 | 5.7 |
| x | - | 1.57E-02 | 0.7 |
| - | x | 1.56E-02 | 0.7 |

**Table 6: Analysis data using TnT2 with linkers of various length**

| **a) Linker L1** | | | |
|---|---|---|---|
| **FAB fragment DNA conjugate A** | **FAB fragment DNA conjugate B** | **kD (/1/s)** | **t1/2 diss (min)** |
| x | x | 1.4E-02 | 0.8 |
| x | - | 4.3E-02 | 0.3 |
| - | x | 1.4E-01 | 0.1 |

| **b) Linker L2** | | | |
|---|---|---|---|
| **FAB fragment DNA conjugate A** | **FAB fragment DNA conjugate B** | **kD (/1/s)** | **t1/2 diss (min)** |
| x | x | 4.9E-03 | 2.3 |
| x | - | 3.5E-02 | 0.3 |
| - | x | 1.3E-01 | 0.1 |

| **c) Linker L3** | | | |
|---|---|---|---|
| **FAB fragment DNA conjugate A** | **FAB fragment DNA conjugate B** | **kD (/1/s)** | **t1/2 diss (min)** |
| x | x | 8.0E-03 | 1.5 |
| x | - | 4.9E-02 | 0.2 |
| - | x | 3.2E-01 | 0.04 |

The avidity effect is further dependent on the length of the linker. In the sub-tables shown under Table 1 the 30mer linker L3 shows the lowest dissociation rate or highest complex stability, in sub-tables shown under Table 2 the 10mer L2 linker exhibits the lowest dissociation rate or highest complex stability. These data taken together demonstrate that the flexibility in linker length as inherent to the approach given in the present invention is of great utility and advantage.

### Example 5

### Formation of FAB'-ss-DNA-conjugates

Two monoclonal antibodies binding to human HER2 (ErbB2 or p185*^{neu}*) at different, non-overlapping epitopes A and B were used. The first antibody is anti-HER2 antibody 4D5 (huMAb4D5-8, rhuMab HER2, trastuzumab or HERCEPTIN^{®}; see US 5,821,337).

The second antibody is anti-HER2 antibody 2C4 (Pertuzumab).

Purification of the monoclonal antibodies from culture supernatant can be carried out using state of the art methods of protein chemistry.

The purified monoclonal antibodies are protease digested with either pre-activated papain or pepsin yielding F(ab')₂ fragments. These are subsequently reduced to FAB'-fragments with a low concentration of cysteamine at 37 °C. The reaction is stopped by separating the cysteamine on a Sephadex G-25 column (GE Healthcare) from the polypeptide-containing part of the sample.

The obtained FAB'-fragments are conjugated with the activated ss-DNA polynucleotides.

### a) anti-HER2 antibody 4D5 FAB'-ss-DNA-conjugate

For preparation of the anti-HER2 antibody 4D5 FAB'-ss-DNA-conjugate a derivative of SED ID NO: 05 is used, i.e. 5'-AGT CTA TTA ATG CTT CTG C(= SEQ ID NO: 05)-XXX-Y-Z-3', wherein X = propylene-phosphate introduced via phosphoramidite C3 (3-(4,4'-dimethoxytrityloxy)propyl-1-[(2-cyanoethyl)-(N,N-diisopropyl)]-phosphoramidite (Glen Research), wherein Y = 5'-amino-modifier C6 introduced via (6-(4-monomethoxytritylamino)hexyl-(2-cyanoethyl)-(N,N-diisopropyl)-phosphoramidite (Glen Research), and wherein Z = 4[N-maleinimidomethyl]cyclohexane-1-carboxy introduced via Sulfosuccinimidyl 4-[N-maleimidomethyl]cyclohexane-1-carboxylate (ThermoFischer).

### b) anti-HER2 antibody 2C4 FAB'-ss-DNA-conjugate

For the preparation of the anti-HER2 antibody 2C4 FAB'-ss-DNA-conjugate B a derivative of SEQ ID NO: 06 is used, i.e. 5'-Y-Z-XXX-AGT TCT ATC GTC GTC CA-3', wherein X = propylene-phosphate introduced via Phosphoramidite C3 (3-(4,4'-Dimethoxytrityloxy)propyl-1-[(2-cyanoethyl)-(N,N-diisopropyl)]-phosphoramidite (Glen Research), wherein Y = 5'-Amino-Modifier C6 introduced via (6-(4-Monomethoxytritylamino)hexyl-(2-cyanoethyl)-(N,N-diisopropyl)-phosphoramidite (Glen Research), and wherein Z = 4[N-maleinimidomethyl]cyclohexane-1-carboxy introduced via Sulfosuccinimidyl 4-[N-maleimidomethyl]cyclohexane-1-carboxylate (ThermoFischer).

The polynucleotides of SEQ ID NO: 05 or SEQ ID NO: 06, respectively, have been synthesized by state of the art polynucleotide synthesis methods. The introduction of the maleinimido group was done via reaction of the amino group of Y which was incorporated during the solid phase polynucleotide synthesis process with the Sulfosuccinimidyl 4-[N-maleimidomethyl] cyclohexane-1-carboxylate (ThermoFischer).

The single-stranded DNA constructs bear a thiol-reactive maleimido group that reacts with a cysteine of the FAB' hinge region generated by the cysteamine treatment. In order to obtain a high percentage of single-labeled FAB'-fragments the relative molar ratio of ss-DNA to FAB'-fragment is kept low. Purification of single-labeled FAB'-fragments (ss-DNA:FAB' = 1:1) occurs via anion exchange chromatography (column: MonoQ, GE Healthcare). Verification of efficient labeling and purification is achieved by analytical gel filtration chromatography and SDS-PAGE.

### Example 6

### Biomolecular Interaction Analysis

For this experiment a BIAcore T100 instrument (GE Healthcare) was used with a BIAcore SA sensor mounted into the system at T = 25 °C. Preconditioning occurred at 100 µl/min with 3x1 min injection of 1 M NaCl in 50 mM NaOH, pH 8.0 followed by a 1 min injection of 10 mM HCl. The system buffer was HBS-EP (10 mM HEPES pH 7.4, 150 mM NaCl, 1mM EDTA, 0.05 % P 20). The sample buffer was the system buffer supplemented with 1 mg/ml CMD (carboxymethyldextrane).

Biotinylated ss-L-DNA linkers were captured on the SA surface in the respective flow cells. Flow cell 1 was saturated with amino-PEO-Biotin (PIERCE).

40 RU of the biotinylated 35mer oligonucleotide linker were captured on flow cell 2. 55 RU of the biotinylated 75mer oligonucleotide linker were captured on flow cell 3. 60 RU of biotinylated 95mer oligonucleotide linker were captured on flow cell 4.

250 nM anti-HER2 antibody 4D5-FAB'-ss-L-DNA was injected into the system for 3 min. 300 nM anti-HER2 antibody 2C4-FAB'-ss-L-DNA was injected into the system at 2 µl/min for 5 min. The DNA-labeled FAB fragments were injected alone or in combination.

As a control only 250 nM anti-HER2 antibody 4D5-FAB'-ss-D-DNA and 300 nM anti-HER2 antibody 2C4-FAB'-ss-D-DNA was injected into the system. As a further control, buffer was injected instead of the DNA-labeled FAB fragments. After hybridization of the ss-L-DNA-labeled FAB fragments on the respective ss-L-DNA bi-linkers, the analyte in solution hHER2-ECD was injected at different concentration series from 24 nM, 8 nM, 3 nM, 1 nM, 0.3 nM, 0 nM into the system for 3.5 min association phase at 100 µl/min. The dissociation phase was monitored at 100 µl/min for 15 min. The system was regenerated by a 30 sec injection at 20 µl/min of 100 mM glycine buffer (Glycine pH 11, 150 mM NaCl), followed by a second 1 min injection of water at 30 µl/min.

The signals were measured as analyte concentration-dependent, time resolved sensorgrams. The data was evaluated using the BIAcore BIAevaluation software 4.1. As a fitting model a standard Langmuir binary binding model was used.

### Results:

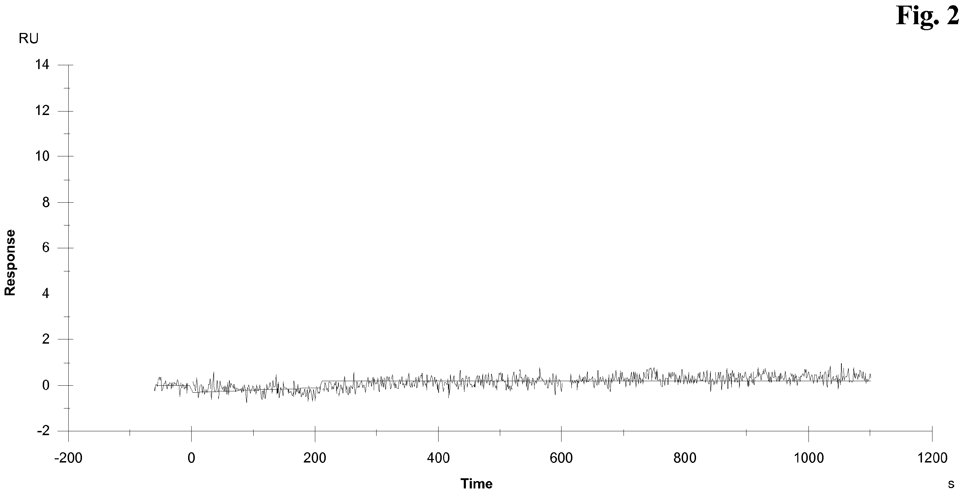

No HER2-ECD interaction could be observed when ss-D-DNA labeled FAB fragments were injected into the system, because the ss-D-DNA-labeled FAB fragments didn't hybridize with spiegelmeric ss-L-DNA linkers presented on the sensor surface (Figure 2).

Table 7: Kinetic results of the complexation experiment. Linker: Surface presented biotinylated ss-L-DNA polynucleotide linker, Oligo_35mer-Bi, Oligo_75mer-Bi and Oligo_95mer-Bi differing in linker length. ss-L-DNA-FAB: 2C4-ss-L-DNA: anti-HER2 antibody 2C4-FAB'-ss-L-DNA labeled with 19mer-Fluorescein. 4D5-ss-L-DNA: anti-HER2 antibody 4D5-FAB'-ss-L-DNA labeled with 17mer-Fluorescein. 4D5-+2C4-ss-L-DNA: surface presented combination of both fragments. LRU: mass in response units, which is hybridized on the sensor surface. Antigen: An 87 kDa HER2-ECD was used as analyte in solution. ka: association rate in (1/Ms). kd: dissociation rate in (1/s). t1/2 diss: antigen complex halftime calculated in hours according to the solution ln(2)/kD*3600 of a first order kinetic equation. kD: affinity in molar. kD: affinity calculated in picomolar. Rₘₐₓ: Maximum analyte response signal at saturation in response units (RU). MR: Molar Ratio, indicating the stoichiometry of the interaction. Chi2, U-value: quality indicator of the measurements.

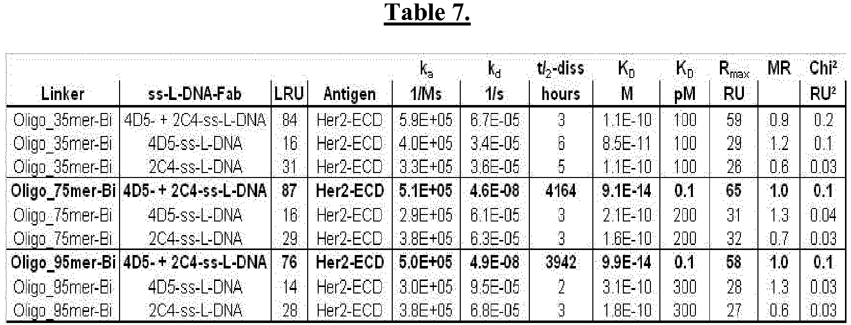

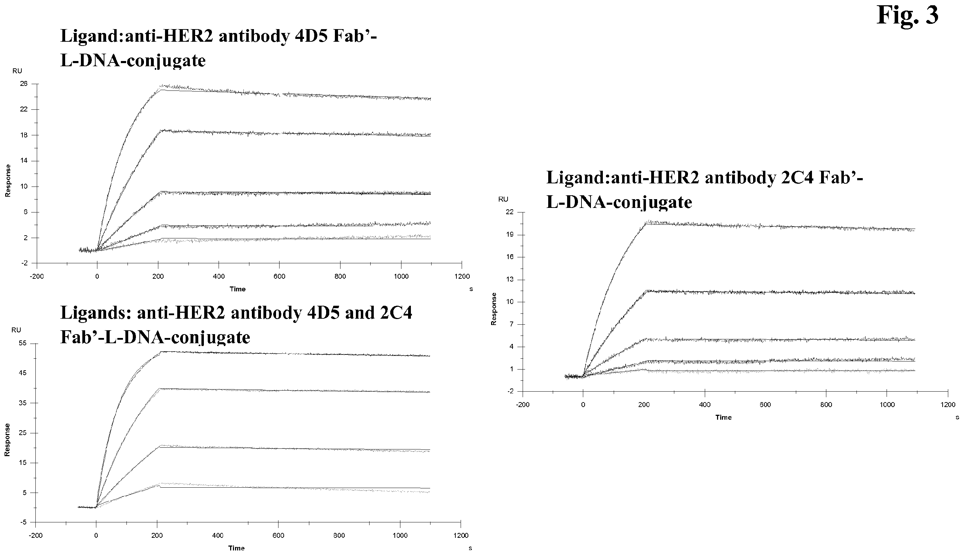

The BIAcore sensorgrams show concentration dependent measurements of the 35-mer complex HER2-ECD interaction (Figure 3). This linker is consisting of solely the hybridization motives sequences of the DNA labels. The kinetic data indicates that the fully established complex shows no improvement of the kinetic performance. This is due to the insufficient linker length and lacking flexibility of the 35-mer.

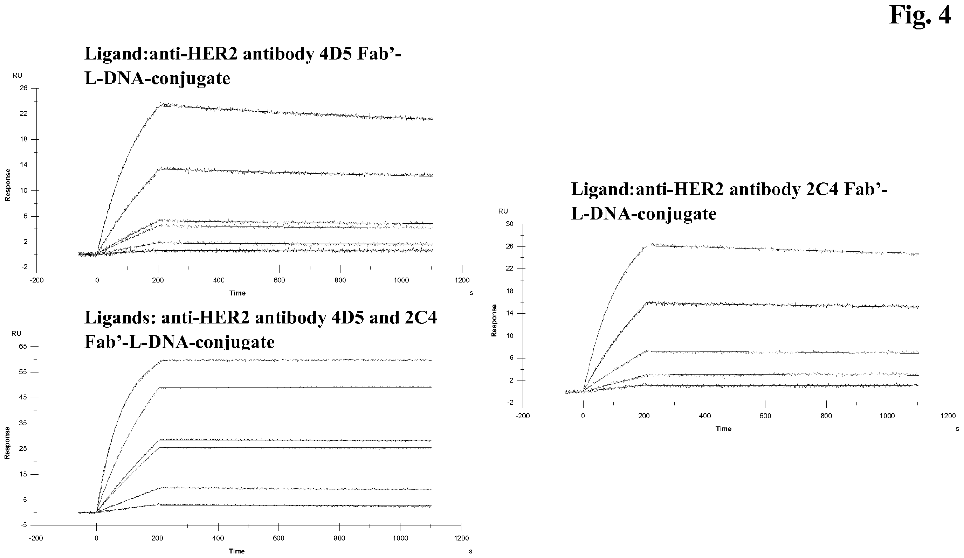

The BIAcore sensorgrams showing concentration dependent measurements of the 75-mer complex HER2-ECD interaction (Figure 4). The 75-mer linker carries poly-T to increase the linker length compared to the 35-mer linker. The kinetic data indicates that the fully established complex shows a dramatic improvement of its kinetic performance. This is due to an optimal linker length and flexibility of the 75-mer.

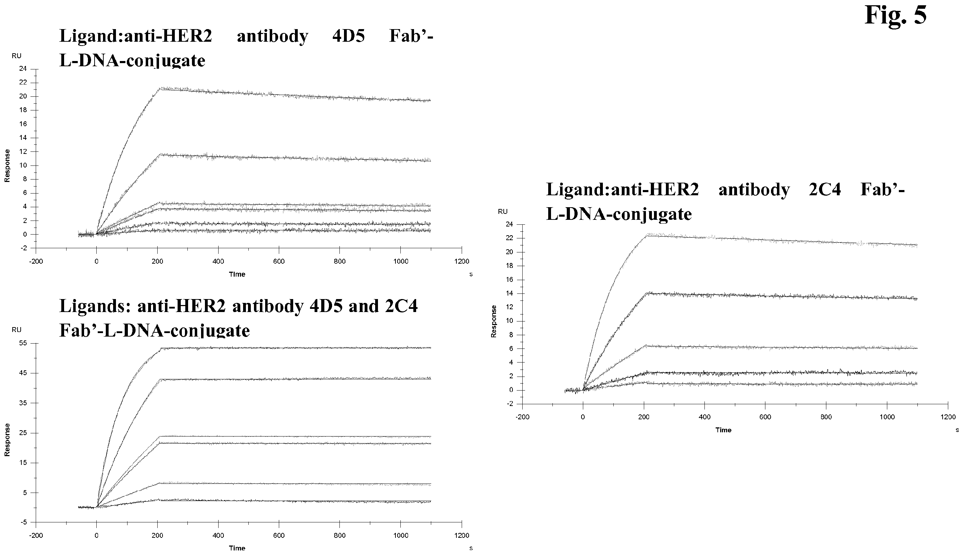

The BIAcore sensorgrams showing concentration dependent measurements of the 95-mer complex HER2-ECD interaction (Figure 5). The 95-mer linker carries poly-T to increase the linker length compared to the 35-mer linker. The kinetic data indicates that the fully established complex shows a dramatic improvement of its kinetic performance. This is due to increased linker length and flexibility of the 95-mer.

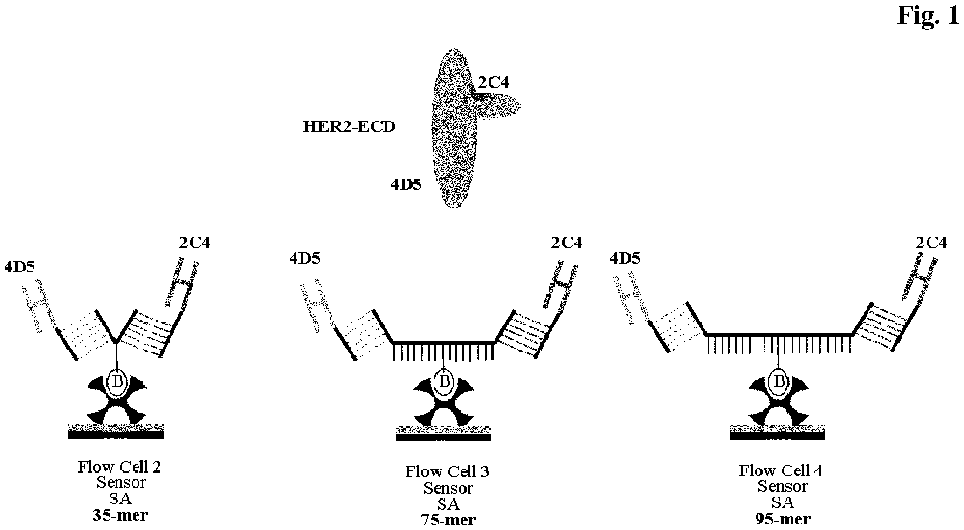

The BIAcore assay setup comprised the following (see also Figure 1): ss-L-DNA-bi linkers were presented on a BIAcore SA sensor. Flow cell 1 served as a control. As analyte in solution Her2-ECD was used. Anti-HER2 antibody 2C4-FAB'-ss-L-DNA and anti-HER2 antibody 2C4-FAB'-ss-L-DNA were hybridized to the surface presented linkers.

Here is shown, for the first time, a fully functional cooperative binding event between Herceptin-FAB and Pertuzumab-FAB linked together via a highly flexible ss-L-DNA linker. The data in Table 3 provides evidence for the presence of a cooperative binding event. Despite the Rmax values of the fully established complex s are roughly double the signal height of the singly FAB-armed constructs, the Molar Ratio values are exactly 1 (MR = 1). This is a clear evidence for the presence of a simultaneous, cooperative binding event of both FAB fragments. The complex counts as a single molecule with a 1:1 Langmuir binding stoichiometry. Despite having 2 independently binding HER2 interfaces no inter molecule binding between one complex and two HER2 domains can be detected.

The avidity constants for synergizing pairs of monoclonal antibodies or for a chemically cross-linked bispecific F(ab')2 is generally only up to 15 times greater than the affinity constants for the individual monoclonal antibodies, which is significantly less than the theoretical avidity expected for ideal combination between the reactants (Cheong, H.S., et al., Biochem. Biophys. Res. Commun. 173 (1990) 795-800). Without being bound by this theory one reason for this might be that the individual epitope/paratope interactions involved in a synergistic binding (resulting in a high avidity) must be orientated in a particular way relative to each other for optimal synergy.

Furthermore, the data presented in Table 7 provides evidence, that the short 35-mer linker, which consists just from the ss-L-DNA hybridization motives doesn't show enough flexibility or/and linker length to produce the cooperative binding effect. The 35-mer linker is a rigid, double helix L-DNA construct. The hybridization generates a double L-DNA helix, which is shorter and less flexible than the ss-L-DNA sequence. The helix shows reduced degrees of freedom and can be seen as a rigid linker construct. Table 7 shows, that the 35-mer linker isn't able to generate a cooperative binding event.

Extending the linker length by a highly flexible poly-T ss-L-DNA to form a 75-mer and a 95-mer, respectively, provides for an increase in affinity and especially in antigen complex stability kD (1/s).

The chi2 values indicate a high quality of the measurements. All measurements show extremely small errors. The data can be fitted to a Langmuir 1:1 fitting model residuals deviate only +/- 1 RU, small chi2 values and only 10 iterative calculations were necessary for obtaining the data.

A cooperative binding effect works according to the physical law, in that the free binding energies ΔG1 and ΔG2 summarize. The affinities multiply: KDcoop = KD1 x KD2. Furthermore, the dissociation rates also multiply: KD coop = kd1 x KD 2. This is exactly observable in the 75-mer and 95-mer linker experiment. This results in very long complex half-lives of 4146 hours (173 days) and 3942 hours (164 days), respectively. The affinities are in the range of 100 fmol/l. It is obvious, that a cooperative binding event occurs.

The association rates of all fully established complex s are faster, when compared to the singly hybridized constructs. Despite showing a higher molecular weight the association rate increases.

Here we could show, that trastuzumab and Pertuzumab linked together in a complex as reported herein simultaneously binds to the HER-2 extracellular domain (ECD). Both FAB fragments bind to genuine epitopes on the HER2-ECD (PDB 1S78 and PDB 1N82). Additionally both FAB fragments strongly differ in their binding angles. By using the optimal 75-mer (30 nm) ss-L-DNA linker length and its beneficial flexibility and length properties a cooperative binding event could be shown.

The signals were measured as analyte concentration-dependent, time resolved sensorgrams. The data was evaluated using the BIAcore BIAevaluation software 4.1. As a fitting model a standard Langmuir binary binding model was used.

### Example 7

### Additional Biomolecular Interaction Analysis

A BIAcore 3000 instrument was mounted with a CM-5 sensor chip. The sensor was preconditioned as recommended by the manufacturer (GE healthcare, Uppsala, Sweden). The system buffer was (10 mM HEPES pH 7.4, 150 mM NaCl, 1 mM EDTA, 0.05 % Tween® 20). The system buffer was also used as the sample buffer. The system was operated at 25 °C under the control software 4.1.

30 µg/ml polyclonal goat anti human IgG-Fc gamma antibody (Jackson Laboratories, USA) in 10 mM acetate buffer pH 4.5 were immobilized by standard NHS/EDC chemistry at 13,952 RU on flow cell 1 and 15,047 RU on flow cell 2. The system was regenerated at 20 µl/min using a 20 sec. pulse of a 10 mM glycine pH 1.5 buffer, a 1 min pulse of 10 mM glycine pH 1.7 buffer, and a 30 sec. pulse of 10 mM glycine pH 1.5 buffer. On flow cell 1, 5 nM huIgG (Bayer Healthcare) were injected for 1 min at 10 µl/min as a reference.

On flow cell 2, 10 nM human HER2 extracellular receptor FC chimera (hHER2-ECDpresSFc) were injected for 1 min at 10 µl/min. Typically 100 response units of the prebuilt homodimeric hHER2-ECDpresSFc were captured via the human FC portion on flow cell 2 by a goat anti human IgG-Fc gamma antibody. Typically 130 response units of huIgG were captured via the human FC portion on flow cell 1.

The signal on flow cell 2 was referenced versus flow cell 1.

The ss-L-DNA labeled FAB fragments anti-HER2 antibody 4D5-FAB'-ss-L-DNA and anti-HER2 antibody 2C4-FAB'-ss-L-DNA were hybridized with the 75mer ss-L-DNA linker by a 1:1:1 molar stoichiometry. The fully established complex 2C4-75mer-4D5 was injected for three minutes at 50 nM into the system. As a control, the single FAB fragments were injected at 50 nM into the system.

Immediately after injection end 250 nM streptavidin or system buffer was injected into the system for 3 min at 10 µl/min. Since the 75mer linker contains a single biotin moiety in the center of its sequence, the SA should work as a probe to recognize the biotin within the linker, but not the presence of the FAB fragments.

In another experiment the fully established 4D5-75mer-2C4 complex was injected into the system at different concentration steps 0 nM, 0.6 nM, 1.9 nM, 2x 5.6 nM, 16.7 nM, 50 nM at 10 µl/min for 3 min. The concentration dependent response levels of the hHER2-ECDpresSFc analyte were monitored. The response levels were plotted over the concentration steps of the hHER2-ECDpresSFc. The data was visualized using the software Origin 7. The data was fitted using the Hill equation y=Vₘₐₓ*xⁿ/(kⁿ+xⁿ) as provided by the Origin 7 software.

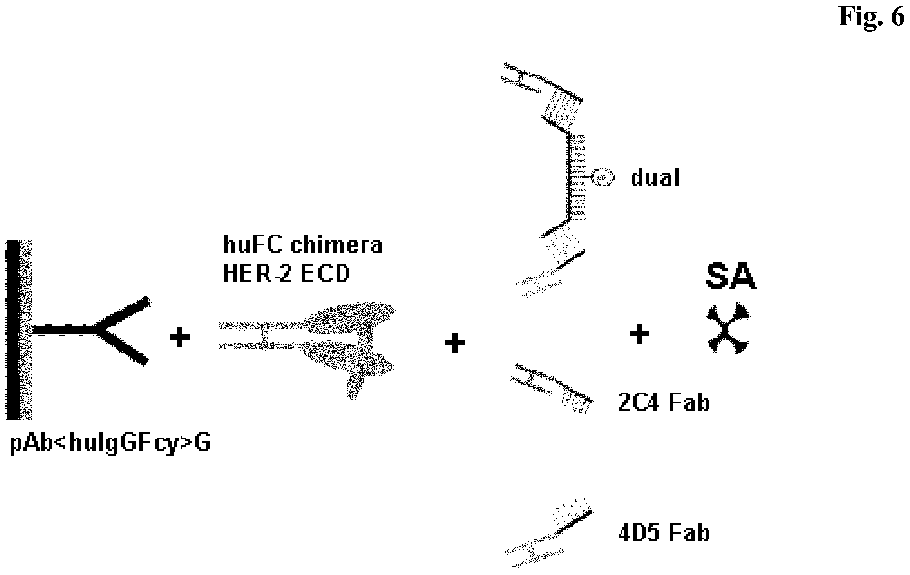

The BIAcore assay setup comprised the following (see also Figure 6): A polyclonal goat anti human IgG-Fc gamma antibody was immobilized on the BIAcore CM5 sensor and serves as a capture system for the huFc chimera HER2 ECD. Anti-HER2 antibody 2C4-FAB'-ss-L-DNA (2C4 FAB), anti-HER2 antibody 4D5-FAB'-ss-L-DNA (4D5 FAB) and fully established complexes were injected, followed by the injection of streptavidin (SA). The aim of the experiment is to demonstrate the presence and accessibility of the biotin moiety within the 75mer ss-L-DNA linker.

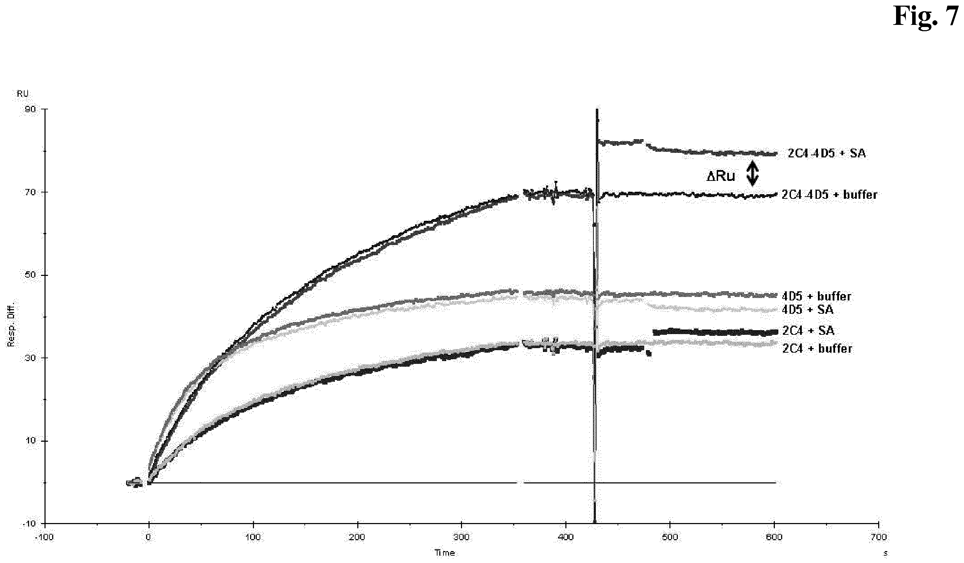

Results of the experiment are depicted in Figure 7. The BIAcore sensorgram shows an overlay plot of interaction signals upon 50 nM injections of anti-HER2 antibody 2C4-FAB'-ss-L-DNA (2C4), anti-HER2 antibody 4D5-FAB'-ss-L-DNA (4D5) and fully established complex (2C4-75mer-4D5) connected by a 75mer ss-L-DNA linker. The overlay plot shows that due to its higher mass of 137 kDa the fully established complex binder (2C4-75mer-4D5 + buffer) generates a higher signal response level, when compared to the FAB fragment injections (2C4 + buffer, 4D5 + buffer). The FAB fragments have a calculated molecular weight of 57 kDa, each. Immediately after injection end at 420 sec, 250 nM streptavidin or system buffer was injected. The double headed arrow marks the 14 RU signal shift (ΔRU) induced by the 250 nM streptavidin injection (2C4-75mer-4D5 + SA) compared to the buffer injection (2C4-75mer-4D5 + buffer). The FAB fragments show no signal shift upon SA injection and remain at the buffer signal level ((2C4 + SA), (2C4 + buffer), (4D5 + SA), (4D5 + buffer)). Streptavidin is the effector moiety. It shows the accessibility of the ss-L-DNA linker.

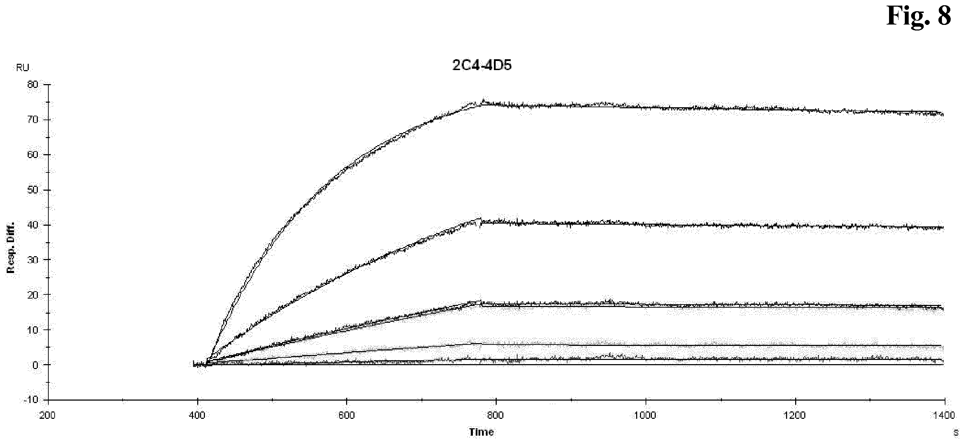

BIAcore sensorgram showing an overlay plot of concentration-dependent measurements of the fully established 75-mer complex as analyte in solution interacting with the surface presented huFc chimera HER2 ECD is shown in Figure 8. The black lines represent the 1:1 Langmuir fit on the data. Kinetic data, association rate ka = 1.25*10⁵ 1/Ms, dissociation rate KD = 3.39*10⁻⁵ 1/s, affinity constant 0.3 nM.

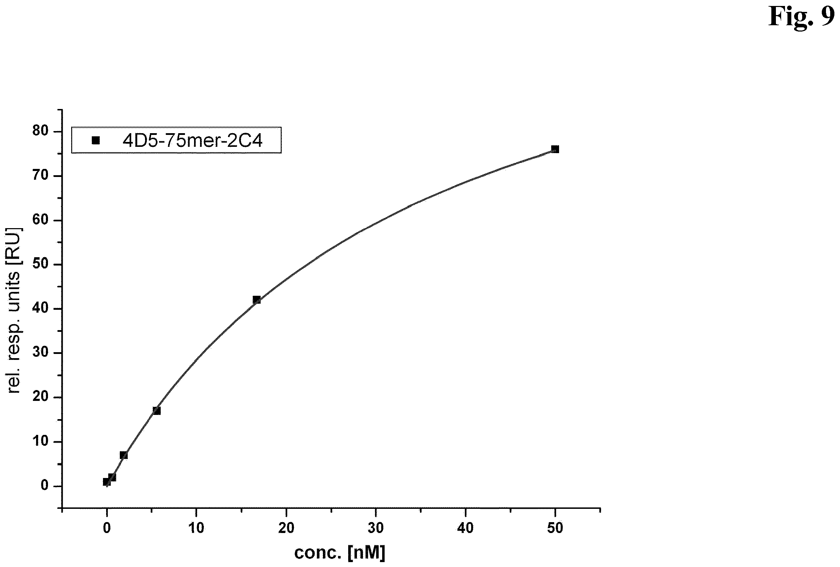

The response levels of Figure 8 were plotted versus the analyte concentration of the fully established complex (Figure 9). The data was fitted according to the hill equation and the hill factor was determined (Origin 7). Equation: y=Vₘₐₓ*xⁿ/(kⁿ+xⁿ), Chi2/DoF = 0.6653, R2= 0.99973; n = 1.00201 +/- 0.06143.

In Table 8 the kinetic data from the BIAcore assay format as depicted in Figure 6 is shown. The cooperative binding effect can be produced with the complex in solution. The Molar Ratios show, that exactly a single complex recognizes a single HER2-ECD chimera. Kinetic data, association rate ka 1/Ms, dissociation rate kd 1/s, affinity constant KD (M) and in (nM), maximum binding response signal (Rmax), amount of captured huFc Chim Her2ECD Ligand (RU), Complex halftime according to Langmuir t1/2 diss. Molar Ratio MR, indicating the stoichiometry of the binding events. Error chi2. 4D5-2C4-75mer is the fully established complex. 4D5-75mer and 2C4-75mer are the FAB fragments, but hybridized to the ss-L-DNA 75mer linker.

**Table 8.**

| **Ligand** | **Ligand (RU)** | **Analyte** | **ka (1/Ms)** | **kd (1/s)** | **t1/2 diss (min)** | **KD (M)** | **KD (nM)** | **Rmax (RU)** | **MR** | **Chi2** |
|---|---|---|---|---|---|---|---|---|---|---|
| **huFC chim. HER2 ECD** | **106** | **4D5-2C4-75mer** | **1.25E+05** | **3.39E-05** | **342** | **2.71E-10** | **0.3** | **83** | **1.1** | **0.28** |
| | **104** | **4D5-75mer** | **8.54E+04** | **1.45E-04** | **80** | **1.69E-09** | **1.7** | **46** | **1.1** | **0.18** |
| | **103** | **2C4-75mer** | **8.87E+04** | **1.17E-04** | **99** | **1.32E-09** | **1.3** | **46** | **1.1** | **0.15** |

The data presented in Table 8 demonstrate, that the fully established complex, connected via a 75mer ss-L-DNA linker shows cooperative binding. The single FAB fragments show lower affinity, when compared to the fully established complex. The signal levels at Rmax shows the increased molecular mass of the complex versus the single FAB fragments. Despite a higher signal level, the Molar Ratios are exactly at 1.1. This shows that statistically each complex binds to a single huFc chimeric HER2 ECD molecule.

The amplification factor by cooperativity is not so high when compared to the previous assay format, wherein the complex was assembled on the sensor surface. KDcoop is triggered up to 6-fold. Without being bound by theory, this could be due to the nature of the homodimeric huFc chimeric HER2 ECD. Potentially the dual binder recognizes the two separated HER2 ECDs in the huFc HER2 chimera and cannot fully establish cooperativity.

The efficient delivery of an effector moiety in form of a dye could be shown by the FACS analysis (see next example) sing the phycoerythrin-labeled streptavidin probe on living cells. The streptavidin labeled probe could easily access the biotin moiety in the 75mer ss-L-DNA linker construct.

Data form the measurement as outlined above was used for the generation of the Hill Plot (Figure 9). The Hill analysis of the complex shows, that the binding events of the FAB fragments are independent from each other and don't interfere with each other. No cooperative binding in terms of a structural disturbance of the HER2 molecule could be detected, the Hill coefficient (n = 1.00201 +/- 0.06143) is exactly 1. Therefore, the linker chemistry, the nature of the ss-L-DNA linker and the oligo-labeled FAB fragment are not negatively interfering with the target molecule recognition.

### Example 8

### Further complexes - synthesis and characterization

### Synthesis of hybridizable oligonucleotides

The following amino modified precursors comprising the sequences given in SEQ ID NOs: 05 and 06, respectively, were synthesized according to standard methods. The below given oligonucleotides not only comprise the so-called aminolinker, but also a fluorescent dye. As the skilled artisan will readily appreciate, this fluorescent dye is very convenient to facilitate purification of the oligonucleotide as such, as well as of components comprising them.
a) 5'-Fluorescein-AGT CTA TTA ATG CTT CTG C-(Spacer C3)3-C7Aminolinker-;
b) 5'-Cy5 AGT CTA TTA ATG CTT CTG C-(Spacer C3)3-C7Aminolinker-;
c) 5'-Aminolinker-(Spacer C3)3-AGT TCT ATC GTC GTC CA-Fluorescein-3';
d) 5'-Fluorescein-(beta L AGT CTA TTA ATG CTT CTG C)-(Spacer C3)3-C7Aminolinker-; (beta L indicates that this is an L-DNA oligonucleotide); and
e) 5'-Aminolinker-(Spacer C3)3-(beta L-AGT TCT ATC GTC GTC CA)-Fluorescein-3' (beta L indicates that this is an L-DNA oligonucleotide).

Synthesis was performed on an ABI 394 synthesizer at a 10 µmol scale in the trityl on (for 5' amino modification) or trityl off mode (for 3' amino modification) using commercially available CPGs as solid supports and standard dA(bz), dT, dG (iBu) and dC(Bz) phosphoramidites (Sigma Aldrich).

The following amidites, amino modifiers and CPG supports were used to introduce the C3-spacer, a dye and amino moieties, respectively, during oligonucleotide synthesis:
- spacer phosphoramidite C3 (3-(4,4'-Dimethoxytrityloxy) propyl-1-[(2-cyanoethyl)-(N,N-diisopropyl)]-phosphoramidite (Glen Research);
- 5' amino modifier is introduced by using 5'-Amino-Modifier C6 (6-(4-Monomethoxytritylamino) hexyl-(2-cyanoethyl)-(N,N-diisopropyl)-phosphoramidite (Glen Research);
- 5'-Fluorescein Phosphoramidite 6-(3',6'-dipivaloylfluoresceinyl-6-carboxamido)-hexyl-1-O-(2-cyanoethyl)-(N,N-diisopropyl)-phosphoramidite (Glen Research);
- Cy5™ Phosphoramidite 1-[3-(4-monomethoxytrityloxy) propyl]-1'-[3-[(2-cyanoethyl)-(N,N-diisopropyl phosphoramidityl] propyl]-3,3,3',3'-tetramethylindodicarbocyanine chloride (Glen Research);
- LightCycler Fluorescein CPG 500 A (Roche Applied Science); and
- 3'-Amino Modifier TFA Amino C-6 lcaa CPG 500 A (ChemGenes).

For Cy5 labeled oligonucleotides, dA(tac), dT, dG(tac), dC(tac) phosphoramidites, (Sigma Aldrich), were used and deprotection with 33% ammonia was performed for 2h at room temperature.

L- DNA oligonucleotides were synthesized by using beta-L-dA(bz), dT, dG (iBu) and dC(Bz) phosphoramidites (ChemGenes)

Purification of fluorescein modified hybridizable oligonucleotides was performed by a two-step procedure: First the oligonucleotides were purified on reversed-phase HPLC (Merck-Hitachi-HPLC; RP-18 column; gradient system [A: 0.1 M (Et₃NH)OAc (pH 7.0)/MeCN 95:5; B: MeCN]: 3 min, 20% B in A, 12 min, 20-50% B in A and 25 min, 20% B in A with a flow rate of 1.0 ml/min, detection at 260 nm. The fractions (monitored by analytical RP HPLC) containing the desired product were combined and evaporated to dryness. (Oligonucleotides modified at the 5' end with monomethoxytrityl protected alkylamino group are detriylated by incubating with 20 % acetic acid for 20 min). The oligomers containing fluorescein as label were purified again by IEX chromatography on a HPLC [Mono Q column: Buffer A: Sodium hydroxide (10 mmol/l; pH ~12) Buffer B 1 M Sodium chloride dissolved in Sodium hydroxide (10 mmol/l; pH ~12) gradient: in 30 minutes from 100% buffer A to 100% buffer B flow 1 ml/min detection at 260 nm]. The product was desalted via dialysis.

Cy5 labeled oligomers were used after the first purification on reversed-phase HPLC (Merck-Hitachi-HPLC; RP-18 column; gradient system [A: 0.1 M (Et₃NH)OAc (pH 7.0)/MeCN 95:5; B: MeCN]: 3 min, 20% B in A, 12 min, 20-50% B in A and 25 min, 20% B in A with a flow rate of 1.0 ml/min, detection at 260 nm. The oligomers were desalted by dialysis and lyophilized on a Speed-Vac evaporator to yield solids which were frozen at -24 °C.

### Activation of hybridizable oligonucleotides

The amino modified oligonucleotides from Example 2 were dissolved in 0.1 M sodium borate buffer pH 8.5 buffer (c= 600 µmol) and reacted with a 18-fold molar excess of Sulfo SMCC (Sulfosuccinimidyl 4-[N-maleimidomethyl] cyclohexane-1-carboxylate dissolved in DMF (c= 3mg/100µl) from Thermo Scientific, The reaction product was thoroughly dialyzed against water in order to remove the hydrolysis product of sulfo-SMCC 4-[N-maleimidomethyl] cyclohexane-1-carboxylate.

The dialysate was concentrated by evaporation and directly used for conjugation with a monovalent binder comprising a thiol group.

### Synthesis of linker oligonucleotides comprising hybridizable oligonucleotides at both ends

Oligonucleotides were synthesized by standard methods on an ABI 394 synthesizer at a 10 µmol scale in the trityl on mode using commercially available dT-CPG as solid supports and using standard dA(bz), dT, dG (iBu) and dC(Bz) phosphoramidites (Sigma Aldrich).

L- DNA oligonucleotides were synthesized by using commercially available beta L-dT-CPG as solid support and beta-L-dA(Bz), dT, dG (iBu) and dC(Bz) phosphoramidites (ChemGenes)

Purification of the oligonucleotides was performed as described under Example 3 on a reversed-phase HPLC. The fractions (monitored by analytical RP HPLC) containing the desired product were combined and evaporated to dryness. Detritylation was performed by incubating with 80 % acetic acid for 15 min). The acetic acid was removed by evaporation. The reminder was dissolved in water and lyophilized

The following amidites and CPG supports were used to introduce the C18 spacer, digoxigenin and biotin group during oligonucleotide synthesis:
- spacer phosphoramidite 18 (18-O-Dimethoxytritylhexaethyleneglycol,1-[(2-cyanoethyl)-(N,N-diisopropyl)]-phosphoramidite (Glen Research);
- biotin-dT (5'-Dimethoxytrityloxy-5-[N-((4-t-butylbenzoyl)-biotinyl)-aminohexyl)-3-acrylimido]-2'-deoxyUridine-3'-[(2-cyanoethyl)-(N,N-diisopropyl)]-phosphoramidite (Glen Research);
- biotin Phosphoramidite 1-Dimethoxytrityloxy-2-(N-biotinyl-4-aminobutyl)-propyl-3-O-(2-cyanoethyl)-(N,N-diisopropyl)-phosphoramidite and
- 5'-Dimethoxytrityl-5-[N-(trifluoroacetylaminohexyl)-3-acrylimido]-2'-deoxy uridine, 3'-[(2-cyanoethyl)-(N,N-diisopropyl)]-phosphoramidite for amino modification and postlabeling with Digoxigenin-N-Hydroxyl-succinimidyl ester.

**The following bridging constructs or linkers were synthesized:**

| | |
|---|---|
| Linker 1: | |
| Linker 2: | |
| Linker 3: | |
| Linker 4: | |
| Linker 5: | |
| Linker 6: | |
| Linker 7: | |
| Linker 8: | |
| Linker 9: | |
| Linker 10: | |
| Linker 11: | |
| Linker 12: | |
| Linker 13: | |
| Linker 14: | |
| Linker 15: | |
| Linker 16: | |
| Linker 17: | |

The above bridging construct examples comprise at least a first hybridizable oligonucleotide and a second hybridizable oligonucleotide. Linkers 3 to 18 in addition to the hybridizable nucleic acid stretches comprise a central biotinylated or digoxigenylated thymidine, respectively, or a spacer consisting of thymidine units of the length given above.

The 5'-hybridizable oligonucleotide corresponds to SEQ ID NO: 07 and the 3'-hybridizable oligonucleotide corresponds to SEQ ID NO: 08, respectively. The oligonucleotide of SEQ ID NO: 07 will readily hybridize with the oligonucleotide of SED ID NO: 06. The oligonucleotide of SEQ ID NO: 08 will readily hybridize with the oligonucleotide of SED ID NO: 05.

In the above bridging construct examples [B-L] indicates that an L-DNA oligonucleotide sequence is given; spacer C 18, Biotin and Biotin dT respectively, refer to the C18 spacer, the Biotin and the Biotin-dT as derived from the above given building blocks; and T with a number indicates the number of thymidine residues incorporated into the linker at the position given.

### Assembly of the complex

### A) Cleavage of IgGs and labeling of FAB' fragments with ss-DNA

Purified monoclonal antibodies were cleaved with the help of pepsin protease yielding F(ab')₂ fragments that are subsequently reduced to FAB' fragments by treatment with low concentrations of cysteamine at 37°C. The reaction is stopped via separation of cysteamine on a PD 10 column. The FAB' fragments are labeled with an activated oligonucleotide as produced according to Example 3. This single-stranded DNA (=ss-DNA) bears a thiol-reactive maleimido group that reacts with the cysteines of the FAB' hinge region. In order to obtain high percentages of single-labeled FAB' fragments the relative molar ratio of ss-DNA to FAB'-fragment is kept low. Purification of single-labeled FAB' fragments (ss-DNA: FAB' = 1:1) occurs via ion exchange chromatography (column: Source 15 Q PE 4.6/100, Pharmacia/GE). Verification of efficient purification is achieved by analytical gel filtration and SDS-PAGE.

### B) Assembly of a complex comprising two polypeptides specifically binding to a target

The anti-pIGF-1R complex is based on two FAB' fragments that target different epitopes of the intracellular domain of IGF-1R: FAB' 8.1.2 detects a phosphorylation site (pTyr 1346) and FAB' 1.4.168 a non-phospho site of the target protein. The FAB' fragments have been covalently linked to single-stranded DNA (ss-DNA): FAB' 1.4.168 to a 17mer ss-DNA comprising SEQ ID NO: 05 and containing fluorescein as a fluorescent marker and FAB' 8.1.2 to a 19mer ss-DNA comprising SEQ ID NO: 06 and containing Cy5 as fluorescent marker. In the following, these FAB's with covalently bound 17mer or 19mer ss-DNA are named ss-FAB' 1.4.168 and ss-FAB' 8.1.2 respectively. Complex assembly is mediated by a linker (i.e. a bridging construct comprising two complementary ss-DNA oligonucleotides (SEQ ID NOs: 7 and 8, respectively) that hybridize to the corresponding ss-DNAs of the ss-FAB' fragments. The distance between the two ss-FAB' fragments of the complex can be modified by using spacers, e.g. C18-spacer or DNAs of different length, respectively.

For assembly evaluation the complex components ss-FAB' 8.1.2, ss-FAB' 1.4.168 and the linker constructs (1) (= linker 17 of example 2.4) 5'-G CAG AAG CAT TAA TAG ACT T(-Bi)-TGG ACG ACG ATA GAA CT-3' and (2) (= linker 10 of example 2.4) 5'-G CAG AAG CAT TAA TAG ACT-T(20)-T(-Bi)-(T20)-TGG ACG ACG ATA GAA CT-3' were mixed in equimolar quantities at room temperature. After a 1 minute incubation step the reaction mix was analyzed on an analytical gel filtration column (Superdex™ 200, 10/300 GL, GE Healthcare). Comparison of the elution volumes (V_{E}) of the single complex components with the V_{E} of the reaction mix demonstrates that the complex has been formed successfully (Figure 10).

### BIAcore experiment assessing binding of anti-pIGF-1R complex to immobilized IGF-1R and IR peptides

For this experiment a BIAcore 2000 instrument (GE Healthcare) was used with a BIAcore SA sensor mounted into the system at T = 25° C. Preconditioning occurred at 100 µl/min with 3x1 min injection of 1 M NaCl in 50 mM NaOH and 1 min 10 mM HCl.

HBS-ET (10 mM HEPES pH 7.4, 150 mM NaCl, 1mM EDTA, 0.05% Tween® 20 was used as system buffer. The sample buffer was identical with the system buffer. The BIAcore 2000 System was driven under the control software V1.1.1.

Subsequently biotinylated peptides were captured on the SA surface in the respective flow cells. 16 RU of IGF-1R(1340-1366)[1346-pTyr; Glu(Bi-PEG-1340]amid (i.e. the - 1346 tyrosine phosphorylated - peptide of SEQ ID NO:11 comprising a PEG-linker bound via glutamic acid corresponding to position 1340 and being biotinylated at the other end of the linker) was captured on flow cell 2. 18 RU of IGF-1R(1340-1366); Glu(Bi-PEG-1340]amid (i.e. the - 1346 tyrosine non-phosphorylated - peptide of SEQ ID NO:11 comprising a PEG-linker bound via glutamic acid corresponding to position 1340 and being biotinylated at the other end of the linker) was captured on flow cell 3. 20 RU of hInsR(1355-1382)[1361-pTyr; Glu(Bi-PEG-1355]amid (i.e. the - 1361 tyrosine phosphorylated - peptide of SEQ ID NO: 12 comprising a PEG-linker bound via glutamic acid corresponding to position 1355 of human insulin receptor and being biotinylated at the other end of the linker) was captured on flow cell 4. Finally all flow cells were saturated with d-biotin.

For the complex formation the assembly protocol as described above was used. When individual runs with only one of the two ss-FAB's were performed, the absence or presence of linker DNA did not affect the association or dissociation curves.

100 nM of analyte (i.e. in these experiments a bivalent dual binding agent) in solution was injected at 50 µl/min for 240 sec association time and dissociation was monitored for 500 sec. Efficient regeneration was achieved by using a 1 min injection step at 50 µl /min with 80 mM NaOH. Flow cell 1 served as a reference. A blank buffer injection was used instead of an antigen injection to double reference the data by buffer signal subtraction.

In each measurement cycle one of the following analytes in solution was injected over all 4 flow cells: 100 nM ss-FAB' 8.1.2, 100 nM ss-FAB' 1.4.168, a mixture of 100 nM ss-FAB' 8.1.2 and 100 nM ss-FAB', 100 nM bivalent binding agent consisting of ss-FAB' 8.1.2 and ss-FAB' 1.4.168 hybridized on linker (3) (5'-G CAG AAG CAT TAA TAG ACT-T(20)-T(-Dig)-(T20)-TGG ACG ACG ATA GAA CT-3'(= linker 15) ), and 100 nM bivalent binding agent consisting of ss-FAB' 8.1.2 and ss-FAB' 1.4.168 hybridized on linker (1) (5'-G CAG AAG CAT TAA TAG ACT-T(-Dig) -TGG ACG ACG ATA GAA CT-3'(= linker 16)), respectively.

The signals were monitored as time-dependent BIAcore sensorgrams.

Report points were set at the end of the analyte association phase (Binding Late, BL) and at the end of the analyte dissociation phase (Stability Late, SL) to monitor the response unit signal heights of each interaction. The dissociation rates kd (1/s) were calculated according to a linear 1:1 Langmuir fit using the BIAcore evaluation software 4.1. The complex halftimes in minutes were calculated upon the formula ln(2)/(60*kD).

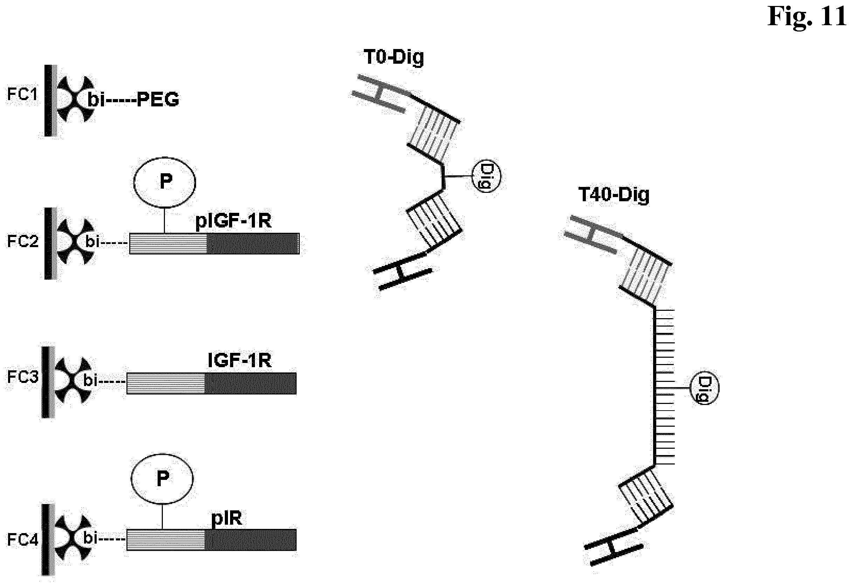

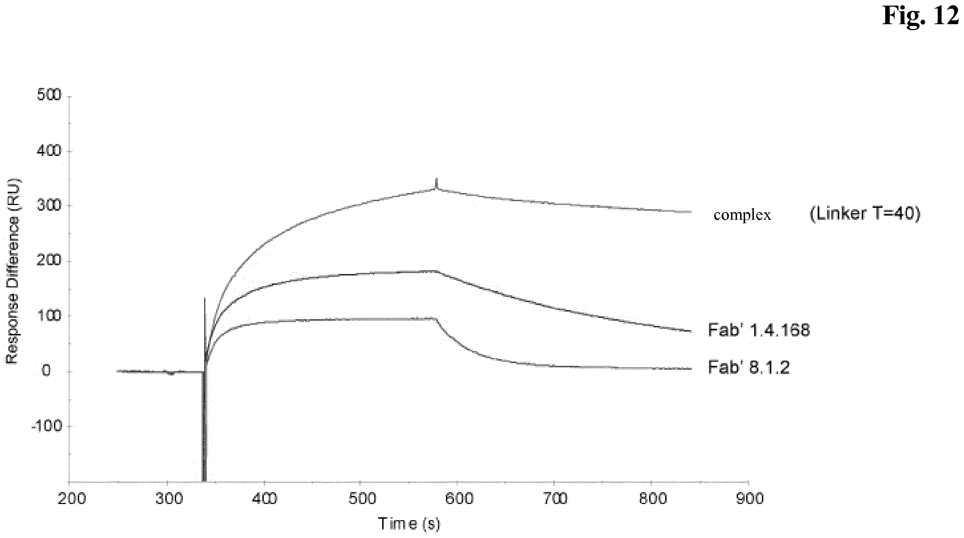

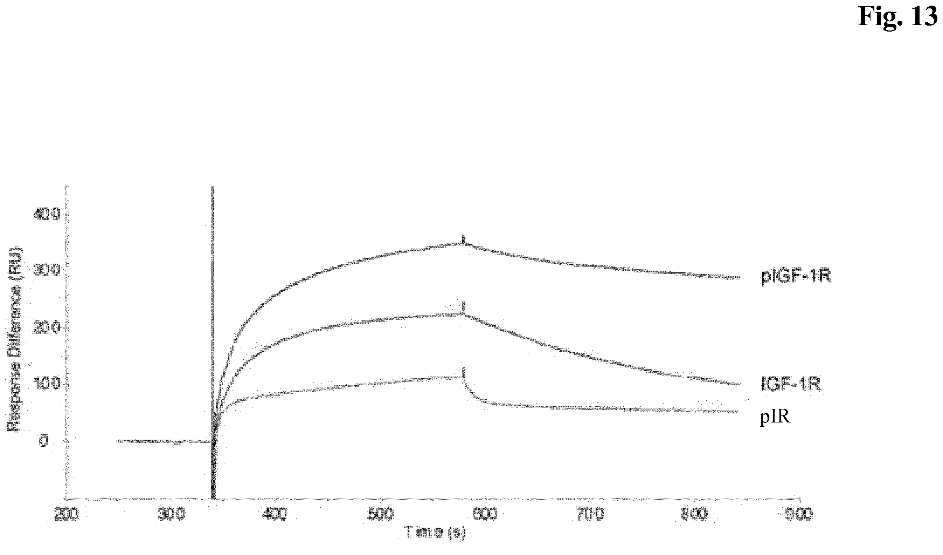

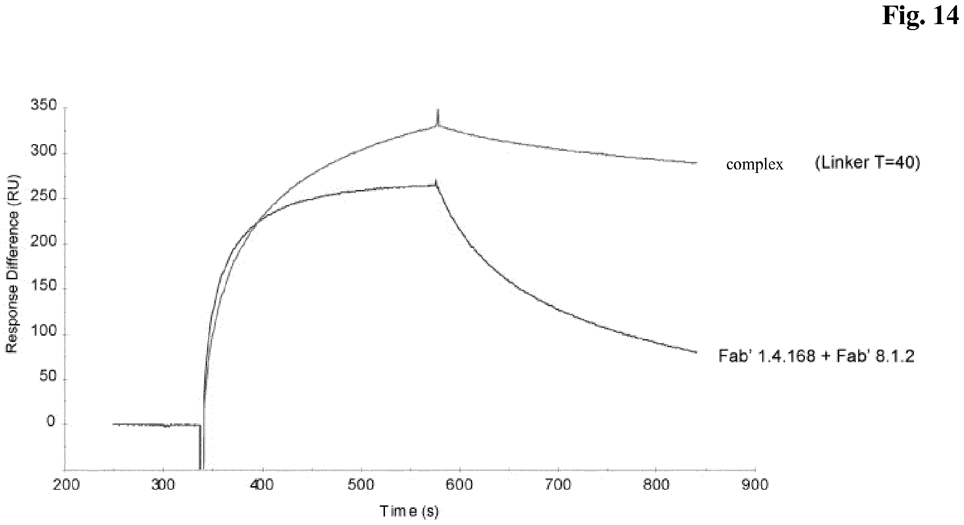

The sensorgrams (Figure 11 to Figure 14) show a gain in both specificity and complex stability in pIGF-1R binding when ss-FAB' 1.4.168 and ss-FAB' 1.4.168 are used in form of a complex (= bivalent binding agent), probably due to the underlying cooperative binding effect. FAB' 1.4.168 alone shows no cross reactivity for the pIR peptide but does not discriminate between the phosphorylated and non-phosphorylated form of IGF-1R (T1/2 dis = 3 min in both cases). FAB' 8.1.2, however, binds only to the phosphorylated version of the IGF1-R peptide but exhibits some undesired cross reactivity with phosphorylated Insulin Receptor. The complex discriminates well between the pIGF-1R peptide and both other peptides (see Figure 13) and thus helps to overcome issues of unspecific binding. Note that the gain in specificity is lost when both FAB's are applied without linker DNA (Figure 14). The gain in affinity of the Complex towards the pIGF-1R peptide manifests in increased dissociation half times compared to individual FAB's and the FAB' mix omitting the linker DNA (Figure 12 and Figure 14). Although the tested Complex s with two different DNA linker share an overall positive effect on target binding specificity and affinity, the longer linker (with T40-Dig as a spacer) (i.e. linker 15) seems to be advantageous with respect to both criteria.

### BIAcore assay sandwich of M-1.4.168-IgG and M-8.1.2

A BIAcore T100 instrument (GE Healthcare) was used with a BIAcore CM5 sensor mounted into the system. The sensor was preconditioned by a 1 min injection at 100 µl/min of 0.1 % SDS, 50 mM NaOH, 10 mM HCl and 100 mM H3PO4.

The system buffer was HBS-ET (10 mM HEPES pH 7.4, 150 mM NaCl, 1mM EDTA, 0.05% Tween® 20). The sample buffer was the system buffer.

The BIAcore T100 System was driven under the control software V1.1.1. Polyclonal rabbit IgG antibody <IgGFCγM>R (Jackson ImmunoResearch Laboratories Inc.) at 30 µg/ml in 10 mM Na-Acetate pH 4.5 was immobilized at 10 000 RU on the flow cells 1, 2, 3, and 4, respectively, via EDC/NHS chemistry according to the manufacturer's instructions. Finally, the sensor surface was blocked with 1M ethanolamine. The complete experiment was driven at 13 °C.

500 nM primary mAb M-1.004.168-IgG was captured for 1 min at 10 µl /min on the <IgGFCγM>R surface. 3 µM of an IgG fragment mixture (of IgG classes IgG1, IgG2a, IgG2b, IgG3) containing blocking solution was injected at 30 µl/min for 5 min. The peptide IGF-1R(1340-1366)[1346-pTyr; Glu(Bi-PEG-1340]amid was injected at 300 nM for 3 min at 30 µl/min. 300 nM secondary antibody M-8.1.2-IgG was injected at 30 µl min. The sensor was regenerated using 10 mM Glycine-HCl pH 1.7 at 50 µl/min for 3 min.

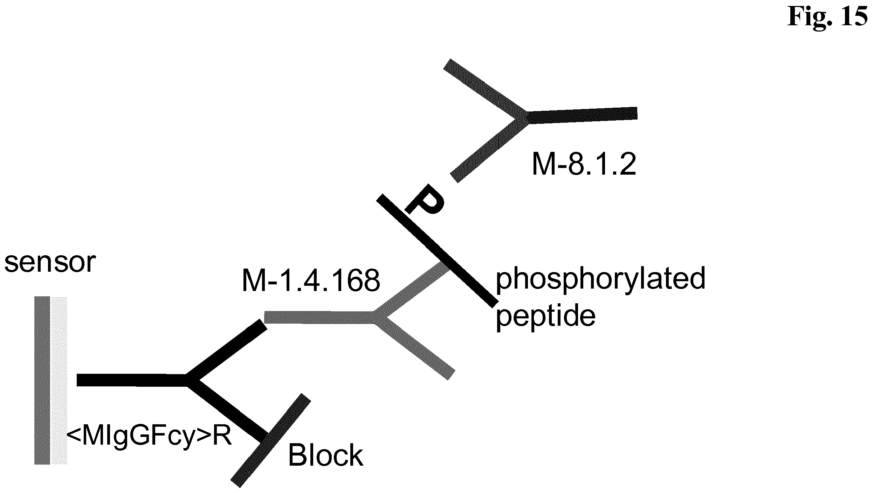

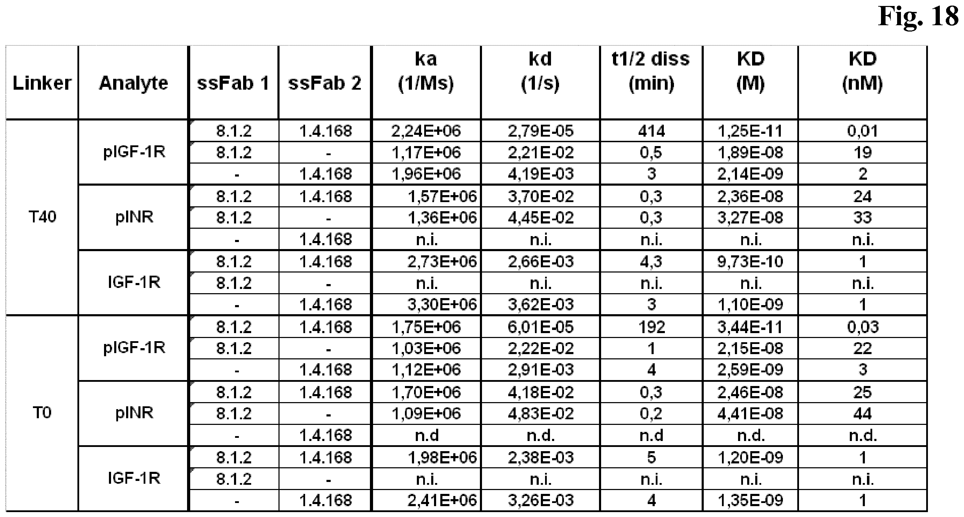

In Figure 15 the assay setup is presented. In Figure 18 the measurement results are given. The measurements clearly indicate that both monoclonal antibodies are able to simultaneously bind two distinct, unrelated epitopes on their respective target peptide. This is a prerequisite to any latter experiments with the goal to generate cooperative binding events.

### BIAcore assay complex on sensor surface

A BIAcore 3000 instrument (GE Healthcare) was used with a BIAcore SA sensor mounted into the system at T = 25 °C. The system was preconditioned at 100 µl/min with 3x1 min injection of 1 M NaCl in 50 mM NaOH and 1 min 10 mM HCl.

The system buffer was HBS-ET (10 mM HEPES pH 7.4, 150 mM NaCl, 1mM EDTA, 0.05% Tween® 20). The sample buffer was the system buffer.

The BIAcore 3000 System was driven under the control software V4.1.

124 RU amino-PEO-biotin were captured on the reference flow cell 1. 1595 RU biotinylated 14.6 kDa T0-Bi 37-mer ss-DNA-Linker (1) (5'-G CAG AAG CAT TAA TAG ACT-T(-Bi)-TGG ACG ACG ATA GAA CT-3') (= linker 17 of example 2.4) and 1042 RU biotinylated 23.7 kDa T40-Bi 77-mer ss-DNA-Linker (2) (5'-G CAG AAG CAT TAA TAG ACT-T(20)- (Biotin-dT)-(T20)-TGG ACG ACG ATA GAA CT-3'= linker 10) were captured on different flow cells.

300 nM ss-FAB 8.1.2 and 300 nM ss-FAB 1.004.168 were injected into the system at 50 µl/min for 3 min. As a control only 300 nM ss-FAB 8.1.2 or 300 nM ss-FAB 1.004.168 was injected to test the kinetic contribution of each ss-FAB. As a control, buffer was injected instead of the ss-Fabs. The peptides IGF-1R(1340-1366)[1346-pTyr]amid, INR(1355-1382)[1361-pTyr]amid IGF-1R(1340-1366)amid and were injected into system at 50 µl/min for 4 min, free in solution, in concentration steps of 0 nM, 4 nM, 11 nM, 33 nM (twice), 100 nM and 300 nM. In another embodiment to measure the affinities versus peptides IGF-1R(1340-1366)[1346-pTyr]amid the concentration steps of 0 nM, 0.4 nM, 1.1 nM, 3.3 nM (twice), 10 nM and 30 nM.

The dissociation was monitored at 50 µl/min for 5.3 min. The system was regenerated after each concentration step with a 12 sec pulse of 250 mM NaOH and was reloaded with ss-FAB ligand.

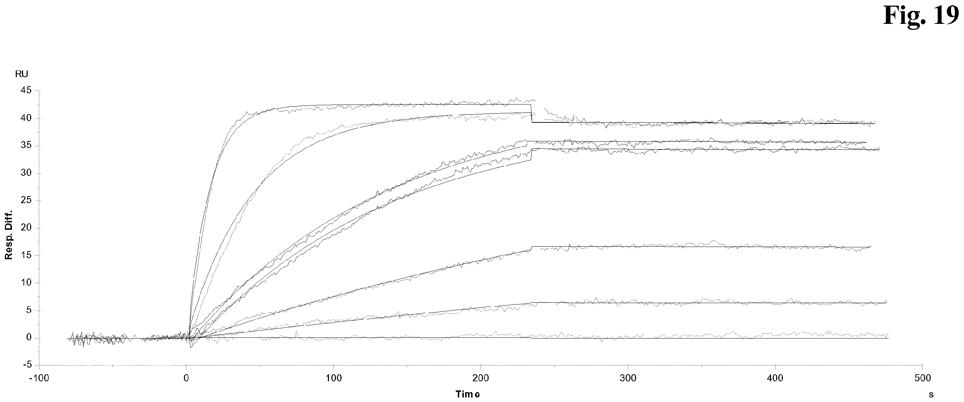

Figure 17 schematically describes the assay setup on the BIAcore instrument. The tables given in Figure 18 show the quantification results from this approach. Figure 19, Figure 20 and Figure 21 depict exemplary BIAcore results from this assay setup.

The table in Figure 18 demonstrates the benefits of the complex concept. The T40 dual binding agent (a dual binding agent with linker 10 of example 2.4, i.e. a linker with a spacer of T20-Biotin-dT-T20) results in a 2-fold improved antigen complex halftime (414 min) and a 3-fold improved affinity (10 pM) as compared to the T0 dual binding agent (i.e. a dual binding agent with linker 16) with 192 min and 30 pM, respectively. This underlines the necessity to optimize the linker length to generate the optimal cooperative binding effect.

The T40 dual binding agent (i.e. the dual binding agent comprising the T40-Bi linker (linker 10)) exhibits a 10 pM affinity versus the phosphorylated IGF-1R peptide (table in Figure 18, Figure 19). This is a 2400-fold affinity improvement versus the phosphorylated insulin receptor peptide (24 nM) and a 100-fold improvement versus the non-phosphorylated IGF-1R peptide.

Therefore, the goal to increase specificity and affinity by the combination of two distinct and separated binding events is achieved.

The cooperative binding effect especially becomes obvious from the dissociation rates against the phosphorylated IGF-1R peptide, where the complex shows 414 min antigen complex halftime, versus 0.5 min with the monovalent binder 8.1.2 alone and versus 3 min with the monovalent binder 1.4.168 alone, respectively.

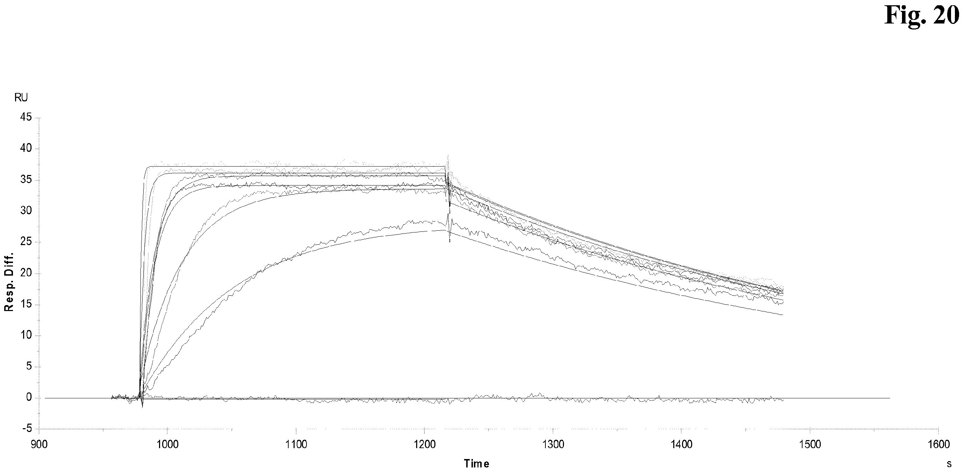

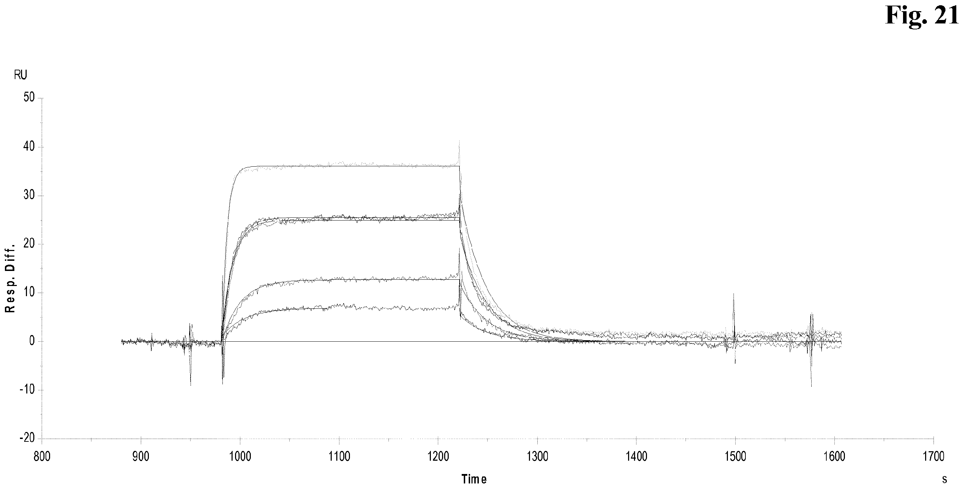

Furthermore, the fully assembled construct roughly multiplies its dissociation rates kd (1/s), when compared to the singly FAB hybridized constructs (Fig 21, Figure 20, Figure 21 and table in Figure 18). Interestingly, also the association rate ka (1/Ms) slightly increases when compared to the single FAB interaction events, this may be due to an increase of the construct's molecular flexibility.

### Example 9

### Binding assays - in vitro and ex vivo

### Detection oligonucleotide Probe-Cy5

The ss-L-DNA detection oligonucleotide Probe-Cy5 5' Cy5-Y- ATG CGA-GTA CCT TAG AGT C -Z-Cy5 3' (SEQ ID NO: 72), has been synthesized by state of the art oligonucleotide synthesis methods. The introduction of the Cy5 dye was done via reaction of the amino groups with Cy5 monoreactive NHS ester. (GE Healthcare Lifescience, STADT, LAND). For the nucleotides L-DNA amidites (ChemGenes, STADT, LAND) were used. The 5' and 3' amino groups were introduced during the solid phase oligonucleotide synthesis process wherein Y = 5'-Amino-Modifier C6 introduced via (6-(4-Monomethoxytritylamino)hexyl-(2-cyanoethyl)-(N,N-diisopropyl)-phosphoramidite (Glen Research), and Z = 3'-Aminomodifier C6 introduced via 3'Aminomodifier TFA Amino C6 long chain aminoalkyl Controlled Pore Glass 1000 A (ChemGenes).

### Dual Binder Linker oligonucleotide

The ss-L-DNA oligonucleotide linker SEQ ID NO: 73 5'-G CAG AAG CAT TAA TAG ACT-T20-GAC TCT AAG GTA CTC GCA T-T20-TGG ACG ACG ATA GAA CT-3' has been synthesized by state of the art oligonucleotide synthesis methods.

### Assembly of the complex

The complex was assembled by hybridizing the anti-HER2 antibody 2C4-FAB'-ss-L-DNA labeled with FITC and the anti-HER2 antibody 4D5-FAB'-ss-L-DNA labeled with FITC in equimolar stoichiometry with the ss-L-DNA linker of SEQ ID NO: 73. In order to verify the correct assembly of the complex, the complex was subjected to an SEC chromatography step and was filtered through a sterile filter.

### In vitro binding assay

Human breast cancer KPL-4 cells were seeded with a concentration of 2x10⁶ cells/ml in a volume of 30 µl into µ-slides VI (ibidi, Germany). Three hours thereafter, 70 µl medium (RPMI 1640, 2 mM L-glutamine, 10% FCS) was added to allow the cells to adhere.

After an incubation of 24 hours at 37 °C and 5 % CO₂ in a water saturated atmosphere (effective for all following incubations), the supernatant was removed and cells were washed once with 100 µl PBS to remove residual medium.

For the sequential application, 50 µl of the complex 4D5-2C4 as prepared above labeled with FITC solution (c = 2.5 µg/ml) was added and incubated for 45 minutes, followed by one washing step with 100 µl PBS and a further incubation with 50 µl of the DNA-probe (SEQ ID NO: 72) at an equimolar amount (0.13 µg/ml).

The pre-mixed procedure was performed by first mixing the complex and the detection Probe. Thereafter it was added to the cells (concentrations see above) followed by incubation for 45 minutes.

Xolair®, a humanized IgG1 monoclonal antibody targeting human IgE immunoglobulin was used as a negative control and Herceptin® labeled with Cy5 targeting human HER-2 receptor was used as a positive control. Both antibodies were applied at the same concentration (2.5 µg/ml).

Subsequently, the supernatant was removed and cells were washed once with 100 µl PBS. Cell nuclei were afterwards stained with DAPI by adding 50 µl of a HOECHST33342 solution (c =10 µg/ml) and incubated for 15 minutes. To remove the cell staining dye, cells were washed twice with 100 µl PBS after removal of the supernatant. Another 120 µl PBS were added to keep the cells moist to ensure viability. All dilutions were made with medium (without L-Glutamine and FCS) to ensure viability of the cells and to avoid detachment of the cells. After this procedure, slides were imaged by multispectral fluorescence analysis using the NUANCE System (CRi, Cambridge, USA). Images were normalized for comparability of the fluorescence intensities.

### Ex vivo analysis

Immunodeficient SCID beige mice with established KPL-4 tumors (orthotopically implanted) were injected i.v. with 50 µg complex in 100 µl PBS and 18 hours thereafter the Cy5-labeled DNA-probe was injected at an equimolar concentration (2.63 µg per mouse). Tumors were explanted 48 hours thereafter and examined by multispectral fluorescence analysis using the MAESTRO system (CRi, Cambridge, USA).

### Results

### In vitro binding assay

The complex is doubly FITC labeled via each of its FAB'-ss-L-DNA components. The detection probe is a doubly Cy5 labeled ss-L-DNA 20-mer oligonucleotide probe, which can be hybridized to the 95mer ss-L-DNA linker of the complex.

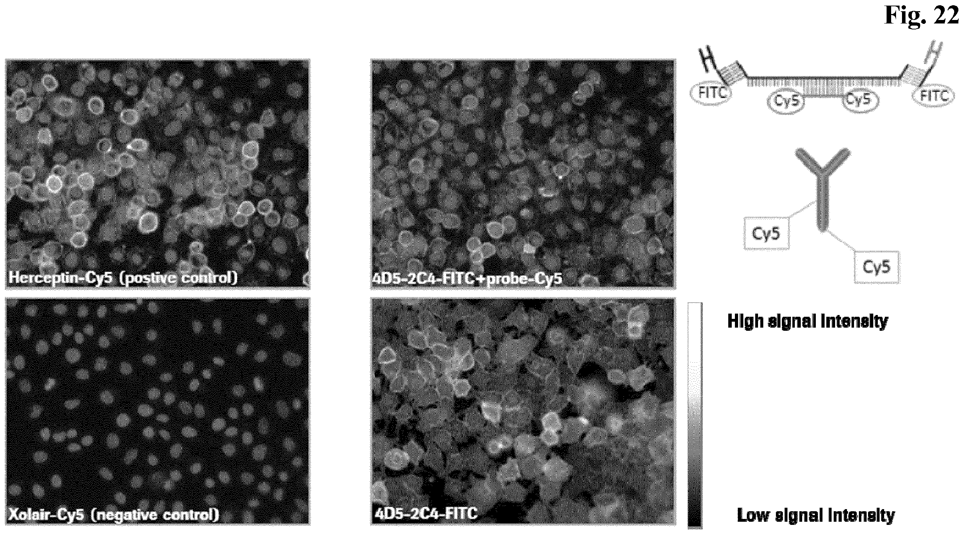

In contrast to Xolair-Cy5 (no fluorescence signal, negative control) Herceptin-Cy5 specifically stained the tumor cells (Figure 22). The FITC labeled 4D5-2C4-95mer complex specifically binds to KPL-4 tumor cells as can be seen by sequential incubation with the detection Probe (measured in the Cy5 fluorescence channel) which is co-localized with the complex to the tumor cells indicating the hybridization of the detection oligonucleotide Probe-Cy5 to the complex. In the sequential incubation mode as well as in the pre-mixed setting specific staining of the tumor cells to the Her-2 antigen could be demonstrated (Figure 2).

In Figure 22 the near infrared image of the cancer cells incubated with the complex and the detection probe is shown (NIRF imaging). In the top right of the Figure a sketch of the fully assembled 4D5-2C4-95mer complex hybridized to the Cy5 labeled detection oligonucleotide is shown. In the middle right of the Figure a cartoon of Cy5 labeled Herceptin is shown. In the bottom right of the Figure the signal intensity bar is shown.

In the top left of Figure 22 the binding of Cy5 labeled Herceptin® to the cancer cells is shown (positive control). The KPL-4 cell membranes appear as bright lighting rings surrounding the DAPI-stained cell nuclei. In the bottom left the incubation of Cy5 labeled Xolair® is shown (negative control). No membrane staining but the DAPI stain of the cell nuclei can be detected. In the bottom middle of the Figure the binding of the 4D5-2C4-FITC complex is shown. The fluorescein signal of the membrane bound complex appears as lighting rings surrounding the DAPI stained cell nuclei. In the top middle of the Figure the binding of the 4D5-2C4-FITC complex and the Cy5 labeled detection probe is shown. The detection of the complex via the Cy5 labeled ss-L-DNA detection probe, which was sequentially hybridized, can be seen. The Cy5 signal of the detection oligonucleotide appears as membrane staining, showing bright lighting rings surrounding the DAPI stained cell nuclei.

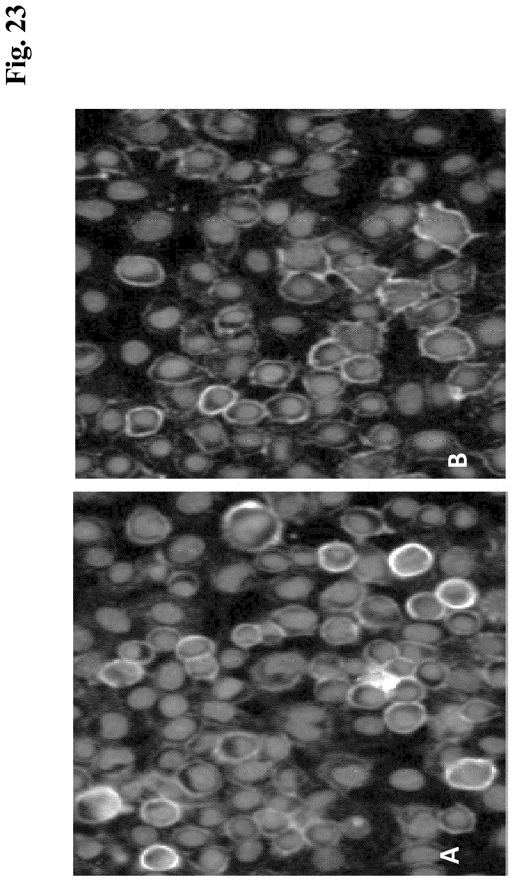

In Figure 23 the near infrared (NIRF) imaging of KPL-4 cells is shown. In Figure 23A the results of the sequential application of FITC labeled 4D5-2C4 complex and the Cy5 labeled detection probe is shown. In Figure 23B the results of the incubation of KPL-4 cells with premixed FITC labeled 4D5-2C4 complex and Cy5 labeled detection probe is shown. Both images show membrane-located signals. As a control, cells were stained with DAPI.

The experiment demonstrates that the complex as reported herein can first be applied in order to specifically target HER-2 positive cells. In a second step, the labeled detection probe can be applied in order to hybridize to the target bound complex. The fluorescence labeled detection probe is thereby a proof of concept for the time delayed, sequential application and specific targeting of an oligonucleotide-based effector moiety. In this case the payload is a fluorescent dye for the purpose of in vitro cell imaging.

### Ex vivo binding assay

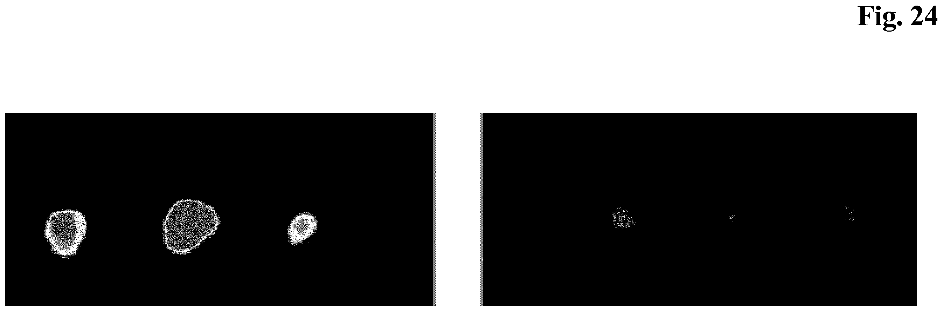

As depicted in Figure 24 (left image) a strong fluorescence signal is detectable in the experimental setting where the sample was incubated first with the complex and thereafter with the Cy5 labeled detection probe. In contrast (right image), no fluorescence signal could be detected in the tumors previously injected in the KPL-4 xenograft with the Cy5 labeled detection probe alone.

Figure 24 shows explanted KPL-4 tumors subjected to NIRF Imaging. In the first image Cy5 fluorescence signals obtained from three KPL-4 tumors explanted from mice, which were sequentially treated with the first the 4D5-2C4 complex and thereafter the detection probe is shown. In the right image it is shown that no fluorescence signal was obtained from three KPL-4 tumors, when three mice where treated with detection probe alone, omitting the 4D5-2C4 complex.

### Example 10

### Inhibition of cell proliferation in MDA-MB-175 breast cancer cell line

2 x 10⁴ MDA-MB-175 breast cancer cells cultured in DMEM / F12 medium supplemented with 10 % fetal calve serum, 2 mM Glutamine and Penicillin/Streptomycin were seeded in 96-well plates. Antibodies and complex, respectively, were added in the indicated concentrations the next day (40 to 0.0063 µg/ml). Alter 6 day incubation Alamar Blue was added and plates were incubated for 3-4 h in a tissue culture incubator. Fluorescence was measured (excitation 530 nm/emission 590) and percentage inhibition was calculated using untreated cells as reference.

### Results

The anti-HER2 antibody 2C4 (Pertuzumab) showed a maximum inhibition of 44 %. The anti-HER2 antibody Herceptin showed a maximum inhibition of 9 %. The complex as reported herein comprising the FAB fragments of Pertuzumab and Herceptin® shows a maximum inhibition of 46 %.

It has to be pointed out that Pertuzumab was tested as full length IgG antibody with two HER2 binding sites, whereas the complex comprises a single Pertuzumab Fab fragment with a single HER2 binding site.

### Example 11

### Freeze-Thaw-Stability of the complex

The complex was assembled by hybridizing the anti-HER2 antibody 2C4-FAB'-ss-L-DNA labeled with FITC and the anti-HER2 antibody 4D5-FAB'-ss-L-DNA labeled with FITC in equimolar stoichiometry with the ss-L-DNA linker of SEQ ID NO: 73. In order to verify the correct assembly of the complex, the complex was subjected to a SEC chromatography step and was filtered through a sterile filter.

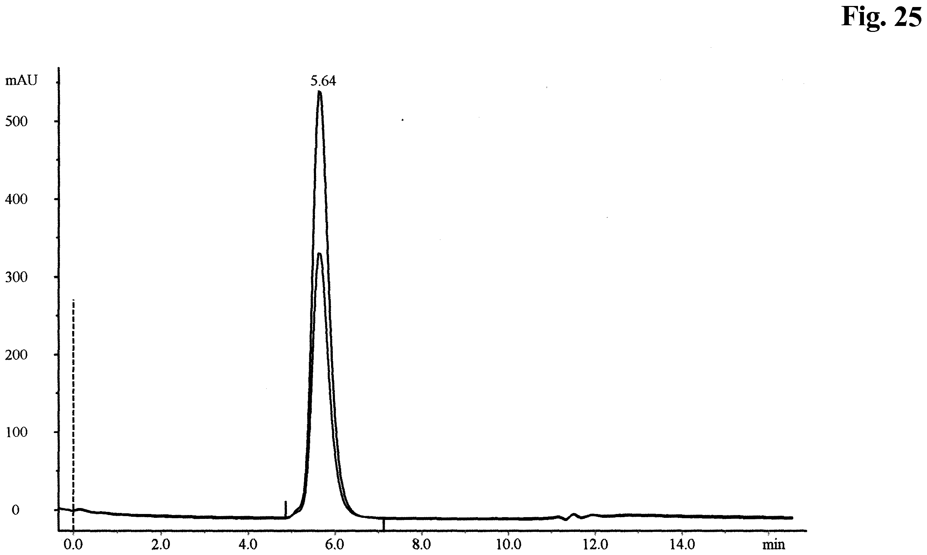

Fifty µl of the complex (1.5 mg/ml) were analyzed by analytical SEC using a TSK3000 column (GE). The running buffer was 0.1 M KH₂PO₄ pH 6.8. The flow rate was 1 ml/min. The chromatogram is shown in Figure 25.

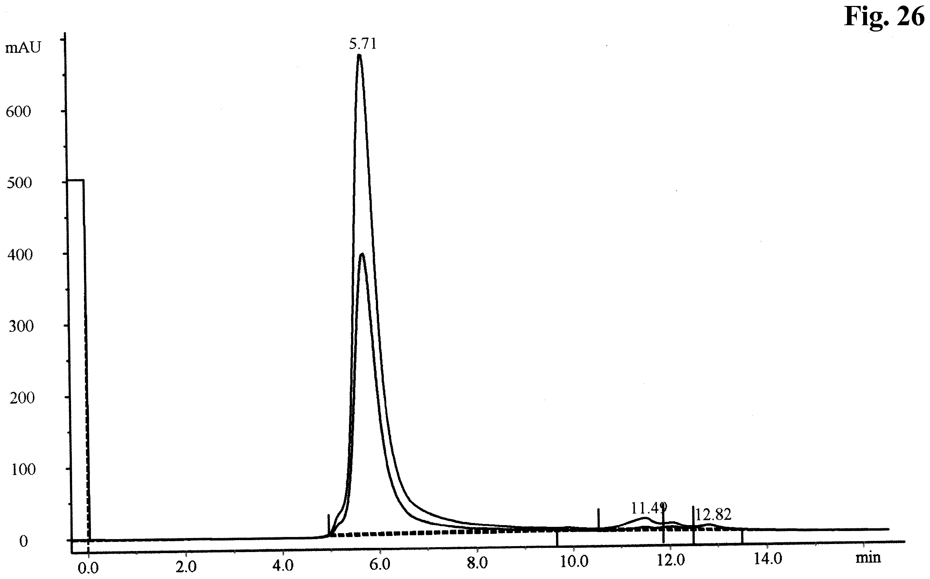

After freezing and thawing, the complex was re-chromatographed. Fifty µl of the complex (1.5 mg/ml) were analyzed by analytical SEC using a TSK3000 column (GE). The running buffer was 0.1 M KH₂PO₄ pH 6.8. The flow rate was 1 ml/min. The chromatogram is shown in Figure 26.

### Example 12

### Cloning and expression of the binding entities

### Description of the basic/standard mammalian expression plasmid

Desired proteins were expressed by transient transfection of human embryonic kidney cells (HEK 293). For the expression of a desired gene/protein a transcription unit comprising the following functional elements was used:
- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence (SS),
- a gene/protein to be expressed (e.g. full length antibody heavy chain), and
- the bovine growth hormone polyadenylation sequence (BGH pA).

Beside the expression unit/cassette including the desired gene to be expressed the basic/standard mammalian expression plasmid contains
- an origin of replication from the vector pUC18 which allows replication of this plasmid in E. coli, and
- a beta-lactamase gene which confers ampicillin resistance in E. coli.

### Cloning

First, cloning of the binding entity (such as an antibody Fab fragment) encoding constructs is performed. The plasmid with the binding entity encoding nucleic acid is usually obtained by gene synthesis, whereby the C-terminal region of the encoded binding entity contains a sortase-motive and a His-tag. The plasmids are individually transferred into a separate well of a multi-well plate (a whole plate can be loaded). Thereafter, the plasmids are digested with a restriction enzyme mix that cuts out the binding entity-coding region. It is desirable to design all gene synthesis in a way that only one restriction enzyme mix is needed for all plasmids. Afterwards, an optional cleaning step yields purified DNA fragments. These fragments are ligated into a plasmid backbone that had been cut out of an acceptor vector with the same restriction mix as mentioned above. Alternatively, the cloning procedure can be performed by a SLIC-mediated cloning step. After ligation, the automated platforms transfers all ligation mixes into a further multi-well plate with competent E. coli cells (e.g. Top10 Multi Shot, Invitrogen) and a transformation reaction is performed. The cells are cultivated to the desired density. From an aliquot of the cultivation mixture glycerol stocks can be obtained. From the culture plasmid is isolated (e.g. using a plasmid isolation mini kit (e.g. NucleoSpin 96 Plasmid, Macherey& Nagel)). Plasmid identity is checked by digesting an aliquot with an appropriate restriction mix and SDS-gel electrophoresis (e.g. E-Gel 48, Invitrogen). Afterwards, a new plate can be loaded with an aliquot of the plasmid for performing a control sequencing reaction.

### Expression

The antibody Fab fragments were generated by transient transfection of HEK293 cells (human embryonic kidney cell line 293-derived) cultivated in F17 Medium (Invitrogen Corp.). For transfection "293-Fectin" Transfection Reagent (Invitrogen) was used. The antibody Fab fragments were expressed from two different plasmids, coding for a full length light chain and a corresponding truncated heavy chain containing a C-terminal LPXTG sequences (SEQ ID NO: 74). The two plasmids were used at an equimolar plasmid ratio upon transfection. Transfections were performed as specified in the manufacturer's instructions. Antibody Fab fragment-containing cell culture supernatants were harvested seven days after transfection. Supernatants were stored frozen temperature until purification.

The antibody Fab fragment-containing culture supernatants were filtered and purified by two chromatographic steps. The antibody Fab fragments were captured by affinity chromatography using HisTrap HP Ni-NTA columns (GE Healthcare) equilibrated with PBS comprising 20 mM imidazole (1 mM KH₂PO₄, 10 mM Na₂HPO₄, 137 mM NaCl, 2.7 mM KCl, 20 mM imidazole), pH 7.4. Unbound proteins were removed by washing with equilibration buffer. The histidine-tagged protein was eluted with a 20 mM to 400 mM linear imidazole gradient in PBS (1 mM KH₂PO₄ 10 mM Na₂HPO₄, 137 mM NaCl, 2.7 mM KCl, 400 mM Imidazole) in 10 column volumes. Size exclusion chromatography on Superdex 200^{™} (GE Healthcare) was used as second purification step. The size exclusion chromatography was performed in 40 mM Tris-HCl buffer, 0.15 M NaCl, pH 7.5. The antibody Fab fragments were concentrated with an Ultrafree-CL centrifugal filter unit equipped with a Biomax-SK membrane (Millipore, Billerica, MA) and stored at -80 °C.

The protein concentration of the antibody Fab fragments was determined by measuring the optical density (OD) at 280 nm, using the molar extinction coefficient calculated on the basis of the amino acid sequence. Purity and proper antibody Fab formation were analyzed by SDS-PAGE in the presence and absence of a reducing agent (5 mM 1. 4-dithiotreitol) and staining with Coomassie brilliant blue.

### Example 13

### Generation of bispecific binding molecules via the linkage of antibody Fab fragment-oligonucleotide conjugates

Coupling of antibody Fab fragments to oligonucleotides was performed using the enzyme sortase A. Hereby, a molecule with a moiety containing an LPXTG peptide (SEQ ID NO: 74) was covalently attached to another molecule possessing a GG moiety. Therefore, the oligonucleotide had one of the moieties while the antibody Fab fragment had the respective other moiety. The enzymatic reaction can be more advantageous than a chemical reaction because of higher turn-over, higher specificity, less by-products and less hazardous waste.

The reaction of the antibody Fab fragment with the oligonucleotide was performed in the filtrated HEK medium after the expression of the antibody Fab fragment by adding 10x sortase buffer (1x: 50 mM Tris-HCl, 150 mM NaCl, 5 mM CaCl₂, pH 7.5), sortase enzyme (0.15 µg enzyme per µg Fab) and the oligonucleotide in 4-fold molar excess to the antibody Fab fragment. The oligonucleotides consisted of an 18mer L-DNA to which two glycine residues were added through an amino linker and a spacer (5'-(Gly)₄-Aminolinker-(Spacer C3)₃-AG TTC TAT CGT CGT CCA-Fluorescein-3') (SEQ ID NO: 75). The incubation was at 37 °C for 4 - 16 h.

For purification, a (semi-)automated approach was applied. As not all antibody Fab fragments were converted to conjugates, these two populations had to be separated. This was achieved by a negative His-Tag selection, using a His MultiTrap HP 96-well filtration plate (GE Healthcare), which was loaded with Nickel Sepharose. The plate was washed twice with 400 µl water per well and filtrated by vacuum, thereafter equilibrated twice with 400 µl binding buffer per well (25 mM Tris-HCl, 200 mM NaCl, 10 mM imidazole, pH 8.0) and filtrated by vacuum. To the sortase reaction mixture an equal volume of binding buffer was added. The mixture was loaded onto the column and incubated for 5 minutes. Applying vacuum to the column filtrates the conjugate through the column, while the unconjugated antibody Fab fragments remained bound to the column through its His-Tag moiety. As the used sortase was genetically engineered to contain a His-Tag moiety, it was also bound to the column, so that the resulting filtrate is free of the enzyme.

In the next step, free oligonucleotides were removed from the sample, as they would interfere with the subsequent linking reaction. There are two possibilities to achieve this task: Either by ultrafiltration or by an affinity-based approach. For ultrafiltration, those devices are appropriate, that retain the conjugate while they allow the free oligonucleotide to pass the membrane, e.g. Zeba 96-well Spin Desalting Plates, 40K MWCO (Thermo Scientific cat.no. 89807) or AcroPrep 96, 30K (Pall, cat.no. 5035) or through any other comparable ultrafiltration device. After applying the sample and a filtration step, samples are washed three times with linking buffer (PBS). The solution is transferred to a new plate and the volume adjusted to 200 µl. An aliquot is removed for quantitation. As an alternative to ultrafiltration, an affinity-based approach can be applied, requiring a matrix that allows binding of the conjugate, while free oligonucleotide remains unbound. Examples of such a matrix are KappaSelect (GE Healthcare, cat.no. 17-5458-01), CaptoL (GE Healthcare, cat.no. 17-5478-99) or CaptureSelect IgG-CH1 Affinity Matrix (BAC, cat.no. 191.3120.05). The Matrix can be available as columns within a 96well filter plate or it can be bought as a suspension. In the latter case, it can be aliquoted into a 96well filter plate like MSGVN2250 (MultiScreen HTS Millipore cat.no. MSGVN2250) that serves a mounting plate/carrier plate. The affinity matrix can be specific for light chain (as in the case of KappaSelect and CaptoL) or it can be specific for heavy chain (as in the case of Capture Select IgG-CH1). As for each matrix different protocols might be applied, the following outline is based on the protocol for KappaSelect as an example. Plates containing KappaSelect are washed with water, thereafter three times with 400 µl PBS pH 4.0. Afterwards the sample is applied (diluted in PBS pH 4.0) and allowed to bind to the matrix for 90 min with agitation. Three wash steps with 400 µl PBS pH 4.0 are performed, followed by two elution steps with 200 µl elution buffer (0.1 M glycine, 250 mM NaCl, 5 % PEG, pH 2.5). After the elution, 30 µl neutralization buffer (1M Tris-HCl pH 8.0) are added to each of the 200 µl buffer. If necessary, an ultrafiltration step can be performed to bring the sample in another buffer like PBS.

For the generation of bispecific binding molecules two antibody Fab fragment-oligonucleotide conjugates are pipetted together including the linker L-DNA (5'-G CAG AAG CAT TAA TAG ACT- T10-GAC TCT AAG GTA CTC GCA T -T10-TGG ACG ACG ATA GAA CT-3', SEQ ID NO: 76) in equal molar ratios. The linker DNA hybridizes with its 5' end to the first antibody Fab fragment-oligonucleotide conjugate, while its 3' end is complementary to the second antibody Fab fragment-oligonucleotide conjugate, thereby establishing a physical connection between the two different antibody Fab fragment-oligonucleotide conjugates. For proper hybridization, the solution is heated to 60°C and then slowly cooled down to room temperature and thereafter, for storage, to 4°C. For fast protocols, room temperature for a few minutes is also sufficient.

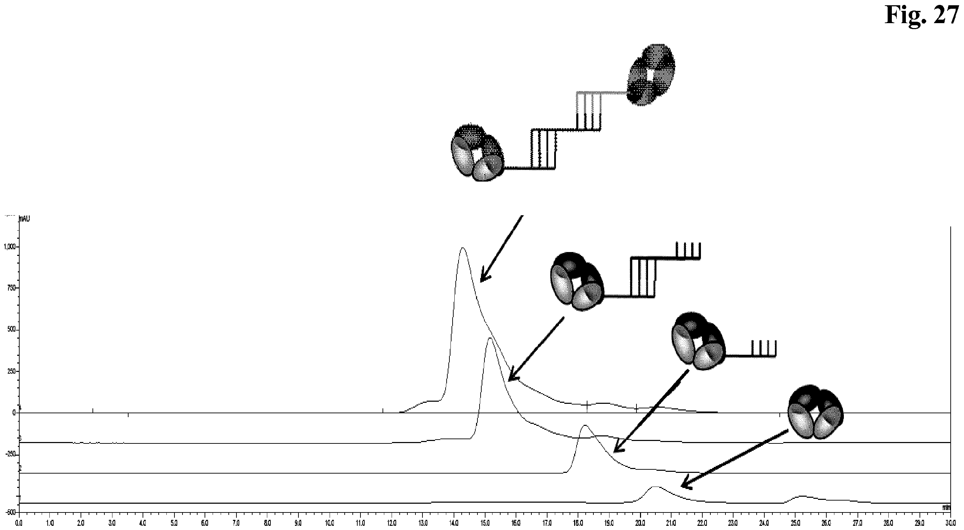

The bispecific binding molecule is purified by preparative size exclusion chromatography. On a Superdex200 column with 2x PBS as running buffer, the protein fractions are separated according to their size. A typical chromatogram (analytical SEC) is shown in Figure 27. In the linking reaction, a high molecular species is formed that is clearly distinguishable from the pure Fab, the conjugate and the intermediate product of one antibody Fab fragment-oligonucleotide conjugate associated with the linker. The fraction containing bispecific binding molecule can be further analyzed in cellular assays.

### Preparative approach

If larger amounts of the bispecific binding molecule are needed or if there are other constraints, a so-called preparative approach can be applied. Hereby, the sortase reaction mixture is applied on a Superdex200 column with 1x PBS as running buffer. Fractions are collected in 0.4 ml volumes. Afterwards aliquots of all relevant fractions are analyzed via the LabChip system (Perkin Elmer) to determine the fractions containing the antibody Fab fragment-oligonucleotide conjugate. Moreover, aliquots of all relevant fractions are loaded on an agarose-gel, whereby a so-called catcher oligonucleotide is added to the sample before it is applied on the gel. This catcher oligonucleotide is complementary to the oligonucleotide of the antibody Fab fragment-oligonucleotide conjugate, thereby resulting in a dsDNA moiety, which can be more easily visualized on the agarose gel than the ssDNA of the antibody Fab fragment-oligonucleotide conjugate alone. Those fractions, that contain the antibody Fab fragment-oligonucleotide conjugate (as seen on the LabChip system) and that do not contain free oligonucleotide (as seen on the agarose gel) are pooled and, if needed, concentrated by ultrafiltration e.g. with Amicon Ultra 0.5ML,10K (Millipore). For the linking reaction two antibody Fab fragment-oligonucleotide conjugate molecules are pipetted together including the linker L-DNA in equal molar ratios and treated as outlined in the previous section.

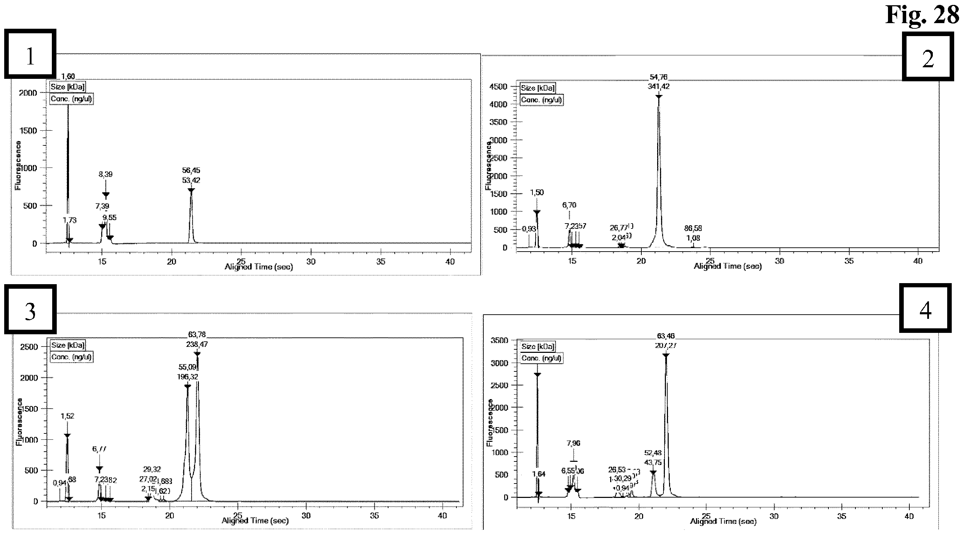

The efficiency of the sortase reaction and of the cleaning process can be monitored with protein gels or with a LabChip system (Perkin Elmer). The latter one delivers electrospherograms with sizing and concentration determination. An example of such a run, in which samples of the different stages of the workflow were analyzed, is shown in Figure 28. Note that peaks lower than 10 kDa are so-called system peaks, which are immanent for the LabChip device and do not belong to the samples. The antibody Fab fragment has a size of 55-56 kDa, depending on the buffer. Figure 28-1 shows the starting material (pure antibody Fab fragment), Figure 28-2 the start of the sortase reaction (note the appearance of the sortase with a mass of 27 kDa). At the end of the sortase reaction (Figure 28-3) a prominent peak after the antibody Fab fragment peak appears at 64 kDa, representing antibody Fab fragment-oligonucleotide conjugate (coupling rate/efficacy about 60 %). After purification by negative His-Tag selection and ultrafiltration an almost pure antibody Fab fragment-oligonucleotide conjugate peak can be seen (Figure 28-4).

## Claims

1. A method for producing a bispecific antibody comprising the following steps
(i) determining surface markers present on the surface of a cell in a sample and selecting thereof a first surface marker and a second surface marker,
(ii) incubating (a) an antibody Fab fragment or a scFv antibody fragment conjugated to a first member of a first binding pair, whereby the Fab fragment or scFv specifically binds to the first surface marker, (b) an antibody Fab fragment or a scFv antibody fragment conjugated to a first member of a second binding pair, whereby the Fab fragment or scFv antibody fragment specifically binds to the second surface marker, and (c) an enantiomeric DNA polynucleotide linker comprising at one of its termini the second member of the first binding pair and at the respective other terminus the second member of the second binding pair,
and thereby producing the bispecific antibody.

2. A method for determining a combination of antigen binding sites comprising the following steps
(i) determining the binding specificity and/or affinity of a multitude of bispecific antibodies prepared by combining each member of a first multitude of antibody Fab fragments or scFv antibody fragments each linked to the same first member of a first binding pair with each member of a second multitude of antibody Fab fragments or scFv antibody fragments each linked to the same first member of a second binding pair, and an enantiomeric DNA polynucleotide linker comprising at one of its termini the second member of the first binding pair and at the respective other terminus the second member of the second binding pair,
whereby the first multitude specifically binds to a first cell surface marker and the second multitude specifically binds to a second cell surface marker,
and
(ii) choosing the bispecific antibody with suitable binding specificity and/or affinity and thereby determining a combination of antigen binding sites.

3. The method according to any one of the preceding claims, **characterized in that** the bispecific antibody is a complex comprising
a) a first Fab fragment or scFv antibody fragment
i) that specifically binds to a first surface marker, and
ii) that is conjugated to a first member of a first binding pair,
b) a second Fab fragment or scFv antibody fragment
i) that specifically binds to a second surface marker, and
ii) that is conjugated to a first member of a second binding pair, and
c) an enantiomeric DNA polynucleotide linker
i) that is conjugated to the second member of the first binding pair, and
ii) that is conjugated to the second member of the second binding pair.

4. The method according to any one of the preceding claims, **characterized in that** the complex is a non-covalent complex.

5. The method according to any one of the preceding claims, **characterized in that** the binding pair is a complementary pair of polynucleotides.

6. The method according to any one of the preceding claims, **characterized in that** the complex further comprises an effector moiety conjugated to a polynucleotide that is complementary to at least a part of the linker.

7. The method according to claim 6, **characterized in that** the complex comprises a second effector moiety conjugated to a polynucleotide that is complementary to at least a part of the polynucleotide that is conjugated to the first effector moiety and not complementary to the polynucleotide linker.

8. The method according to any one of the preceding claims, **characterized in that** the first and second Fab fragment or scFv antibody fragment bind to the same target and to non-overlapping epitopes thereon.

9. The method according to any one of the preceding claims, **characterized in that** the polynucleotide linker comprises of from 8 to 1000 nucleotides.

10. The method according to any one of the preceding claims, **characterized in that** the enantiomeric DNA is L-DNA.

11. The method according to claim 10, **characterized in that** the L-DNA is single stranded L-DNA (ss-L-DNA).

## Patentansprüche

1. Verfahren zur Herstellung eines bispezifischen Antikörpers, umfassend die folgenden Schritte:
(i) Bestimmen von Oberflächenmarkern, die auf der Oberfläche einer Zelle in einer Probe vorhanden sind, und Auswählen daraus eines ersten Oberflächenmarkers und eines zweiten Oberflächenmarkers,
(ii) Inkubieren (a) eines Antikörper-Fab-Fragments oder eines scFv-Antikörper-Fragments, das an einen ersten Partner eines ersten Bindungspaares konjugiert ist, wobei das Fab-Fragment oder scFv-Fragment spezifisch an den ersten Oberflächenmarker bindet, (b) eines Antikörper-Fab-Fragments oder eines scFv-Antikörper-Fragments, das an einen ersten Partner eines zweiten Bindungspaars konjugiert ist, wobei das Fab-Fragment oder scFv-Fragment spezifisch an den zweiten Oberflächenmarker bindet, und (c) eines enantiomeren DNA-Polynucleotid-Linkers, der an einem seiner Enden den zweiten Partner des ersten Bindungspaares und am entsprechenden anderen Ende den zweiten Partner des zweiten Bindungspaares umfasst,
und dadurch Herstellen des bispezifischen Antikörpers.

2. Verfahren zum Bestimmen einer Kombination aus Antigenbindungsstellen, umfassend die folgenden Schritte:
(i) Bestimmen der Bindungsspezifität und/oder -affinität einer Menge an bizspezifischen Antikörpern, die hergestellt werden durch das Kombinieren jeden Mitglieds einer ersten Menge an Antikörper-Fab-Fragmenten oder scFv-Antikörper-Fragmenten, die alle mit dem gleichen ersten Partner eines ersten Bindungspaars verbunden sind, mit jedem Mitglied einer zweiten Menge an Antikörper-Fab-Fragmenten oder scFv-Antikörper-Fragmenten, die alle mit dem gleichen ersten Partner eines zweiten Bindungspaares verbunden sind, und einem enantiomeren DNA-Polynucleotid-Linker, der an einem seiner Enden den zweiten Partner des ersten Bindungspaares und am entsprechenden anderen Ende den zweiten Partner des zweiten Bindungspaares umfasst,
wobei die erste Menge spezifisch an einen ersten Zelloberflächenmarker und die zweite Menge spezifisch an einen zweiten Zelloberflächenmarker bindet, und
(ii) Auswählen eines bispezifischen Antikörpers mit geeigneter Bindungsspezifität und/oder -affinität und dadurch Bestimmen einer Kombination aus Antigenbindungsstellen.

3. Verfahren nach einem der vorangehenden Ansprüche, **dadurch gekennzeichnet, dass** der bispezifische Antikörper ein Komplex ist, der umfasst:
a) ein erstes Fab-Fragment oder scFv-Antikörper-Fragment,
i) das spezifisch an einen ersten Oberflächenmarker bindet, und
ii) das an einen ersten Partner eines ersten Bindungspaares konjugiert ist,
b) ein zweites Fab-Fragment oder scFv-Antikörper-Fragment,
i) das spezifisch an einen zweiten Oberflächenmarker bindet, und
ii) das an einen ersten Partner eines zweiten Bindungspaares konjugiert ist, und
c) einen enantiomeren DNA-Polynucleotid-Linker,
i) der an den zweiten Partner des ersten Bindungspaares konjugiert ist, und
ii) der an den zweiten Partner des zweiten Bindungspaares konjugiert ist.

4. Verfahren nach einem der vorangehenden Ansprüche, **dadurch gekennzeichnet, dass** der Komplex ein nicht-kovalenter Komplex ist.

5. Verfahren nach einem der vorangehenden Ansprüche, **dadurch gekennzeichnet, dass** das Bindungspaar ein komplementäres Polynucleotidpaar ist.

6. Verfahren nach einem der vorangehenden Ansprüche, **dadurch gekennzeichnet, dass** der Komplex des Weiteren einen Effektor-Anteil umfasst, der an ein Polynucleotid konjugiert ist, das zu mindestens zu einem Teil des Linkers komplementär ist.

7. Verfahren nach Anspruch 6, **dadurch gekennzeichnet, dass** der Komplex einen zweiten Effektor-Anteil umfasst, der an ein Polynucleotid konjugiert ist, das zu mindestens zu einem Teil des Polynucleotids komplementär ist, das an den ersten Effektor-Anteil konjugiert ist, und zu dem Polynucleotid-Linker nicht komplementär ist.

8. Verfahren nach einem der vorangehenden Ansprüche, **dadurch gekennzeichnet, dass** das erste und zweite Fab-Fragment oder scFv-Antikörper-Fragment an das gleiche Target und an nicht-überlappende Epitope davon binden.

9. Verfahren nach einem der vorangehenden Ansprüche, **dadurch gekennzeichnet, dass** der Polynucleotid-Linker 8 bis 1000 Nucleotide umfasst.

10. Verfahren nach einem der vorangehenden Ansprüche, **dadurch gekennzeichnet, dass** die enantiomere DNA L-DNA ist.

11. Verfahren nach Anspruch 10, **dadurch gekennzeichnet, dass** die L-DNA einzelsträngige L-DNA (ss-L-DNA) ist.

## Revendications

1. Procédé de production d'un anticorps bispécifique comprenant les étapes suivantes :
(i) détermination de marqueurs de surface présents sur la surface d'une cellule dans un échantillon et sélection d'un premier marqueur de surface et d'un second marqueur de surface de celle-ci,
(ii) incubation (a) d'un fragment Fab d'anticorps ou d'un fragment d'anticorps scFv conjugué à un premier membre d'une première paire de liaison, moyennant quoi le fragment Fab ou scFv se lie spécifiquement au premier marqueur de surface, (b) un fragment Fab d'anticorps ou un fragment d'anticorps scFv conjugué à un premier membre d'une seconde paire de liaison, moyennant quoi le fragment Fab ou le fragment d'anticorps scFv se lie spécifiquement au second marqueur de surface, et (c) un lieur polynucléotidique d'ADN énantiomère comprenant à une de ses extrémités le second membre de la première paire de liaison et à l'autre extrémité respective le second membre de la seconde paire de liaison,
et produisant ainsi l'anticorps bispécifique.

2. Procédé de détermination d'une combinaison de sites de liaison d'antigène comprenant les étapes suivantes :
(i) détermination de la spécificité de liaison et/ou de l'affinité d'une multitude d'anticorps bispécifiques préparés en combinant chaque membre d'une première multitude de fragments Fab d'anticorps ou de fragments d'anticorps scFv chacun lié au même premier membre d'une première paire de liaison avec chaque membre d'une seconde multitude de fragments Fab d'anticorps ou de fragments d'anticorps scFv chacun lié au même premier membre d'une seconde paire de liaison, et un lieur polynucléotidique d'ADN énantiomère comprenant à une de ses extrémités le second membre de la première paire de liaison et à l'autre extrémité respective le second membre de la seconde paire de liaison,
moyennant quoi la première multitude se lie spécifiquement à un premier marqueur de surface cellulaire et la seconde multitude se lie spécifiquement à un second marqueur de surface cellulaire,
et
(ii) choix de l'anticorps bispécifique avec la spécificité de liaison et/ou l'affinité appropriée et déterminant ainsi une combinaison de sites de liaison d'antigène.

3. Procédé selon l'une quelconque des revendications précédentes, **caractérisé en ce que** l'anticorps bispécifique est un complexe comprenant :
a) un premier fragment Fab ou fragment d'anticorps scFv
i) qui se lie spécifiquement à un premier marqueur de surface, et
ii) qui est conjugué à un premier membre d'une première paire de liaison,
b) un second fragment Fab ou fragment d'anticorps scFv
i) qui se lie spécifiquement à un second marqueur de surface, et
ii) qui est conjugué à un premier membre d'une seconde paire de liaison, et
c) un lieur polynucléotidique d'ADN énantiomère
i) qui est conjugué au second membre de la première paire de liaison, et
ii) qui est conjugué au second membre de la seconde paire de liaison.

4. Procédé selon l'une quelconque des revendications précédentes, **caractérisé en ce que** le complexe est un complexe non covalent.

5. Procédé selon l'une quelconque des revendications précédentes, **caractérisé en ce que** la paire de liaison est une paire complémentaire de polynucléotides.

6. Procédé selon l'une quelconque des revendications précédentes, **caractérisé en ce que** le complexe comprend en outre un groupement effecteur conjugué à un polynucléotide qui est complémentaire à au moins une partie du lieur.

7. Procédé selon la revendication 6, **caractérisé en ce que** le complexe comprend un second groupement effecteur conjugué à un polynucléotide qui est complémentaire à au moins une partie du polynucléotide qui est conjugué au premier groupement effecteur et non complémentaire au lieur polynucléotidique.

8. Procédé selon l'une quelconque des revendications précédentes, **caractérisé en ce que** les premier et second fragments Fab ou fragments d'anticorps scFv se lient à la même cible et à des épitopes non chevauchants dessus.

9. Procédé selon l'une quelconque des revendications précédentes, **caractérisé en ce que** le lieur polynucléotidique comprend 8 à 1000 nucléotides.

10. Procédé selon l'une quelconque des revendications précédentes, **caractérisé en ce que** l'ADN énantiomère est un L-ADN.

11. Procédé selon la revendication 10, **caractérisé en ce que** le L-ADN est un L-ADN simple brin (L-ADN-sb).


### SEQUENCE LISTING

<110> F. Hoffmann-La Roche AG
<120> Method for the selection and production of tailor-made, selective and multi-specific therapeutic molecules comprising at least two different targeting entities und uses thereof
<130> 31067 WO
<150> EP12173878.5
   <151> 2012-06-27
<160> 80
<170> PatentIn version 3.5
<210> 1
   <211> 122
   <212> PRT
   <213> Artificial Sequence
<220>
   <223> VH (mAb 1.4.168)
<400> 1
   Gln Cys Asp Val Lys Leu Val Glu Ser Gly Gly Gly Leu Val Lys Pro
   1               5                   10                  15
   Gly Gly Ser Leu Lys Leu Ser Cys Ala Ala Ser Gly Phe Thr Phe Ser
               20                  25                  30
   Asp Tyr Pro Met Ser Trp Val Arg Gln Thr Pro Glu Lys Arg Leu Glu
           35                  40                  45
   Trp Val Ala Thr Ile Thr Thr Gly Gly Thr Tyr Thr Tyr Tyr Pro Asp
       50                  55                  60
   Ser Ile Lys Gly Arg Phe Thr Ile Ser Arg Asp Asn Ala Lys Asn Thr
   65                  70                  75                  80
   Leu Tyr Leu Gln Met Gly Ser Leu Gln Ser Glu Asp Ala Ala Met Tyr
                   85                  90                  95
   Tyr Cys Thr Arg Val Lys Thr Asp Leu Trp Trp Gly Leu Ala Tyr Trp
               100                 105                 110
   Gly Gln Gly Thr Leu Val Thr Val Ser Ala
           115                 120
<210> 2
   <211> 116
   <212> PRT
   <213> Artificial Sequence
<220>
   <223> VL (mAb 1.4.168)
<400> 2
   Gln Leu Val Leu Thr Gln Ser Ser Ser Ala Ser Phe Ser Leu Gly Ala
   1               5                   10                  15
   Ser Ala Lys Leu Thr Cys Thr Leu Ser Ser Gln His Ser Thr Tyr Thr
               20                  25                  30
   Ile Glu Trp Tyr Gln Gln Gln Pro Leu Lys Pro Pro Lys Tyr Val Met
           35                  40                  45
   Glu Leu Lys Lys Asp Gly Ser His Thr Thr Gly Asp Gly Ile Pro Asp
       50                  55                  60
   Arg Phe Ser Gly Ser Ser Ser Gly Ala Asp Arg Tyr Leu Ser Ile Ser
   65                  70                  75                  80
   Asn Ile Gln Pro Glu Asp Glu Ser Ile Tyr Ile Cys Gly Val Gly Asp
                   85                  90                  95
   Thr Ile Lys Glu Gln Phe Val Tyr Val Phe Gly Gly Gly Thr Lys Val
               100                 105                 110
   Thr Val Leu Gly
           115
<210> 3
   <211> 121
   <212> PRT
   <213> Artificial Sequence
<220>
   <223> VH (mAb 8.1.2)
<400> 3
   Glu Val Gln Leu Gln Gln Ser Gly Pro Ala Leu Val Lys Pro Gly Ala
   1               5                   10                  15
   Ser Val Lys Met Ser Cys Lys Ala Ser Gly Phe Thr Phe Thr Ser Tyr
               20                  25                  30
   Val Ile His Trp Val Lys Gln Lys Pro Gly Gln Gly Leu Glu Trp Ile
           35                  40                  45
   Gly Tyr Leu Asn Pro Tyr Asn Asp Asn Thr Lys Tyr Asn Glu Lys Phe
       50                  55                  60
   Lys Gly Lys Ala Thr Leu Thr Ser Asp Arg Ser Ser Ser Thr Val Tyr
   65                  70                  75                  80
   Met Glu Phe Ser Ser Leu Thr Ser Glu Asp Ser Ala Val Tyr Phe Cys
                   85                  90                  95
   Ala Arg Arg Gly Ile Tyr Ala Tyr Asp His Tyr Phe Asp Tyr Trp Gly
               100                 105                 110
   Gln Gly Thr Ser Leu Thr Val Ser Ser
           115                 120
<210> 4
   <211> 106
   <212> PRT
   <213> Artificial Sequence
<220>
   <223> VL (mAb 8.1.2)
<400> 4
   Gln Ile Val Leu Thr Gln Ser Pro Ala Ile Met Ser Ala Ser Pro Gly
   1               5                   10                  15
   Glu Lys Val Thr Leu Thr Cys Ser Ala Ser Ser Ser Val Asn Tyr Met
               20                  25                  30
   Tyr Trp Tyr Gln Gln Lys Pro Gly Ser Ser Pro Arg Leu Leu Ile Tyr
           35                  40                  45
   Asp Thr Ser Asn Leu Ala Ser Gly Val Pro Val Arg Phe Ser Gly Ser
       50                  55                  60
   Gly Ser Val Thr Ser Tyr Ser Leu Thr Ile Ser Arg Met Glu Ala Glu
   65                  70                  75                  80
   Asp Ala Ala Thr Tyr Tyr Cys Gln Gln Trp Ser Thr Tyr Pro Leu Thr
                   85                  90                  95
   Phe Gly Ala Gly Thr Lys Leu Glu Leu Lys
               100                 105
<210> 5
   <211> 17
   <212> DNA
   <213> Artificial Sequence
<220>
   <223> 17mer ssDNA (covalently bound with 5' end to Fab')
<400> 5
   agttctatcg tcgtcca 17
<210> 6
   <211> 19
   <212> DNA
   <213> Artificial Sequence
<220>
   <223> 19mer ssDNA (covalently bound with 3' end to Fab')
<400> 6
   agtctattaa tgcttctgc 19
<210> 7
   <211> 19
   <212> DNA
   <213> Artificial Sequence
<220>
   <223> complementary 19mer ssDNA (used as part of a linker)
<400> 7
   gcagaagcat taatagact 19
<210> 8
   <211> 17
   <212> DNA
   <213> Artificial Sequence
<220>
   <223> complementary 17mer ssDNA (used as part of a linker)
<400> 8
   tggacgacga tagaact 17
<210> 9
   <211> 33
   <212> PRT
   <213> Artificial Sequence
<220>
   <223> fragment
<220>
   <221> MISC_FEATURE
   <222> (16)..(17)
   <223> beta-alanine
<400> 9
   Glu Arg Ala Glu Gln Gln Arg Ile Arg Ala Glu Arg Glu Lys Glu Xaa
   1               5                   10                  15
   Xaa Ser Leu Lys Asp Arg Ile Glu Lys Arg Arg Arg Ala Glu Arg Ala
               20                  25                  30
   Glu
<210> 10
   <211> 31
   <212> PRT
   <213> Artificial Sequence
<220>
   <223> fragment
<220>
   <221> MISC_FEATURE
   <222> (15)..(16)
   <223> amino-trioxa-octanoic-acid
<400> 10
   Leu Lys Asp Arg Ile Glu Arg Arg Arg Ala Glu Arg Ala Glu Xaa Xaa
   1               5                   10                  15
   Glu Arg Ala Glu Gln Gln Arg Ile Arg Ala Glu Arg Glu Lys Glu
               20                  25                  30
<210> 11
   <211> 27
   <212> PRT
   <213> Artificial Sequence
<220>
   <223> IGF-1R (1340-1366)
<400> 11
   Phe Asp Glu Arg Gln Pro Tyr Ala His Met Asn Gly Gly Arg Lys Asn
   1               5                   10                  15
   Glu Arg Ala Leu Pro Leu Pro Gln Ser Ser Thr
               20                  25
<210> 12
   <211> 27
   <212> PRT
   <213> Artificial Sequence
<220>
   <223> hInsR(1355-1382)
<400> 12
   Tyr Glu Glu His Ile Pro Tyr Thr His Met Asn Gly Gly Lys Lys Asn
   1               5                   10                  15
   Gly Arg Ile Leu Thr Leu Pro Arg Ser Asn Pro
               20                  25
<210> 13
   <211> 35
   <212> DNA
   <213> Artificial Sequence
<220>
   <223> 35-mer polynucleotide linker
<220>
   <221> misc_feature
   <222> (1)..(35)
   <223> n is a, c, g, or t
<400> 13
   nnnnnnnnnn nnnnnnnnnn nnnnnnnnnn nnnnn 35
<210> 14
   <211> 75
   <212> DNA
   <213> Artificial Sequence
<220>
   <223> 75-mer polynucleotide linker
<220>
   <221> misc_feature
   <222> (1)..(75)
   <223> n is a, c, g, or t
<400> 14
   nnnnnnnnnn nnnnnnnnnn nnnnnnnnnn nnnnnnnnnn nnnnnnnnnn nnnnnnnnnn 60
   nnnnnnnnnn nnnnn 75
<210> 15
   <211> 95
   <212> DNA
   <213> Artificial Sequence
<220>
   <223> 95-mer polynucleotide linker
<220>
   <221> misc_feature
   <222> (1)..(95)
   <223> n is a, c, g, or t
<400> 15
   nnnnnnnnnn nnnnnnnnnn nnnnnnnnnn nnnnnnnnnn nnnnnnnnnn nnnnnnnnnn 60
   nnnnnnnnnn nnnnnnnnnn nnnnnnnnnn nnnnn 95
<210> 16
   <211> 218
   <212> PRT
   <213> Artificial Sequence
<220>
   <223> 4D5 Fab' heavy chain amino acid sequence
<400> 16
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Asn Ile Lys Asp Thr
               20                  25                  30
   Tyr Ile His Trp Val Arg Gln Ala Pro Gly Lys Gly Leu Glu Trp Val
           35                  40                  45
   Ala Arg Ile Tyr Pro Thr Asn Gly Tyr Thr Arg Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ser Arg Trp Gly Gly Asp Gly Phe Tyr Ala Met Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val
       210                 215
<210> 17
   <211> 214
   <212> PRT
   <213> Artificial Sequence
<220>
   <223> 4D5 Fab' light chain amino acid sequence
<400> 17
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Asn Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Arg Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Thr Thr Pro Pro
                   85                  90                  95
   Thr Phe Gly Gln Gly Thr Lys Val Glu Ile Lys Arg Thr Gln Pro Lys
               100                 105                 110
   Ala Ala Pro Ser Val Thr Leu Phe Pro Pro Ser Ser Glu Glu Leu Gln
           115                 120                 125
   Ala Asn Lys Ala Thr Leu Val Cys Leu Ile Ser Asp Phe Tyr Pro Gly
       130                 135                 140
   Ala Val Thr Val Ala Trp Lys Ala Asp Ser Ser Pro Val Lys Ala Gly
   145                 150                 155                 160
   Val Glu Thr Thr Thr Pro Ser Lys Gln Ser Asn Asn Lys Tyr Ala Ala
                   165                 170                 175
   Ser Ser Tyr Leu Ser Leu Thr Pro Glu Gln Trp Lys Ser His Arg Ser
               180                 185                 190
   Tyr Ser Cys Gln Val Thr His Glu Gly Ser Thr Val Glu Lys Thr Val
           195                 200                 205
   Ala Pro Thr Glu Cys Ser
                               210
<210> 18
   <211> 217
   <212> PRT
   <213> Artificial Sequence
<220>
   <223> 2C4 Fab' heavy chain amino acid sequence
<400> 18
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Thr Asp Tyr
               20                  25                  30
   Thr Met Asp Trp Val Arg Gln Ala Pro Gly Lys Gly Leu Glu Trp Val
           35                  40                  45
   Ala Asp Val Asn Pro Asn Ser Gly Gly Ser Ile Tyr Asn Gln Arg Phe
       50                  55                  60
   Lys Gly Arg Phe Thr Leu Ser Val Asp Arg Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asn Leu Gly Pro Ser Phe Tyr Phe Asp Tyr Trp Gly Gln Gly
               100                 105                 110
   Thr Leu Val Thr Val Ser Ser Ala Ser Thr Lys Gly Pro Ser Val Phe
           115                 120                 125
   Pro Leu Ala Pro Ser Ser Lys Ser Thr Ser Gly Gly Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Ile Cys Asn Val Asn His Lys Pro
           195                 200                 205
   Ser Asn Thr Lys Val Asp Lys Lys Val
       210                 215
<210> 19
   <211> 212
   <212> PRT
   <213> Artificial Sequence
<220>
   <223> 2C4 Fab' light chain amino acid sequence
<400> 19
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Val Ser Ile Gly
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Tyr Thr Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Tyr Ile Tyr Pro Tyr
                   85                  90                  95
   Thr Phe Gly Gln Gly Thr Lys Val Glu Ile Lys Gln Pro Lys Ala Ala
               100                 105                 110
   Pro Ser Val Thr Leu Phe Pro Pro Ser Ser Glu Glu Leu Gln Ala Asn
           115                 120                 125
   Lys Ala Thr Leu Val Cys Leu Ile Ser Asp Phe Tyr Pro Gly Ala Val
       130                 135                 140
   Thr Val Ala Trp Lys Ala Asp Ser Ser Pro Val Lys Ala Gly Val Glu
   145                 150                 155                 160
   Thr Thr Thr Pro Ser Lys Gln Ser Asn Asn Lys Tyr Ala Ala Ser Ser
                   165                 170                 175
   Tyr Leu Ser Leu Thr Pro Glu Gln Trp Lys Ser His Arg Ser Tyr Ser
               180                 185                 190
   Cys Gln Val Thr His Glu Gly Ser Thr Val Glu Lys Thr Val Ala Pro
           195                 200                 205
   Thr Glu Cys Ser
       210
<210> 20
   <211> 645
   <212> PRT
   <213> Artificial Sequence
<220>
   <223> HER2 ECD fragment
<400> 20
   Met Glu Leu Ala Ala Leu Cys Arg Trp Gly Leu Leu Leu Ala Leu Leu
   1               5                   10                  15
   Pro Pro Gly Ala Ala Ser Thr Gln Val Cys Thr Gly Thr Asp Met Lys
               20                  25                  30
   Leu Arg Leu Pro Ala Ser Pro Glu Thr His Leu Asp Met Leu Arg His
           35                  40                  45
   Leu Tyr Gln Gly Cys Gln Val Val Gln Gly Asn Leu Glu Leu Thr Tyr
       50                  55                  60
   Leu Pro Thr Asn Ala Ser Leu Ser Phe Leu Gln Asp Ile Gln Glu Val
   65                  70                  75                  80
   Gln Gly Tyr Val Leu Ile Ala His Asn Gln Val Arg Gln Val Pro Leu
                   85                  90                  95
   Gln Arg Leu Arg Ile Val Arg Gly Thr Gln Leu Phe Glu Asp Asn Tyr
               100                 105                 110
   Ala Leu Ala Val Leu Asp Asn Gly Asp Pro Leu Asn Asn Thr Thr Pro
           115                 120                 125
   Val Thr Gly Ala Ser Pro Gly Gly Leu Arg Glu Leu Gln Leu Arg Ser
       130                 135                 140
   Leu Thr Glu Ile Leu Lys Gly Gly Val Leu Ile Gln Arg Asn Pro Gln
   145                 150                 155                 160
   Leu Cys Tyr Gln Asp Thr Ile Leu Trp Lys Asp Ile Phe His Lys Asn
                   165                 170                 175
   Asn Gln Leu Ala Leu Thr Leu Ile Asp Thr Asn Arg Ser Arg Ala Cys
               180                 185                 190
   His Pro Cys Ser Pro Met Cys Lys Gly Ser Arg Cys Trp Gly Glu Ser
           195                 200                 205
   Ser Glu Asp Cys Gln Ser Leu Thr Arg Thr Val Cys Ala Gly Gly Cys
       210                 215                 220
   Ala Arg Cys Lys Gly Pro Leu Pro Thr Asp Cys Cys His Glu Gln Cys
   225                 230                 235                 240
   Ala Ala Gly Cys Thr Gly Pro Lys His Ser Asp Cys Leu Ala Cys Leu
                   245                 250                 255
   His Phe Asn His Ser Gly Ile Cys Glu Leu His Cys Pro Ala Leu Val
               260                 265                 270
   Thr Tyr Asn Thr Asp Thr Phe Glu Ser Met Pro Asn Pro Glu Gly Arg
           275                 280                 285
   Tyr Thr Phe Gly Ala Ser Cys Val Thr Ala Cys Pro Tyr Asn Tyr Leu
       290                 295                 300
   Ser Thr Asp Val Gly Ser Cys Thr Leu Val Cys Pro Leu His Asn Gln
   305                 310                 315                 320
   Glu Val Thr Ala Glu Asp Gly Thr Gln Arg Cys Glu Lys Cys Ser Lys
                   325                 330                 335
   Pro Cys Ala Arg Val Cys Tyr Gly Leu Gly Met Glu His Leu Arg Glu
               340                 345                 350
   Val Arg Ala Val Thr Ser Ala Asn Ile Gln Glu Phe Ala Gly Cys Lys
           355                 360                 365
   Lys Ile Phe Gly Ser Leu Ala Phe Leu Pro Glu Ser Phe Asp Gly Asp
       370                 375                 380
   Pro Ala Ser Asn Thr Ala Pro Leu Gln Pro Glu Gln Leu Gln Val Phe
   385                 390                 395                 400
   Glu Thr Leu Glu Glu Ile Thr Gly Tyr Leu Tyr Ile Ser Ala Trp Pro
                   405                 410                 415
   Asp Ser Leu Pro Asp Leu Ser Val Phe Gln Asn Leu Gln Val Ile Arg
               420                 425                 430
   Gly Arg Ile Leu His Asn Gly Ala Tyr Ser Leu Thr Leu Gln Gly Leu
           435                 440                 445
   Gly Ile Ser Trp Leu Gly Leu Arg Ser Leu Arg Glu Leu Gly Ser Gly
       450                 455                 460
   Leu Ala Leu Ile His His Asn Thr His Leu Cys Phe Val His Thr Val
   465                 470                 475                 480
   Pro Trp Asp Gln Leu Phe Arg Asn Pro His Gln Ala Leu Leu His Thr
                   485                 490                 495
   Ala Asn Arg Pro Glu Asp Glu Cys Val Gly Glu Gly Leu Ala Cys His
               500                 505                 510
   Gln Leu Cys Ala Arg Gly His Cys Trp Gly Pro Gly Pro Thr Gln Cys
           515                 520                 525
   Val Asn Cys Ser Gln Phe Leu Arg Gly Gln Glu Cys Val Glu Glu Cys
       530                 535                 540
   Arg Val Leu Gln Gly Leu Pro Arg Glu Tyr Val Asn Ala Arg His Cys
   545                 550                 555                 560
   Leu Pro Cys His Pro Glu Cys Gln Pro Gln Asn Gly Ser Val Thr Cys
                   565                 570                 575
   Phe Gly Pro Glu Ala Asp Gln Cys Val Ala Cys Ala His Tyr Lys Asp
               580                 585                 590
   Pro Pro Phe Cys Val Ala Arg Cys Pro Ser Gly Val Lys Pro Asp Leu
           595                 600                 605
   Ser Tyr Met Pro Ile Trp Lys Phe Pro Asp Glu Glu Gly Ala Cys Gln
       610                 615                 620
   Pro Cys Pro Ile Asn Cys Thr His Ser Cys Val Asp Leu Asp Asp Lys
   625                 630                 635                 640
   Gly Cys Pro Ala Glu
                   645
<210> 21
   <211> 24
   <212> DNA
   <213> Artificial Sequence
<220>
   <223> fragment
<220>
   <221> misc_feature
   <223> X = propylene-phosphate introduced via phosphoramidite C3 (3-(4,4'-dimethoxytrityloxy)propyl-1-[(2-cyanoethyl)-(N,N-diisopr opyl)]-phosphoramidite
<220>
   <221> misc_feature
   <223> Y = 5'-amino-modifier C6 introduced via (6-(4-monomethoxytritylamino)hexyl-(2-cyanoethyl)-(N,N-diisopropy 1)-phosphoramidite
<220>
   <221> misc_feature
   <223> Z = 4[N-maleinimidomethyl]cyclohexane-1-carboxy introduced via Sulfosuccinimidyl 4-[N-maleimidomethyl]cyclohexane-1-carboxylate
<220>
   <221> misc_feature
   <222> (20)..(24)
   <223> n is a, c, g, or t
<400> 21
   agtctattaa tgcttctgcn nnnn 24
<210> 22
   <211> 22
   <212> DNA
   <213> Artificial Sequence
<220>
   <223> fragment
<220>
   <221> misc_feature
   <223> X = propylene-phosphate introduced via Phosphoramidite C3 (3-(4,4'-Dimethoxytrityloxy)propyl-1-[(2-cyanoethyl)-(N,N-diisopr opyl)]-phosphoramidite
<220>
   <221> misc_feature
   <223> Y = 5'-Amino-Modifier C6 introduced via (6-(4-Monomethoxytritylamino)hexyl-(2-cyanoethyl)-(N,N-diisopropy 1)-phosphoramidite
<220>
   <221> misc_feature
   <223> Z = 4[N-maleinimidomethyl]cyclohexane-1-carboxy introduced via Sulfosuccinimidyl 4-[N-maleimidomethyl]cyclohexane-1-carboxylate
<220>
   <221> misc_feature
   <222> (1)..(5)
   <223> n is a, c, g, or t
<400> 22
   nnnnnagttc tatcgtcgtc ca 22
<210> 23
   <211> 36
   <212> DNA
   <213> Artificial Sequence
<220>
   <223> biotinylated ssDNA linker 1
<220>
   <221> misc_feature
   <223> n=biotin dT
<220>
   <221> misc_feature
   <222> (20)..(20)
   <223> n is a, c, g, or t
<400> 23
   gcagaagcat taatagactn ggacgacgat agaact 36
<210> 24
   <211> 46
   <212> DNA
   <213> Artificial Sequence
<220>
   <223> biotinylated ssDNA linker 2
<220>
   <221> misc_feature
   <223> n=biotin dT
<220>
   <221> misc_feature
   <222> (25) .. (25)
   <223> n is a, c, g, or t
<400> 24
   gcagaagcat taatagactt ttttnttttt ggacgacgat agaact 46
<210> 25
   <211> 66
   <212> DNA
   <213> Artificial Sequence
<220>
   <223> biotinylated ssDNA linker 3
<220>
   <221> misc_feature
   <223> n=biotin dT
<220>
   <221> misc_feature
   <222> (35)..(35)
   <223> n is a, c, g, or t
<400> 25
   gcagaagcat taatagactt tttttttttt ttttnttttt tttttttttt ggacgacgat 60
   agaact 66
<210> 26
   <211> 120
   <212> PRT
   <213> Artificial Sequence
<220>
   <223> anti-HER2 antibody 4D5 heavy chain variable domain
<400> 26
<210> 27
   <211> 10
   <212> PRT
   <213> Artificial Sequence
<220>
   <223> VH CDR1
<400> 27
   Gly Phe Asn Ile Lys Asp Thr Tyr Ile His
   1               5                   10
<210> 28
   <211> 17
   <212> PRT
   <213> Artificial Sequence
<220>
   <223> VH CDR2
<400> 28
   Arg Ile Tyr Pro Thr Asn Gly Tyr Thr Arg Tyr Ala Asp Ser Val Lys
   1               5                   10                  15
   Gly
<210> 29
   <211> 11
   <212> PRT
   <213> Artificial Sequence
<220>
   <223> VH CDR3
<400> 29
   Trp Gly Gly Asp Gly Phe Tyr Ala Met Asp Tyr
   1               5                   10
<210> 30
   <211> 109
   <212> PRT
   <213> Artificial Sequence
<220>
   <223> anti-HER2 antibody 4D5 heavy light variable domain
<400> 30
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Asn Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Arg Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Thr Thr Pro Pro
                   85                  90                  95
   Thr Phe Gly Gln Gly Thr Lys Val Glu Ile Lys Arg Thr
               100                 105
<210> 31
   <211> 12
   <212> PRT
   <213> Artificial Sequence
<220>
   <223> VL CDR1
<400> 31
   Arg Ala Ser Gln Asp Val Asn Thr Ala Val Ala Trp
   1               5                   10
<210> 32
   <211> 7
   <212> PRT
   <213> Artificial Sequence
<220>
   <223> VL CDR2
<400> 32
   Ser Ala Ser Phe Leu Tyr Ser
   1               5
<210> 33
   <211> 9
   <212> PRT
   <213> Artificial Sequence
<220>
   <223> VL CDR3
<400> 33
   Gln Gln His Tyr Thr Thr Pro Pro Thr
   1 5
<210> 34
   <211> 119
   <212> PRT
   <213> Artificial Sequence
<220>
   <223> anti-HER2 antibody 2C4 heavy chain variable domain
<400> 34
<210> 35
   <211> 10
   <212> PRT
   <213> Artificial Sequence
<220>
   <223> VH CDR1
<400> 35
   Gly Phe Thr Phe Thr Asp Tyr Thr Met Asp
   1 5 10
<210> 36
   <211> 17
   <212> PRT
   <213> Artificial Sequence
<220>
   <223> VH CDR2
<400> 36
   Asp Val Asn Pro Asn Ser Gly Gly Ser Ile Tyr Asn Gln Arg Phe Lys
   1               5                   10                  15
   Gly
<210> 37
   <211> 10
   <212> PRT
   <213> Artificial Sequence
<220>
   <223> VH CDR3
<400> 37
   Asn Leu Gly Pro Ser Phe Tyr Phe Asp Tyr
   1               5                   10
<210> 38
   <211> 107
   <212> PRT
   <213> Artificial Sequence
<220>
   <223> anti-HER2 antibody 2C4 light chain variable domain
<400> 38
<210> 39
   <211> 11
   <212> PRT
   <213> Artificial Sequence
<220>
   <223> VL CDR1
<400> 39
   Lys Ala Ser Gln Asp Val Ser Ile Gly Val Ala
   1               5                   10
<210> 40
   <211> 7
   <212> PRT
   <213> Artificial Sequence
<220>
   <223> VL CDR2
<400> 40
   Ser Ala Ser Tyr Arg Tyr Thr
   1               5
<210> 41
   <211> 9
   <212> PRT
   <213> Artificial Sequence
<220>
   <223> VL CDR3
<400> 41
   Gln Gln Tyr Tyr Ile Tyr Pro Tyr Thr
   1               5
<210> 42
   <211> 24
   <212> DNA
   <213> Artificial Sequence
<220>
   <223> amino modified polynucleotide linker precursors
<220>
   <221> misc_feature
   <223> X= Fluorescein Y=C7Aminolinker Z=C3 spacer
<220>
   <221> misc_feature
   <222> (1)..(1)
   <223> n is a, c, g, or t
<220>
   <221> misc_feature
   <222> (21)..(24)
   <223> n is a, c, g, or t
<400> 42
   nagtctatta atgcttctgc nnnn 24
<210> 43
   <211> 24
   <212> DNA
   <213> Artificial Sequence
<220>
   <223> amino modified polynucleotide linker precursors
<220>
   <221> misc_feature
   <223> X=Cy5 Y=C7Aminolinker Z=C3 spacer
<220>
   <221> misc_feature
   <222> (1)..(1)
   <223> n is a, c, g, or t
<220>
   <221> misc_feature
   <222> (21)..(24)
   <223> n is a, c, g, or t
<400> 43
   nagtctatta atgcttctgc nnnn 24
<210> 44
   <211> 22
   <212> DNA
   <213> Artificial Sequence
<220>
   <223> amino modified polynucleotide linker precursors
<220>
   <221> misc_feature
   <223> X=Aminolinker Y= Fluorescein Z=C3 spacer
<220>
   <221> misc_feature
   <222> (1)..(4)
   <223> n is a, c, g, or t
<220>
   <221> misc_feature
   <222> (22)..(22)
   <223> n is a, c, g, or t
<400> 44
   nnnnagttct atcgtcgtcc an 22
<210> 45
   <211> 24
   <212> DNA
   <213> Artificial Sequence
<220>
   <223> amino modified L-polynucleotide linker precursors
<220>
   <221> misc_feature
   <223> X=Fluorescein Y= C7Aminolinker Z=C3 spacer
<220>
   <221> misc_feature
   <222> (1)..(1)
   <223> n is a, c, g, or t
<220>
   <221> misc_feature
   <222> (21)..(24)
   <223> n is a, c, g, or t
<400> 45
   nagtctatta atgcttctgc nnnn 24
<210> 46
   <211> 22
   <212> DNA
   <213> Artificial Sequence
<220>
   <223> amino modified L-polynucleotide linker precursors
<220>
   <221> misc_feature
   <223> X= Aminolinker Y= Fluorescein Z=C3 spacer
<220>
   <221> misc_feature
   <222> (1)..(4)
   <223> n is a, c, g, or t
<220>
   <221> misc_feature
   <222> (22)..(22)
   <223> n is a, c, g, or t
<400> 46
   nnnnagttct atcgtcgtcc an 22
<210> 47
   <211> 36
   <212> DNA
   <213> Artificial Sequence
<220>
   <223> ssDNA linker 1
<400> 47
   gcagaagcat taatagactt ggacgacgat agaact 36
<210> 48
   <211> 76
   <212> DNA
   <213> Artificial Sequence
<220>
   <223> ssDNA linker 2
<400> 48
   gcagaagcat taatagactt tttttttttt tttttttttt tttttttttt tttttttttt 60
   ggacgacgat agaact 76
<210> 49
   <211> 37
   <212> DNA
   <213> Artificial Sequence
<220>
   <223> biotinylated ss-L-DNA linker 3
<220>
   <221> misc_feature
   <223> n=biotin dT
<220>
   <221> misc_feature
   <222> (20)..(20)
   <223> n is a, c, g, or t
<400> 49
   gcagaagcat taatagactn tggacgacga tagaact 37
<210> 50
   <211> 47
   <212> DNA
   <213> Artificial Sequence
<220>
   <223> biotinylated ss-L-DNA linker 4
<220>
   <221> misc_feature
   <223> n=biotin dT
<220>
   <221> misc_feature
   <222> (25)..(25)
   <223> n is a, c, g, or t
<400> 50
   gcagaagcat taatagactt ttttnttttt tggacgacga tagaact 47
<210> 51
   <211> 77
   <212> DNA
   <213> Artificial Sequence
<220>
   <223> biotinylated ss-L-DNA linker 5
<220>
   <221> misc_feature
   <223> n=biotin dT
<220>
   <221> misc_feature
   <222> (40)..(40)
   <223> n is a, c, g, or t
<400> 51
   gcagaagcat taatagactt tttttttttt tttttttttn tttttttttt tttttttttt 60
   tggacgacga tagaact 77
<210> 52
   <211> 97
   <212> DNA
   <213> Artificial Sequence
<220>
   <223> biotinylated ss-L-DNA linker 6
<220>
   <221> misc_feature
   <223> n=biotin dT
<220>
   <221> misc_feature
   <222> (50)..(50)
   <223> n is a, c, g, or t
<400> 52
   gcagaagcat taatagactt tttttttttt tttttttttt tttttttttn tttttttttt 60
   tttttttttt tttttttttt tggacgacga tagaact 97
<210> 53
   <211> 47
   <212> DNA
   <213> Artificial Sequence
<220>
   <223> biotinylated ssDNA linker 7
<220>
   <221> misc_feature
   <223> n=biotin dT
<220>
   <221> misc_feature
   <222> (25)..(25)
   <223> n is a, c, g, or t
<400> 53
   gcagaagcat taatagactt ttttnttttt tggacgacga tagaact 47
<210> 54
   <211> 57
   <212> DNA
   <213> Artificial Sequence
<220>
   <223> biotinylated ssDNA linker 8
<220>
   <221> misc_feature
   <223> n= biotin dT
<220>
   <221> misc_feature
   <222> (30)..(30)
   <223> n is a, c, g, or t
<400> 54
   gcagaagcat taatagactt tttttttttn tttttttttt tggacgacga tagaact 57
<210> 55
   <211> 67
   <212> DNA
   <213> Artificial Sequence
<220>
   <223> biotinylated ssDNA linker 9
<220>
   <221> misc_feature
   <223> n= biotin dt
<220>
   <221> misc_feature
   <222> (35)..(35)
   <223> n is a, c, g, or t
<400> 55
   gcagaagcat taatagactt tttttttttt ttttnttttt tttttttttt tggacgacga 60
   tagaact 67
<210> 56
   <211> 76
   <212> DNA
   <213> Artificial Sequence
<220>
   <223> biotinylated ssDNA linker 10
<220>
   <221> misc_feature
   <223> n=biotin dt
<220>
   <221> misc_feature
   <222> (40)..(40)
   <223> n is a, c, g, or t
<400> 56
   gcagaagcat taatagactt tttttttttt tttttttttn tttttttttt tttttttttt 60
   ggacgacgat agaact 76
<210> 57
   <211> 39
   <212> DNA
   <213> Artificial Sequence
<220>
   <223> biotinylated ssDNA linker 11
<220>
   <221> misc_feature
   <223> n=biotin dt s=spacer C18
<220>
   <221> misc_feature
   <222> (21)..(21)
   <223> n is a, c, g, or t
<400> 57
   gcagaagcat taatagacts nstggacgac gatagaact 39
<210> 58
   <211> 41
   <212> DNA
   <213> Artificial Sequence
<220>
   <223> biotinylated ssDNA linker 12
<220>
   <221> misc_feature
   <223> n=biotin dt s=spacer c18
<220>
   <221> misc_feature
   <222> (22)..(22)
   <223> n is a, c, g, or t
<400> 58
   gcagaagcat taatagacts snsstggacg acgatagaac t 41
<210> 59
   <211> 43
   <212> DNA
   <213> Artificial Sequence
<220>
   <223> biotinylated ssDNA linker 13
<220>
   <221> misc_feature
   <223> n=biotin dt s=spacer c18
<220>
   <221> misc_feature
   <222> (23)..(23)
   <223> n is a, c, g, or t
<400> 59
   gcagaagcat taatagacts ssnssstgga cgacgataga act 43
<210> 60
   <211> 45
   <212> DNA
   <213> Artificial Sequence
<220>
   <223> biotinylated ss-L-DNA linker 14
<220>
   <221> misc_feature
   <223> n=biotin dt s=spacer c18
<220>
   <221> misc_feature
   <222> (24)..(24)
   <223> n is a, c, g, or t
<400> 60
   gcagaagcat taatagacts sssnsssstg gacgacgata gaact 45
<210> 61
   <211> 77
   <212> DNA
   <213> Artificial Sequence
<220>
   <223> digoxygenylated ss-L-DNA linker 15
<220>
   <221> misc_feature
   <223> n=digoxigenin dt
<220>
   <221> misc_feature
   <222> (40)..(40)
   <223> n is a, c, g, or t
<400> 61
   gcagaagcat taatagactt tttttttttt tttttttttn tttttttttt tttttttttt 60
   tggacgacga tagaact 77
<210> 62
   <211> 37
   <212> DNA
   <213> Artificial Sequence
<220>
   <223> digoxygenylated ss-L-DNA linker 16
<220>
   <221> misc_feature
   <223> n=digoxigenin dt
<220>
   <221> misc_feature
   <222> (20)..(20)
   <223> n is a, c, g, or t
<400> 62
   gcagaagcat taatagactn tggacgacga tagaact 37
<210> 63
   <211> 37
   <212> DNA
   <213> Artificial Sequence
<220>
   <223> biotinylated ss-L-DNA linker 17
<220>
   <221> misc_feature
   <223> n=biotin dt
<220>
   <221> misc_feature
   <222> (20)..(20)
   <223> n is a, c, g, or t
<400> 63
   gcagaagcat taatagactn tggacgacga tagaact 37
<210> 64
   <211> 9
   <212> PRT
   <213> Artificial Sequence
<220>
   <223> HA-tag
<400> 64
   Tyr Pro Tyr Asp Val Pro Asp Tyr Ala
   1               5
<210> 65
   <211> 15
   <212> PRT
   <213> Artificial Sequence
<220>
   <223> Avi-tag
<400> 65
   Gly Leu Asn Asp Ile Phe Glu Ala Gln Lys Ile Glu Trp His Glu
   1               5                   10                  15
<210> 66
   <211> 5
   <212> PRT
   <213> Artificial Sequence
<220>
   <223> linker
<400> 66
   Ser Gly Gly Gly Ser
   1               5
<210> 67
   <211> 3
   <212> PRT
   <213> Artificial Sequence
<220>
   <223> fMLP
<220>
   <221> MISC_FEATURE
   <223> X=f-met
<220>
   <221> misc_feature
   <222> (1)..(1)
   <223> Xaa can be any naturally occurring amino acid
<400> 67
   Xaa Leu Phe
   1
<210> 68
   <211> 4
   <212> PRT
   <213> Artificial Sequence
<220>
   <223> f-Met-Leu-Phe-o-methyl ester
<220>
   <221> MISC_FEATURE
   <222> (1)..(1)
   <223> f-met
<220>
   <221> MISC_FEATURE
   <222> (4)..(4)
   <223> o-methyl ester
<400> 68
   Xaa Leu Phe Xaa
   1
<210> 69
   <211> 107
   <212> PRT
   <213> Homo sapiens
<400> 69
<210> 70
   <211> 105
   <212> PRT
   <213> Homo sapiens
<400> 70
   Gln Pro Lys Ala Ala Pro Ser Val Thr Leu Phe Pro Pro Ser Ser Glu
   1 5 10 15
   Glu Leu Gln Ala Asn Lys Ala Thr Leu Val Cys Leu Ile Ser Asp Phe
   20 25 30
   Tyr Pro Gly Ala Val Thr Val Ala Trp Lys Ala Asp Ser Ser Pro Val
   35 40 45
   Lys Ala Gly Val Glu Thr Thr Thr Pro Ser Lys Gln Ser Asn Asn Lys
   50 55 60
   Tyr Ala Ala Ser Ser Tyr Leu Ser Leu Thr Pro Glu Gln Trp Lys Ser
   65 70 75 80
   His Arg Ser Tyr Ser Cys Gln Val Thr His Glu Gly Ser Thr Val Glu
   85 90 95
   Lys Thr Val Ala Pro Thr Glu Cys Ser
   100 105
<210> 71
   <211> 330
   <212> PRT
   <213> Homo sapiens
<400> 71
<210> 72
   <211> 330
   <212> PRT
   <213> Homo sapiens
<400> 72
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Glu Glu
   225                 230                 235                 240
   Met Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 73
   <211> 330
   <212> PRT
   <213> Homo sapiens
<400> 73
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Ala Ala Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 74
   <211> 327
   <212> PRT
   <213> Homo sapiens
<400> 74
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
   165 170 175
   Asn Ser Thr Tyr Arg Val Val Ser Val Leu Thr Val Leu His Gln Asp
   180 185 190
   Trp Leu Asn Gly Lys Glu Tyr Lys Cys Lys Val Ser Asn Lys Gly Leu
   195 200 205
   Pro Ser Ser Ile Glu Lys Thr Ile Ser Lys Ala Lys Gly Gln Pro Arg
   210 215 220
   Glu Pro Gln Val Tyr Thr Leu Pro Pro Ser Gln Glu Glu Met Thr Lys
   225 230 235 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Arg Leu Thr Val Asp Lys Ser Arg Trp Gln Glu Gly Asn Val Phe Ser
   290 295 300
   Cys Ser Val Met His Glu Ala Leu His Asn His Tyr Thr Gln Lys Ser
   305 310 315 320
   Leu Ser Leu Ser Leu Gly Lys
   325
<210> 75
   <211> 327
   <212> PRT
   <213> Homo sapiens
<400> 75
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Glu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305 310 315 320
   Leu Ser Leu Ser Leu Gly Lys
   325
<210> 76
   <211> 23
   <212> DNA
   <213> Artificial Sequence
<220>
   <223> detection probe
<220>
   <221> misc_feature
   <223> Y = 5'-Amino-Modifier Z = 3'-Aminomodifier C6 Cy5-dye at 5' and 3' terminus
<220>
   <221> misc_feature
   <222> (1)..(2)
   <223> n is a, c, g, or t
<220>
   <221> misc_feature
   <222> (22)..(23)
   <223> n is a, c, g, or t
<400> 76
   nnatgcgagt accttagagt cnn 23
<210> 77
   <211> 95
   <212> DNA
   <213> Artificial Sequence
<220>
   <223> T20-linker
<400> 77
   gcagaagcat taatagactt tttttttttt tttttttttg actctaaggt actcgcattt 60
   tttttttttt tttttttttg gacgacgata gaact 95
<210> 78
   <211> 5
   <212> PRT
   <213> Artificial Sequence
<220>
   <223> sortase tag
<220>
   <221> MISC_FEATURE
   <222> (3)..(3)
   <223> X = any amino acid residue
<400> 78
   Leu Pro Xaa Thr Gly
   1               5
<210> 79
   <211> 19
   <212> DNA
   <213> Artificial Sequence
<220>
   <223> oligonucleotide
<220>
   <221> misc_structure
   <222> (1)..(1)
   <223> n = 5'-(Gly)4-Aminolinker-(Spacer C3)3
<220>
   <221> misc_feature
   <222> (1)..(1)
   <223> n is a, c, g, or t
<220>
   <221> misc_structure
   <222> (19)..(19)
   <223> n = Fluorescein
<220>
   <221> misc_feature
   <222> (19)..(19)
   <223> n is a, c, g, or t
<400> 79
   nagttctatc gtcgtccan 19
<210> 80
   <211> 66
   <212> DNA
   <213> Artificial Sequence
<220>
   <223> L-DNA linker
<400> 80
   gcagaagcat taatagactt gactctaagg tactcgcatt tttttttttt ggacgacgat    60
   agaact                                                                 66